# EUROPEAN PATENT SPECIFICATION

(11) **EP 2 585 612 B1**
(45) Date of publication and mention of the grant of the patent: **22.10.2014**
(21) Application number: 11724556.3
(22) Date of filing: 03.06.2011
(51) Int. Cl.: C12Q 1/68

(54) **GENE EXPRESSION ANALYSES FOR CHARACTERIZING AND IDENTIFYING GENOTOXIC COMPOUNDS**
GENEXPRESSIONSANALYSEN ZUR CHARAKTERISIERUNG UND IDENTIFIZIERUNG GENOTOXISCHER VERBINDUNGEN
ANALYSES DE L'EXPRESSION DE GÈNES POUR CARACTÉRISER ET IDENTIFIER DES COMPOSÉS GÉNOTOXIQUES

(30) Priority: 24.06.2010 DE 102010024898
(43) Date of publication of application: 01.05.2013
(73) Proprietor: Merck Patent GmbH, 64293 Darmstadt (DE)
(72) Inventor: MUELLER, Stefan, 64625 Bensheim (DE); HEWITT, Philip, 64390 Erzhausen (DE); BOEHME, Kathleen, 04159 Leipzig (DE)
(86) International application number: PCT/EP2011/002742
(87) International publication number: WO 2011/160767

(56) References cited:
- LE FEVRE ET AL: "Characterization of DNA reactive and non-DNA reactive anticancer drugs by gene expression profiling", MUTATION RESEARCH, ELSEVIER, AMSTERDAM, NL, vol. 619, no. 1-2, 21 April 2007 (2007-04-21), pages 16-29, XP022042430, ISSN: 0027-5107, DOI: DOI:10.1016/J.MRFMMM.2006.12.007
- ELLINGER-ZIEGELBAUER H ET AL: "Application of toxicogenomics to study mechanisms of genotoxicity and carcinogenicity", TOXICOLOGY LETTERS, ELSEVIER BIOMEDICAL PRESS, AMSTERDAM, NL, vol. 186, no. 1, 10 April 2009 (2009-04-10), pages 36-44, XP026001793, ISSN: 0378-4274, DOI: DOI:10.1016/J.TOXLET.2008.08.017 [retrieved on 2008-09-04]
- THYBAUD VERONIQUE ET AL: "Application of toxicogenomics to genetic toxicology risk assessment", June 2007 (2007-06), ENVIRONMENTAL AND MOLECULAR MUTAGENESIS, VOL. 48, NR. 5, PAGE(S) 369-379, XP002652542, ISSN: 0893-6692 the whole document
- VINKEN M ET AL: "The carcinoGENOMICS project: Critical selection of model compounds for the development of omics-based in vitro carcinogenicity screening assays", MUTATION RESEARCH. REVIEWS IN MUTATION RESEARCH, ELSEVIER, AMSTERDAM, NL, vol. 659, no. 3, 1 September 2008 (2008-09-01), pages 202-210, XP024100887, ISSN: 1383-5742, DOI: DOI:10.1016/J.MRREV.2008.04.006 [retrieved on 2008-04-26]
- ELLINGER-ZIEGELBAUER ET AL: "Prediction of a carcinogenic potential of rat hepatocarcinogens using toxicogenomics analysis of short-term in vivo studies", 18 December 2007 (2007-12-18), MUTATION RESEARCH, ELSEVIER, AMSTERDAM, NL, PAGE(S) 23 - 39, XP022392711, ISSN: 0027-5107 the whole document
- LAUER B ET AL: "Profiling the toxicity of new drugs: a non animal-based approach integrating toxico-dynamics and biokinetics", NAUNYN-SCHMIEDEBERG'S ARCHIVES OF PHARMACOLOGY, vol. 383, no. Suppl. 1, March 2011 (2011-03), page 84, XP009150558, & 77TH ANNUAL MEETING ON GERMAN-SOCIETY-FOR-EXPERIMENTAL-AND-CLINIC AL-P HARMACOLOGY-AND-TOXICOLOGY; FRANKFURT, GERMANY; MARCH 30 -APRIL 01, 2011

## Description

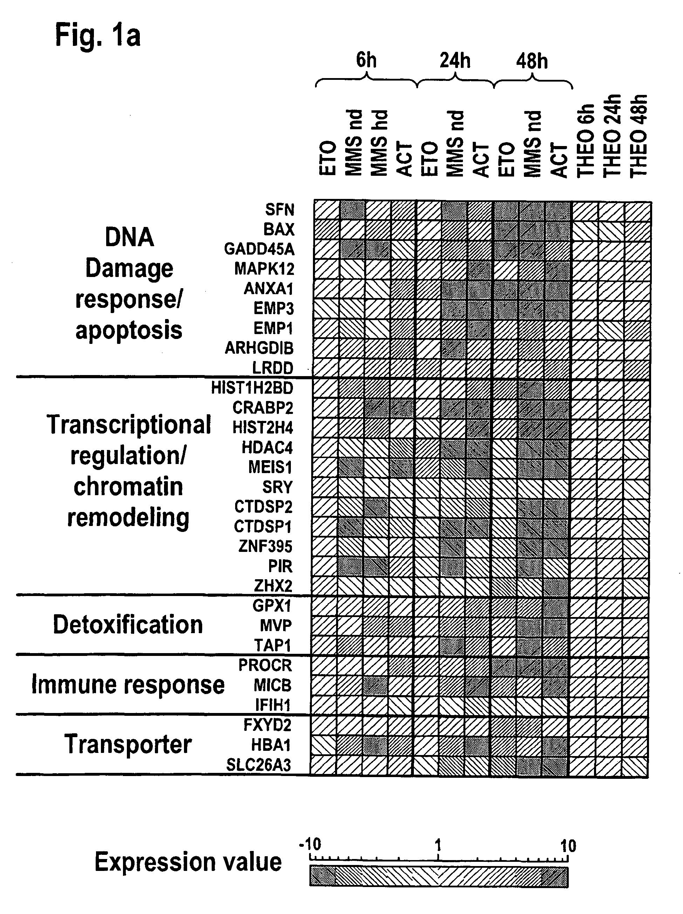

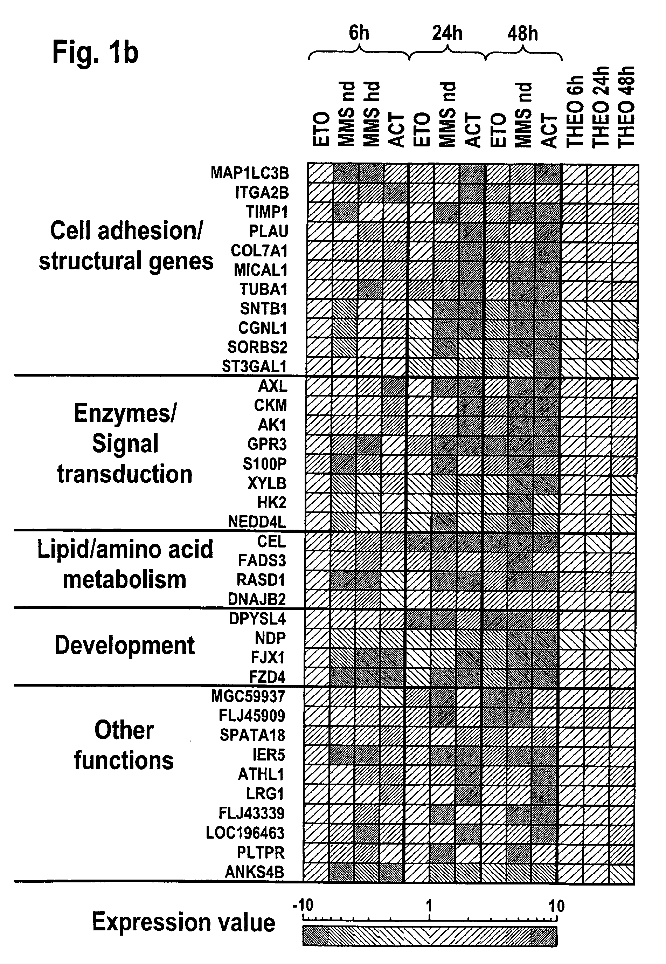

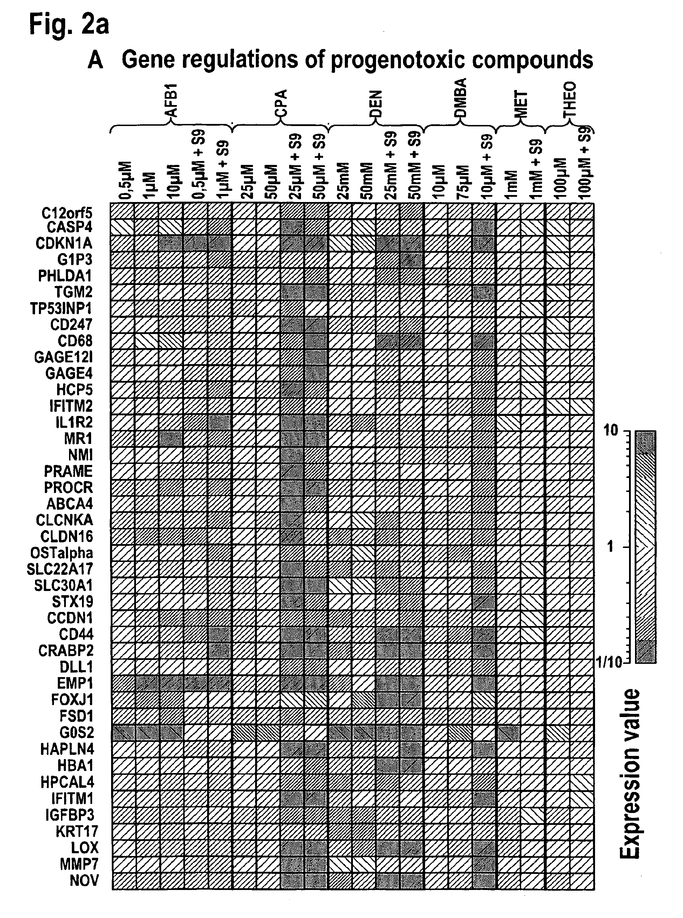

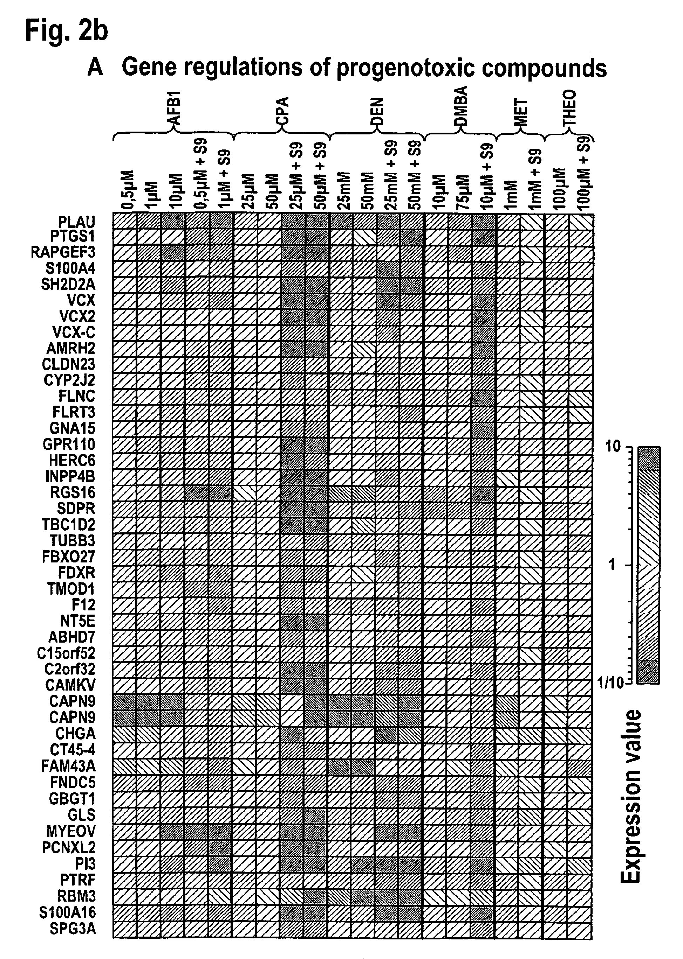

The invention relates to a method for screening compounds with (pro-)genotoxic activity by providing a cellular system being capable of expressing at least a panel of 11 defined genes, incubating at least a portion of the system with compounds to be screened, and comparing the expression of the genes in the system with the gene expression in a control cellular system, thereby detecting the (pro-)genotoxic activity. Another object of the invention concerns a method for monitoring physiological and/or pathological conditions, which are caused, mediated and/or propagated by the genetic deregulation of proliferation, differentiation and/or damage repair, by administering an effective amount of at least a single (pro-)genotoxic compound to a mammal in need of such treatment and determining an expression of 11 defined genes in a biological sample withdrawn from the mammal. The invention also relates to arrays for screening compounds with (pro-)genotoxic activity comprising nucleic acid probes that specifically hybridize under stringent conditions with the marker genes of Table 1, Figure 1a+b and/or Figure 2 a+b.

Genotoxicity testing is an important part of the standard testing strategy within pharmaceutical development and for risk evaluation of chemical substances. In order to reflect various different genotoxic mechanisms of action, standard testing involves a battery assessment of mutagenicity in bacteria and of chromosomal damaging properties to mammalian cells *in vitro* and/or *in vivo.*

Recently, mechanistic investigations have gained significantly in interest - in particular within the scope of modern risk assessment of genotoxic and carcinogenic substances and for the chemical characterization within the EU REACH regulation program. Integrative biology's "'omics" technologies stand opposite to the classical, reductionist approach of single endpoint measurements by generating a comprehensive view of cellular mechanisms via recording all components in a given system.

Within the past few years the first studies applying Toxicogenomics (TXG) for genotoxicity and carcinogenicity evaluation were published and demonstrated overlapping gene expression signatures for different genotoxic hepatocarcinogens in rats that were distinct to those of non-genotoxic hepatocarcinogens, and unknown substances could be classified correctly with an accuracy of 75-88 % by means of a classification model built up from data of well-described hepatocarcinogens (Ellinger-Ziegelbauer et al., 2008, Mutat. Res. 637, 23-39). In 2006, the carcinoGENOMICS project was originated to develop alternatives to the rodent carcinogenicity life-time bioassays *in vitro.*

In spite of being highly sensitive, mammalian cell assays are limited by their low specificity producing a high rate of false positives which in turn complicates result interpretation. False positive outcomes complicate human hazard extrapolation (Kirkland et al., 2005, Mutat. Res. 584, 1-256; Kirkland et al., 2006, Mutat. Res. 608, 29-42) and initiate laborious follow-up testing *in vitro* and/or *in vivo* encouraging the development of new *in vitro* tools.

Efforts to reduce the false positive rate are reflected in the development of the new ICH S2 (R1) guideline and the consideration of the most effective assay combinations in standard testing. The difficulties are now forcing the development of new *in vitro* tools.

Therefore, the technical problem forming the basis of the present invention is to provide a method for screening compounds, which effectively allows the identification and characterization of their genotoxic and/or pro-genotoxic properties. It is another problem of the invention to provide an array for the detection of genotoxic and/or pro-genotoxic activity, which makes a simple and fast monitoring of genotoxicity-dependent diseases possible.

The present invention solves the problem by providing a method for screening compounds with genotoxic and/or pro-genotoxic activity comprising the steps of:
(a) providing a cellular system or a sample thereof capable of expressing at least the genes GLS2, IER5, TMEM194, PROCR, ITGA2B, FADS3, STMN3, PIB5PA, ROBO3, EDA2R and KIF1A, wherein the system is selected from the group of single cells, cell cultures, tissues, organs and mammals or a sample thereof,
(b) incubating at least a portion of the system with compounds to be screened, and
(c) detecting the genotoxic and/or pro-genotoxic activity by gene expression analysis, wherein the expression of said genes in the system is compared with the gene expression in a control cellular system.

It has been surprisingly demonstrated by the inventors that the aforementioned group of at least 11 genes is correlated with genotoxicity. Consequently, the aforementioned plurality of marker genes represents novel genotoxicity target genes, which themselves and their gene products, respectively, are well suited targets for differentiating the stage of genotoxicity. The underlying genes are selected as result of a differential expression analysis. The identified genes are not inevitably associated by function or location in their entity as presently known, but it is not excluded that such relations appear between a single member or more members of the group. Instead of that, all genes are characterized by a distinct difference to untreated cells, which is exhibited by either up-regulation or repression. The genes are already described in the state of the art by sequence and other features, but lacking a linkage to genotoxicity either alone or in the defined combination of the invention. Either the 11 marker gene or supplemental marker genes pursuant to Table 1 can be used for the utmost test reliability. The genes may be named in another way, but are easily assigned by the accession number, which is generally accepted and fixed in numerous data bases, such as the GenBank, SwissProt and the like.

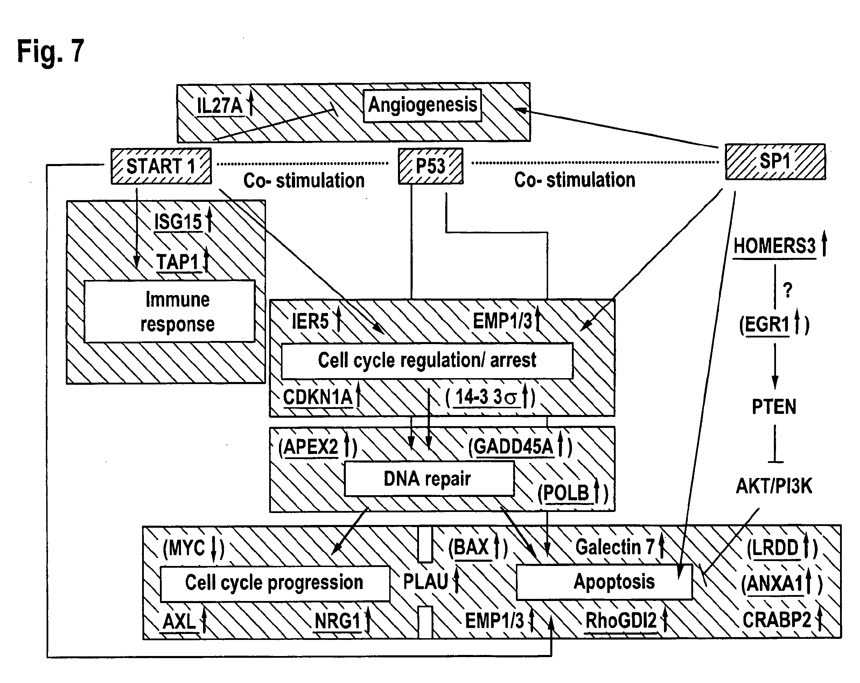

The inventors have unexpectedly identified characteristic gene regulating processes related to DNA damage induced by (pro-)genotoxic test compounds, particularly genotoxic compounds. A comprehensive overview of the DNA damage response network is provided (Figure 7). Although STAT1, SP1 and P53 were not regulated themselves at the gene expression level, target gene regulation indicated an activation of these transcriptional modulators in response to the treatment with genotoxicants. The activity of these transcription factors is crucially regulated by means of protein phosphorylation which can be induced by multiple signals, including general cellular stress, DNA damage or interferons.

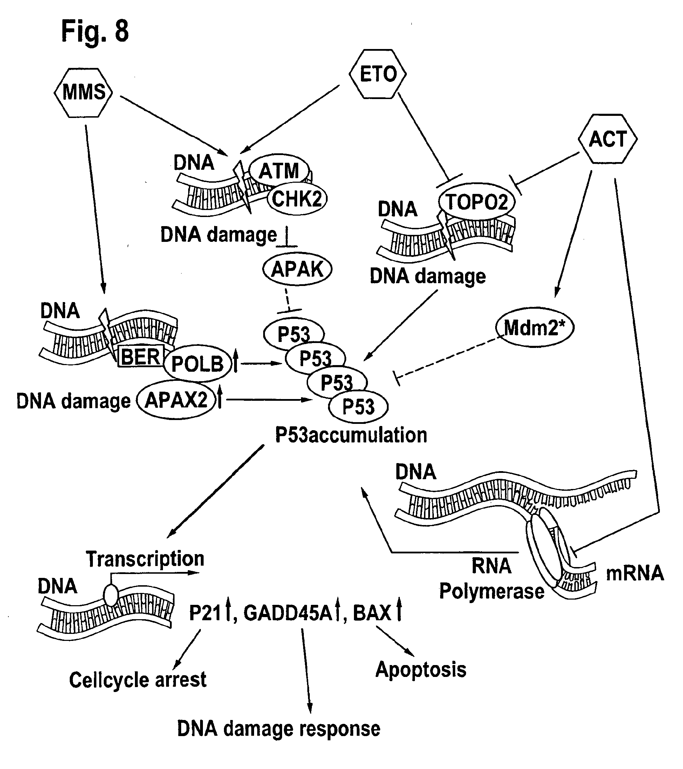

P53 is significantly elevated in response to genotoxic and pro-genotoxic test compounds. The mechanism of the genotoxic test compounds ETO, MMS, and ACT leads to p53 activation and the mediation of the p53 signaling response (Figure 8). The dose of ACT inducing p53 inhibition correlates with doses needed for mRNA synthesis inhibition. Moreover, the relatively rare rate of DNA strand breaks at high ACT doses indicated that topoisomerase inhibition is of secondary importance for p53 accumulation. The mechanism of p53 activation can be seen as a sensory function of the ACT-blocked RNA polymerase II (POLR2) or with associated transcription factors of this polymerase. A down-regulation of the topoisomerase II (TOPO2) and RNA polymerase II were observed by ACT (Figure 8). In addition, the lack of mdm2 function, as well as the activation of APAK (ATM and P53-associated KZNF protein) - a recently discovered p53 regulator, suggests a role for ACT, MMS and presumably also for ETO-mediated p53 induction. A novel mdm2-related mechanism of p53 accumulation is mediated via the induction of a truncated mdm2 splice variant by ACT, mdm2⁺¹⁰⁸, which lacks the critical p53 regulatory domain. Thus, p53-mdm2 feedback regulation will be disturbed causing a massive increase in p53. APAK was not found to be regulated at the gene expression level in response to the genotoxic test compounds. In addition to the mechanism of P53 activation, various genes were found to be up-regulated mediated by the P53 response. BAX, encoding a mitochondrial permeability-promoting protein and being involved in apoptosis induction was found to be strongly up-regulated by all direct-acting genotoxic test compounds (Figures 7 and 8). Moreover, AP endonuclease 2 (APEX2) was found to be up-regulated after 24 h and 48 h MMS treatment and β-polymerase (POLB) after 48 h exposure with MMS. Both proteins are suggested to mediate p53 functions within the base excision repair of alkylated DNA bases, originating from exposure with agents such as MMS. GADD45α was also markedly up-regulated by all genotoxic test substances (Figure 7 and 8). GADD45α triggers cell cycle arrest via CDK1/Cyclin B inhibition and mediates DNA repair via recognition of modified DNA areas and facilitating the accessibility of the damaged positions by destabilization of DNA-histone interactions. The expression of two other P53-regulated genes was increased in response to the treatment with the (pro-)genotoxic test compounds: 14-3-3-σ (Stratifin, SFN) and CDKN1A (p21). Both protein products provoke cell cycle arrest in response to DNA damage. 14-3-3-σ prevents the nuclear import of cdc25, a protein phosphatase needed for active dephosphorylation of cdk1 and thus, cell cycle progression under non-inhibitive conditions. In addition to the regulatory functions at the G₂ checkpoint, 14-3-3-σ as well as p21 provoke cell cycle arrest during the G₁ phase.

Apart from P53, STAT1 (signal transducer and activator of transcription 1) has also been attributed to have tumor suppressor characteristics and inductive functions with regards to promoting cell cycle arrest and apoptosis after genotoxic stress. The postulated mechanism proceeds through ATM-NBS1-SMC1 and ATM-Chk2-Cdc25 signaling cascades leading to an efficient inhibition of DNA synthesis after DNA damage. Furthermore, a direct interaction between STAT1 and P53 is thought to modulate P53 dependent transcriptional effects and apoptosis in a co-regulatory manner. In addition, STAT1 has been described as a negative regulator of the p53 inhibitor mdm2. Direct targets of STAT1 are apoptosis and cell cycle regulatory genes such as Fas, Fas ligand and the Cdk inhibitors p21 and p27. Among the 91 genes discovered, three STAT1 target genes were identified to be up-regulated in response to the exposure with (pro-)genotoxicants: IL27RA (Interleukin 27 receptor, alpha; also designated as TCCR/ WSX1), ISG15 (interferon-stimulated gene 15 kDa) and TAP1 (transporter 1, ATP-binding cassette, sub-family B) (Figure 7). IL27, the ligand of IL27R, has functionalities in immune response suppression, T helper type 1 differentiation as well as anti-angiogenic and anti-proliferative (anti-tumor) properties. TAP1 is a transporter of the TAP/ MDR family, responsible for the loading of MHC class I molecules for antigen presentation. Down-regulation of TAP1 in tumor cells is associated with malignant transformation by preventing the immune reaction against the degenerative cells. Dysregulation of ISG15 is also linked to tumor promotion.

Another transcriptional regulator, the zinc-cysteine-histidine motif containing SP1, was highlighted during biological analysis of 91 genes presently found (Figure 7). SP1 has been reported to be involved in a variety of processes, for instance, cell cycle regulation, hormone activation, apoptosis, and angiogenesis and is activated by many different cell cycle regulators, including CDK4, Rad51, E2-DP1, p21 or Stat3. Furthermore, it has been demonstrated, that SP1 can be activated after DNA damage through phosphorylation by the damage-sensing kinases DNA-PK and ATM. Moreover, the functionality of SP1 seems to be strongly dependent upon the P53 status of the cell as previous publications have shown that SP1-induced apoptotic cell death is triggered exclusively in the presence of P53. However, beside pro-apoptotic characteristics, SP1 has also been proposed to have growth stimulatory properties. One target of SP1 is c-Myc, which stimulates cell cycle progression and therefore, plays an important role in carcinogenesis. MYC was found to be down-regulated by ACT, ETO and MMS. EGR1 (early growth response 1), a further transcription factor of the SP1 regulatory network, was also deregulated, however, up-regulation was observed for MMS, DEN, AFB1 and CPA. Therefore, MYC and EGR1 were not assigned to the 91 putative marker genes. EGR1 induction has been associated with the influence of DNA damaging compounds and EGR1 facilitates P53 activation as well as inhibits the PI3K/Akt signaling pathway by up-regulating the tumor suppressor PTEN. An indirect interaction of EGR1 and HOMER3 (homer homolog 3) via CEBPB (CCAAT/enhancer binding protein, beta) is postulated. HOMER3 was found to be up-regulated by the (pro-)genotoxic test substances in the microarray study. In addition, RhoGDI2 (Rho GDP dissociation inhibitor (GDI) beta, ARHGDIB), the receptor tyrosine kinase AXL and Neuregulin 1 (NRG1) were found to be up-regulated in this study. While GDIs (GDP dissociation inhibitors) are targets of caspases and therefore, are involved in programmed cell death, AXL and NRG1 generally exert growth stimulatory functions mediated via Akt (protein kinase B, v-akt murine thymoma viral oncogene). In contrast, a growth inhibitory mode of action has been described also for NRG1, depending on the cellular situation. AKT1 mRNA itself was not differentially regulated suggesting another mechanism by which NRG1 and AXL responded to the (pro-)genotoxicants.

Among the 91 top scored genes, IER5 (immediate early response 5), EMP 3 (epithelial membrane protein 3), EMP1 (epithelial membrane protein 1), CRABP2 (cellular retinoic acid binding protein 2), PROCR (protein C receptor, endothelial/ EPCR) and PLAU (plasminogen activator, urokinase) were consistently up-regulated among the samples annotated as genotoxic (Table 3/ Figure 7). Recently, IER5 and PROCR induction in response to ionizing radiation has been discovered. Moreover, siRNA-mediated suppression of IER5 in Hela cells enhanced the radiation-induced G₂/M arrest. EMP1 also revealed cell cycle regulatory properties by prolongation of the G₁ phase and shortened S phase and has been found to be over expressed in different tumors. Transient expression of EMP1 lead to a specific inhibition of cell proliferation and an apoptosis-like phenotype was observed after over expression of EMP1 in NIH3T3 cells. EMP3, another member of the family of peripheral 22-kDa myelin proteins (PMP22 or TMP gene family), is likely to play a role in cell communication and proliferation. Reintroduction of EMP3 to deficient tumor cells inhibited tumor growth and colony formation suggesting tumor suppressive roles of EMP3. Moreover, cytoprotective properties of EMP3 could be demonstrated in HepG2 cells. CRABP2 encodes a small 15-kDa protein containing a lipocalin domain for retinoic acid (RA) binding. Therefore, CRABP2 is important for nuclear translocation of RA, which regulates the transcription of genes involved in development, embryogenesis, differentiation and apoptosis after binding to the retinoic acid receptor (RAR). CRABP2 is hypothesized to mediate the anti-proliferative effects of the RAR signaling pathway. In contrast to the genes aforementioned, PROCR and PLAU preferably stimulate cell survival. PROCR, a MHC I family member, typically exerts anti-inflammatory and anti-coagulative properties. The cell growth-promoting activities of PLAU are probably realized via the activation of MAPK (p38) signaling after binding of PLAU to uPAR. PLAU is thought to have anti-apoptotic functions by activating RAS-ERK and PI3K-Akt signaling. Moreover, a functional link between Bcl2-family members and Fas (TNF/death receptor) signaling within PLAU-mediated apoptosis suppression has been addressed.

The analysis of the 91 top scored genes and associated molecules discussed above, demonstrates the complex regulation of cell death and survival in response to the exposure of HepG2 cells with (pro-)genotoxicants. Mainly affected processes comprise cell cycle regulation, cell proliferation and apoptosis. Genes found to be differentially regulated could predominantly be assigned to pro-apoptotic and anti-proliferative functions. The data indicate an induction of cell cycle arrest and programmed cell death in response to test compound-induced DNA damage.

The linkage of genotoxicity to distinct genes is utilized for the *in vitro* detection of mutagens and pro-mutagens, which are able to interfere with signaling in proliferation, differentiation or damage repair. Building a compound specific gene expression profile, which is based on the plurality of genes according to Table 1, is of unexpected benefit in establishing a genotoxic mechanism of action and, therefore, supports the evaluation of potential hazards or benefits of novel compounds supplementary to the classical screening methods. That means the inventive principle underlying the present method comprises prospecting for the defined genes or gene products thereof that can be either detected on the nucleic acid level or on the protein level, wherein the nucleic acid level is preferred, more preferably mRNA. The gene product is chosen in respect of both its absolute and relative amount as well as the specificity for a certain cell type.

In general, "a gene" is a region on the genome that is capable of being transcribed to RNA that either has a regulatory function, a catalytic function and/or encodes a protein. A gene typically has introns and exons, which may organize to produce different RNA splice variants that encode alternative versions of a mature protein. "Gene" contemplates fragments of genes that may or may not represent a functional domain.

A "plurality of genes" as used herein refers to a group of identified or isolated genes whose levels of expression vary in different tissues, cells or under different conditions or biological states. The different conditions may be caused by exposure to certain agent(s) - whether exogenous or endogenous - which include hormones, receptor ligands, chemical compounds and the like. The expression of a plurality of genes demonstrates certain patterns. That is, each gene in the plurality is expressed differently in different conditions or with or without exposure to a certain endogenous or exogenous agents. The extent or level of differential expression of each gene may vary in the plurality and may be determined qualitatively and/or quantitatively according to this invention. A gene expression profile, as used herein, refers to a plurality of genes that are differentially expressed at different levels, which constitutes a "pattern" or a "profile." As used herein, the term "expression profile", "profile", "expression pattern", "pattern", "gene expression profile" and "gene expression pattern" are used interchangeably.

The term "gene product" denotes molecules that are formed from the substrate of said genes by biochemical, chemical or physical reactions, such as DNA synthesis, transcription, splicing, translation, fragmentation or methylation. Preferred gene products of the invention are RNA, particularly mRNA and cRNA, cDNA and proteins.

As used herein, a "compound with genotoxic activity", also referred to as "mutagen", is a physical or chemical agent that changes the genetic material, usually DNA, of an organism and thus increases the frequency of mutations above the natural background level. The skilled artisan would know that, for instance, one of the biological effects of mutagens is to promote the development cancer. Other biological effects of mutagens are well documented and discussed. The changes in nucleic acid sequences by mutations comprise substitution of nucleotide base-pairs and insertions and deletions of one or more nucleotides in DNA sequences.

In the first step (a), a cellular system is provided. The cellular system is defined to be any subject provided that the subject comprises cells. Hence, the cellular system can be selected from the group of single cells, cell cultures, tissues, organs and mammals. The scope of the cellular system also comprises parts of such biological entities, i.e. samples of tissues, organs and mammals. It shall be understood that each cellular system in the aforementioned order could represent a sample of the respective following system.

Particularly, the cellular sample is taken *in vivo* or *in situ* from a mammal to be tested. The withdrawal of the cellular sample follows good medical practice. Biological samples may be taken from any kind of biological species, but the sample is especially taken from a human, rat or a mouse, more preferably a human.

In the present invention, the cellular system may also comprise a biological fluid, wherein the sample of body fluid preferably consists of blood, serum, plasma, saliva or urine. It is also preferred to gather a tissue sample by biopsy, especially taken close to the location of ailment. The biological samples can be originated from any tissue, including the uterus, pituitary gland, liver, brain, colon, breast, adipose tissue, etc. In preferred embodiments, the biological samples are from the kidney, pituitary gland and the uterus. The sample may be purified to remove disturbing substances, such as inhibitors for the formation of hydrogen bonds.

The cell sample refers to any type of primary cells or genetically engineered cells, either in the isolated status, in culture or as cell line, provided that they are capable of expressing at least the genes GLS2, IER5, TMEM194, PROCR, ITGA2B, FADS3, STMN3, PIB5PA, ROBO3, EDA2R and KIF1A. It shall be understood that variants, mutants, parts or homologous gene sequences having the same function, are included in the scope of definition as well as protection. The degree of alteration between the original sequence and its derivatives is inevitably limited by the requirement of altered gene expression by mutagens. Preferably, the homology amounts to at least 85 %. Possible alterations comprise deletion, insertion, substitution, modification and addition of at least one nucleotide, or the fusion with another nucleic acid. The engineered cells are capable of expressing these genes by transfection with appropriate vectors harboring them or parts thereof. Preferably, the recombinant cells are of eukaryotic origin.

In a more preferred embodiment of the present invention, HepG2 cells are provided in step (a) of the screening method. The dose of S9 used has an influence on the experimental outcome in spite of being negative in cytotoxicity evaluations. It should be noted that the dose is comparable to the dose used within regulatory standard genotoxicity testing. The significantly S9-regulated genes differ from those induced by the (pro-)genotoxic agents and the S9-effect can be adjusted by comparison to the proper controls. In any case, a cellular system having sufficient intrinsic metabolic activity would certainly be appropriate, but currently no such metabolically competent cellular system exists for genotoxicity evaluation. Primary hepatocytes are the current gold standard for drug metabolism and CYP induction/ inhibition studies *in vitro.* Even though adult differentiated hepatocytes lack proliferation competency making them an unsuitable model for genotoxicity investigations, and they also exhibit significant changes in morphology as well as protein and gene expression during cultivation, limited availability of human hepatocytes and often marked donor/batch variability complicating standardization of such systems, a major advantage of HepG2 cells are their human molecular characteristics. For instance, specific targets such as topoisomerases and eukaryotic repair enzymes are expressed and prevent the overestimation of genotoxicity and therefore, contribute to a reduction of false positives.

The cell sample is stored, such as frozen, cultivated for a certain period or immediately subjected to step (b). Before incubating it with compounds to be screened, the cell sample is divided into multiple portions. At least two portions are provided; one is used for screening while the other one serves as control. Preferably, the number of portions for screening exceeds the number of control portions. Usually, numerous portions are subjected to a high-throughput screening.

The compounds are composed of biological and/or chemical structures capable to interact with a target molecule. Herein, any component of genomics signaling shall be considered as "target molecule", which is not limited to the selected genes themselves, or a regulator protein or a gene product thereof, or a component of a signal transduction pathway comprising said gene or gene products thereof. Consequently, the specific interaction of compounds may involve either the mere targeting or the induction of alterations in cell function, or it may even include both effects simultaneously.

The compounds to be screened in the inventive method are not restricted anyway. In particular, the compounds are selected from the group of nucleic acids, peptides, carbohydrates, polymers, small molecules having a molecular weight between 50 and 1.000 Da and proteins. These compounds are often available in libraries. It is preferred to incubate a single compound within a distinct portion of the cell sample. However, it is also possible to investigate the cooperative effect of compounds by incubating at least two compounds within one portion. A further portion of cells is simultaneously incubated in the absence of the compounds.

The term "incubation" denotes the contacting of the compounds with the cells for a distinct period, which depends on the kind of compounds and/or target. The incubation process also depends on various other parameters, e.g. the cell type and the sensitivity of detection, which optimization follows routine procedures known to those skilled in the art. The incubation procedure can be realized without a chemical conversion of mutagens or may involve a metabolic conversion of pro-mutagens. Adding chemical solutions and/or applying physical procedures, e.g. impact of heat, can improve the accessibility of the target structures in the sample. Specific incubation products are formed as result of the incubation.

In step (c), the identification of effective compounds in the meaning of the invention is indirectly performed by determining the expression pattern of at least the defined 11 genes of Table 1, which the system is capable of expressing. The determination is performed at a specified moment and correlated to the signal strength at the beginning of the experiment and the positive/negative control. Either the control system is not incubated with the compounds (negative control) or the control system is incubated with a standard compound having no genotoxic activity (negative control) or a standard compound having (pro-)genotoxic activity (positive control) as set forth at the example of microarray below. The activity is revealed by a change in expression. Preferably, the genes expressed or repressed in cells with mutagen exposure are compared to the genes expressed or repressed in cells that were not exposed to mutagens. Pairwise comparisons are made between each of the treatments. A pairwise comparison is the expression data for a given gene under a given treatment condition compared to the expression data for this gene under a second treatment condition. The comparison is performed using suitable statistical technique with the assistance of known and commercially available programs.

It is a more preferred aspect of the invention that the existing activity is detected in step (c) if the expression of genes is up-regulated or down-regulated in the system in comparison with a negative or positive control system, or if the expression of genes is substantially identical in the system and a positive control system. It is a more preferred aspect of the invention that the existing activity is detected in step (c) by differential gene expression analysis with the negative control system. Suitable tests for monitoring gene expression, determination and variant analysis of nucleotide sequences are known to those skilled in the art or can be easily designed as a matter of routine. The assay according to the invention may be any assay suitable to detect and/or quantify gene expression. The selected markers can be used to establish screening tools with a higher throughput, for instance, High Content Imaging (HCI) or a gene expression panel (e.g. real-time PCR-based TaqMan™ low density arrays or bDNA assays on Luminex). Both technologies allow the combination of several selected endpoints, preserving biological complexity and molecular interactions to a certain extent. Especially HCI offers the possibility to combine classical genotoxic endpoints (e.g. micronuclei induction) and the analysis of cellular markers with the simultaneous acquisition of cell viability/ cytotoxicity. Cell viability is an important parameter to consider for genotoxicity testing because false positives in standard assays can be generated among others via cytotoxicity. The same holds true for measuring molecular marker, such as P53. Although p53 reacts extremely sensitive to DNA damage, nutrient deprivation and hypoxia could also induce activation. Similarly, STAT1 is known to be activated by hyperosmotic stress, elevated glucose levels, hypoxia or reactive oxygen species. Consideration of cytotoxicity for dose selection, together with multiple endpoint measurements may prevent or reduce false positives. Separate (pro-)genotoxic gene regulations were managed from the non-genotoxic compounds MET and THEO.

Many different types of assays are known, examples of which are set forth below, including analyses by nucleotide arrays and nucleotide filters. The hybridization conditions (temperature, time, and concentrations) are adjusted according to procedures also well known in the art. It is preferred to apply chip hybridization and/or PCR for the determination of gene expression. In another preferred embodiment, the assay of the invention involves the use of a high density oligonucleotide array. In still another preferred embodiment, the analysis is performed by multiplex qPCR, more preferably low density TaqMan arrays or branched DNA assays. Other solid supports and microarrays are known and commercially available to the skilled artisan.

Consequently, this invention relates to a method for predicting the cellular effect of a compound having genotoxic activity by preparing a nucleic acid sample from a cell to be evaluated, contacting the nucleic acid sample with an microarray, detecting a nucleic acid hybridizing with the microarray, and comparing a result detected in step (c) with a result detected using a nucleic acid sample prepared from a control cell.

In a preferred embodiment of the present invention, RNA, cRNA, cDNA and/or protein are detected as the gene products, more preferably mRNA, cRNA and/or cDNA. For instance, the total RNA from such cells is prepared by methods known to the skilled artisan such as by Trizol (Invitrogen) followed by subsequent re-purification, e.g. via Rneasy columns (Qiagen).

The total RNA is used to generate a labeled target according to methods and using detectable labels well-know in the art. For instance, the RNA may be labeled with biotin to form a cRNA target for use in an assay. Next, with the extracted mRNA as a template, cDNAs are produced using a reverse transcriptase (for example, SuperScript Reverse Transcriptase; GibcoBRL) and labeled dNTP (for example, Cy3-dUTP and Cy5-dUTP; Amersham Pharmacia Biotech), and a cDNA sample that reflects the amount of genes expressed within the cells to be evaluated is prepared. This causes labeled cDNA to be included in the cDNA sample. Here, either fluorescent label or radiolabel may be used as a label. The cDNA sample prepared in this manner is applied to the below-mentioned microarray in its single stranded denatured form, and cDNAs included in the cDNA sample are hybridized with the genes immobilized on the basal plate.

*"In situ* hybridization" is a methodology for determining the presence of or the copy number of a gene in a sample, for example, fluorescence *in situ* hybridization (FISH). Generally, *in situ* hybridization comprises the following major steps: (1) fixation of tissue or biological structure to be analyzed; (2) pre-hybridization treatment of the biological structure to increase accessibility of target nucleic acid, and to reduce non-specific binding; (3) hybridization of the mixture of nucleic acids to the nucleic acid in the biological structure or tissue; (4) post-hybridization washes to remove nucleic acid fragments not bound in the hybridization; and (5) detection of the hybridized nucleic acid fragments. The probes used in such applications are typically labeled, for example, with radioisotopes or fluorescent reporters. Preferred probes are sufficiently long, for example, from about 50, 100 or 200 nucleotides (nt) to about 1000 or more nucleotides, to enable specific hybridization with the target nucleic acid(s) under stringent conditions. Here, hybridization with cDNA can be accomplished, preferably by incubating at 50 to 80°C for 10 to 20 hours, more preferably about 65°C for 10 to 20 hours.

As used herein, the term "microarray" refers to nucleotide arrays that can be used to detect biomolecules, for instance to measure gene expression. "Array", "slide" and "(DNA) chip" are used interchangeably in this disclosure. A microarray usually comprises a basal plate, e.g. made of slide glass, silicone, or the like, and DNA fragments immobilized as an array on this basal plate. With this microarray, DNAs contained in a sample can be detected by hybridizing them with the DNA fragments immobilized on the basal plate. Since the DNA within the sample is radiolabeled or fluorescently labeled, detection with radio imaging scanner, fluorescence imaging scanner, or the like is possible. Various kinds of arrays are made in research and manufacturing facilities worldwide, some of which are available commercially. There are, for example, two main kinds of nucleotide arrays that differ in the manner in which the nucleic acid materials are placed onto the array substrate: spotted arrays and *in situ* synthesized arrays. One of the most widely used oligonucleotide arrays is GeneChip made by Affymetrix, Inc. The oligonucleotide probes have a length of 10 to 50 nucleotides (nt), preferably 15 to 30 nt, more preferably 20 to 25 nt. They are synthesized in-silico on the array substrate. These arrays tend to achieve high densities, e.g. more than 40,000 genes per cm². The spotted arrays, on the other hand, tend to have lower densities, but the probes, typically partial cDNA molecules, usually are much longer than 25 nucleotides. A representative type of spotted cDNA array is LifeArray made by Incyte Genomics. Pre-synthesized and amplified cDNA sequences are attached to the substrate of these kinds of arrays.

In one embodiment, the array is a matrix, in which each position represents a discrete binding site for a product encoded by a gene, e.g. a protein or RNA, and in which binding sites are present for products of all genes GLS2, IER5, TMEM194, PROCR, ITGA2B, FADS3, STMN3, PIB5PA, ROBO3, EDA2R and KIF1A, or most or almost all of the genes according to Table 1 and optionally Figure 1 a+b and/or Figure 2 a+b. In one embodiment, the "binding site" (hereinafter "site") is a nucleic acid or nucleic acid analogue to which a particular cognate cDNA can specifically hybridize. The nucleic acid or analogue of the binding site can be, e.g. a synthetic oligomer, a full-length cDNA, a less-than full length cDNA or a gene fragment. Preferably, the microarray has binding sites for genes relevant to the action of the gene expression modulating agent of interest or in a biological pathway of interest. It is preferably that more than one DNA fragment, which is capable of hybridizing under stringent conditions to a gene or parts thereof as selected from the defined group of 11 genes, additional genes according to Table 1 and optionally Figure 1 a+b and/or Figure 2 a+b, is immobilized on the basal plate. The DNA fragment to be immobilized on the basal plate may contain the whole or a part of the genes. The term "parts of a gene" used herein means a portion of the gene and a nucleotide sequence equivalent to at least 10 nt, preferably at least 25 nt, more preferably 50 nt, most preferably 300 nt, highly preferably 500 nt.

It is additionally preferable that genes constitutively expressing regardless of the presence or absence of chemical substances having mutagenic activity (hereinafter referred to as negative control genes and the like) are immobilized on the basal plates of the microarray. The expression level of the genes according to the invention can be corrected by immobilizing negative control genes on the basal plate and correcting the expression level of the negative control genes to a constant value. Thus, the changes in the expression level of genes according to Table 1 and optionally Figure 1 a+b and/or Figure 2 a+b can be detected with certainty. Accuracy can be further enhanced by choosing several negative control genes and/or such that have different expression levels.

The nucleic acid or analogue are attached to a solid support or basal plate, which terms are used interchangeably herein, and which may be made from glass, plastic (e.g. polypropylene or nylon), polyacrylamide, nitrocellulose or other materials. When the DNA fragments and negative control genes are immobilized on the basal plate, a conventionally known technique can be used. For example, the surface of the basal plate can be treated with polycations such as polylysines to electrostatically bind the DNA fragments through their charges on the surface of the basal plate. Furthermore, techniques to covalently bind the 5'-end of the DNA fragments to the basal plate may be used. Alternatively, a basal plate having linkers on its surface can be produced, and functional groups that can form covalent bonds with the linkers are introduced at the end of the DNA fragments. The DNA fragments are immobilized by forming a covalent bond between the linker and the functional group. A preferred method for attaching the nucleic acids to a surface is by printing on glass plates.

Finally, cDNAs that hybridized with the DNA fragments on the microarray are detected. In cases where the hybridized cDNAs are fluorescently labeled, the fluorescence is detected with, for example, a fluorescence laser microscope and a CCD camera, and the fluorescence intensity is analyzed with a computer. Similarly, in cases where the hybridized cDNAs are radiolabeled, detection can be carried out using an RI image scanner and such, and the intensity of the radiation can be analyzed with a computer.

In another embodiment of the screening method, the detection of mutagenic and/or pro-mutagenic activity can be additionally refined in step (c). For this purpose, the gene expression is determined by detecting a respective gene product encoded by the genes GLS2, IER5, TMEM194, PROCR, ITGA2B, FADS3, STMN3, PIB5PA, ROBO3, EDA2R and KIF1A, or all genes of Table 1 and correlating an amount of signal or change in signal with the gene expression in the system. The cellular system of the invention is incubated with various concentrations of an identified endocrine active compound. The amount of emitted signal or change in signal observed in the presence of the mutagenic compound is indicative of the change in gene expression experienced by the compound. The change can be then related to the concentration of the mutagen in the sample, i.e. the calibration curve enables the meter-reading of a matching concentration. Preferably, the calibration curve is based on the Lambert-Beer equation if using UV/VIS coloring or luminescence. Genotoxicity of compounds is diagnosed by comparing the concentration of the gene product in the sample with known gene product concentration levels of cells treated with mutagens and/or not. It shall be understood that the known concentrations are statistically proven, therefore representing a certain level or range, respectively. The direction and strength of gene expression have also been figured out by the differential expression analysis of the target genes of the invention such that either a distinct up-regulation or down-regulation with a certain factor has been recognized as set forth below, which forms the basis of biomarker selection. Any measured concentration, which differs from the gene product concentration level of non-stimulated cells, indicates an abnormality of the tested cell sample, whereas a compound cannot be classified as mutagen at a gene product concentration which is comparable to the concentration level of non-stimulated cells. It is preferred to measure concentrations, which are higher than the gene product concentration level of non-stimulated cells, for detecting genotoxicity. Using this method, the inventors demonstrated sensitivity to submicromolar or even nanomolar concentrations. The calibration plot reveals that the method can be applied in a dynamic range that spans over a couple of magnitude.

According to a preferred embodiment of the invention, the "Polymerase Chain Reaction" or "PCR" is an amplification-based assay used to measure the copy number of the gene. In such assays, the corresponding nucleic acid sequences act as a template in an amplification reaction. In a quantitative amplification, the amount of amplification product will be proportional to the amount of template in the original sample. Comparison to appropriate controls provides a measure of the copy number of the gene, corresponding to the specific probe used, according to the principle discussed above.

Detailed protocols for real-time quantitative PCR are provided, for example, for RNA. The "level of mRNA" in a biological sample refers to the amount of mRNA transcribed from a given gene that is present in a cell or a biological sample. One aspect of the biological state of a biological sample (e.g. a cell or cell culture) usefully measured in the present invention is its transcriptional state. The transcriptional state of a biological sample includes the identities and abundances of the constituent RNA species, especially mRNAs, in the cell under a given set of conditions. Preferably, a substantial fraction of all constituent RNA species in the biological sample are measured, but at least a sufficient fraction is measured to characterize the action of a compound of interest.

The primers are designed based on the nucleotide sequence information of the region flanking the site to be amplified. The primers may be designed so as to amplify a region of 100 to 200 base pairs in length. The nucleic acid amplification method includes, but is not particularly limited to, a PCR, preferably a real-time PCR. The level of mRNA may also be quantified by other methods described herein.

After performing an amplification reaction of a nucleic acid using the biological sample to be analyzed and primers as described above, it is checked whether the nucleic acid is amplified or not. In order to facilitate the detection of an amplified nucleic acid, a primer may be labeled in advance. Examples of applicable fluorescent labels include FAM™, TET™, HEX™, TAMRA™ and ROX™ manufactured by Applied Biosystems. In these cases, either the 5'-end or the 3'-end of a primer may be labeled, preferably the 5'-end. Alternatively, the nucleic acid may be labeled during PCR by using labeled nucleotides, or even after PCR is completed. Light emission is measured by a general-purpose luminescence determination device.

Methods of "real-time quantitative PCR" using TaqMan probes are also well-known in the art. Hence, a TaqMan-based assay can be applied to quantify polynucleotides. TaqMan based assays use a fluorogenic oligonucleotide probe that contains a 5'-fluorescent dye and a 3'-quenching agent. The probe hybridizes to a PCR product, but cannot itself be extended due to a blocking agent at the 3'-end. When the PCR product is amplified in subsequent cycles, the 5'-nuclease activity of the polymerase, for example, AmpliTaq, results in the cleavage of the TaqMan probe. This cleavage separates the 5'-fluorescent dye and the 3'-quenching agent, thereby resulting in an increase in fluorescence as a function of amplification.

The presence or absence of an amplified nucleic acid fragment can also be checked by subjecting a reaction solution to electrophoresis, such as for single-strand conformation polymorphism (SSCP) analysis, which may be performed by capillary electrophoresis. However, other electrophoresis methods, for instance gel electrophoresis, are also applicable and well known to those skilled in the art.

Therefore, the present invention relates to the assessment or measurement of modulations of gene expression by the assays as set forth above. Such modulation refers to the induction or inhibition of expression of a gene. Typically, modulation of gene expression may be caused by endogenous or exogenous factors or agents. The effect of a given compound can be measured by any means known to those skilled in the art. For example, expression levels may be measured by PCR, Northern blotting, Primer Extension, Differential Display techniques, etc. The induction of expression (i.e. up-regulation) refers to any observable or measurable increase in the levels of expression of a particular gene, either qualitatively or quantitatively. Contrary to that, the inhibition of expression (i.e. down-regulation) refers to any observable or measurable decrease in the levels of expression of a particular gene, either qualitatively or quantitatively. The measurement of levels of expression may be carried out using any techniques that are capable of measuring RNA transcripts in a biological sample. Examples of these techniques include, as discussed above, PCR, TaqMan, Primer Extension, Differential display and nucleotide arrays, among other things. It is another embodiment of the present invention that in the case of modulation the gene product concentration either exceeds or under-run, respectively, at least twice the gene product concentration in the control system, preferably at least 10 times, more preferably at least 25 times, most preferably at least 40 times

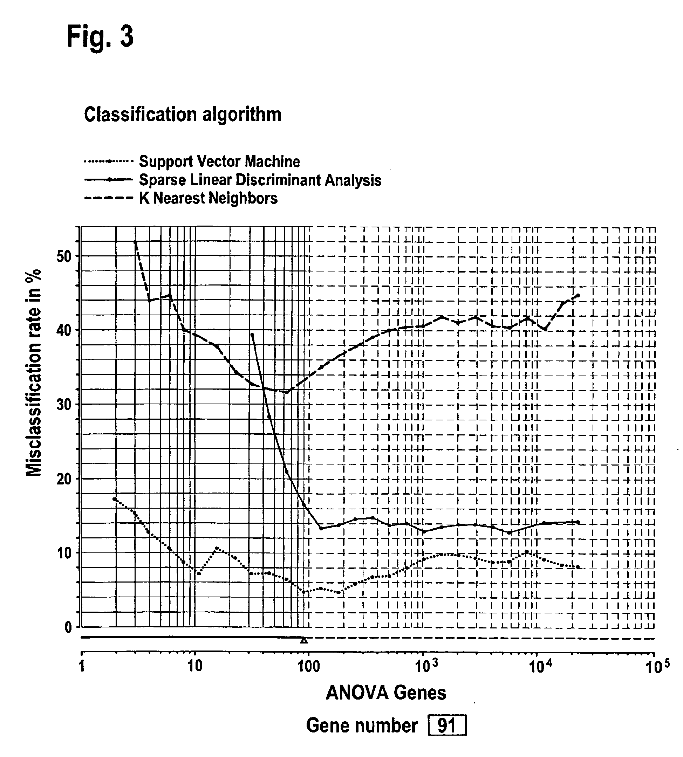

Although the biomarker panel of the invention exhibits a sensitivity that allows the use of only 11 marker genes in the scope of the screening method, it is preferred to apply more than these marker genes for detecting genotoxicity. Pursuant to Figure 3, the ranking shows that low misclassification rates are obtained by using 11 or 32 genes. The rates account below 10% (7.1% for 11 genes and 7.12% for 32 genes, respectively). The gene ranking is given in Table 1. Accordingly, any plurality of genes can be applied while considering the order of genes in the given ranking. In other words, starting with the 11 genes GLS2, IER5, TMEM194, PROCR, ITGA2B, FADS3, STMN3, PIB5PA, ROBO3, EDA2R and KIF1A, which the ranking 1 to 11 is assigned to, the gene panel can be extended by preferably another gene ranking 12, more preferably then by another gene ranking 13, etc., up to 91 genes. In a most preferred embodiment of the invention, the cellular system provided in step (a) is therefore capable of expressing at least genes from ranking 1 to 32 of Table 1, highly preferably the entire panel of 91 genes. Accordingly, the expression of at least 32 genes of Table 1 is most preferably compared with the gene expression in the control system in step (c), highly preferably the entire panel of 91 genes. The inventors have illustrated that analyzing multiple mutagen-responsive genes increases screening stability and reduces error rates by covering a broader spectrum of genotoxic responses than low-plurality-gene reporter assays.

In addition to the expression of genes, which are selected from the group according to Table 1, the cellular system or the sample thereof is preferably capable of expressing at least three genes of Figure 1 a+b and/or at least a single gene of Figure 2 a+b, wherein the additional gene(s) are different from those genes of Table 1, in step (a) of the inventive screening method. More preferred is at least a single different gene of Table 2. Furthermore, in step (c) the expression of the additional gene(s) is compared with the gene expression in the control system.

In another most preferred embodiment of step (a) according to the invention, the provided system is capable of expressing all genes of Table 1, Figure 1 a+b and/or Figure 2 a+b, highly preferably all 91 genes of Table 1, and very highly preferably all different genes of Figure 1 a+b and 2 a+b in addition.

In another embodiment of the invention, it is excluded in step (c) that the gene expression of genes GADD45A, MAPK12 and NTHL1 in the system is compared with the gene expression in the control system.

If in step (a) the cellular system or the sample thereof is capable of expressing multiple genes of Table 1 and/or additionally capable of expressing multiple genes of Figure 1 a+b and/or 2 a+b, and furthermore in step (c) an expression pattern of multiple genes of Table 1, Figure 1 a+b and/or Figure 2 a+b is compared with the expression pattern in the control system, the genotoxicity can be characterized compound-specifically. Particularly, the expression pattern is determined by a correlation of the multiple genes and/or a magnitude of altered regulation. The screening method of this invention not only evaluates the effect of chemical substances having genotoxic activity on cells to be evaluated, but can also indicate the details of this effect. By individually evaluating the expression level of categorized genes, it is possible to distinguish how chemical substances having genotoxic activity that affect the cells to be evaluated.

The invention also teaches an embodiment of the screening method, wherein in step (a) a mammal, preferably a laboratory mammal, is provided, in step (b) the compound to be screened is administered to the mammal, and in step (c) a level of genotoxic and/or pro-genotoxic activity is detected in a biological sample withdrawn from the mammal in comparison with a mammal showing non-genotoxic effects, wherein a difference in level indicates an increased likelihood of said compound to have a therapeutic effect for a genotoxicity-mediated pathological condition. With the therapeutic effect, the qualitative level is incorporated into step (c). A "therapeutic effect" relieves to some extent one or more symptoms of a disease or returns to normality, either partially or completely, one or more physiological or biochemical parameters associated with or causative of the disease or pathological conditions. In addition, the expression "therapeutically effective amount" denotes an amount which, compared with a corresponding subject who has not received this amount, has the following consequence: improved treatment, healing, prevention or elimination of a disease, syndrome, condition, complaint, disorder or side-effects or also the reduction in the advance of a disease, complaint or disorder. The expression "therapeutically effective amount" also encompasses the amounts which are effective for increasing normal physiological function. Testing of several compounds makes the selection of that compound possible that is best suited for the treatment of the mammal subject. The *in vivo* dose rate of the chosen compound is advantageously pre-adjusted to the specific cells with regard to their *in vitro* data. Therefore, the therapeutic efficacy is remarkably enhanced.

The invention also relates to a method for monitoring physiological and/or pathological conditions, which are caused, mediated and/or propagated by deregulation of proliferation, differentiation and/or damage repair, wherein an effective amount of at least one genotoxic or pro-genotoxic compound, or a physiologically acceptable salt thereof, is administered to a mammal in need of such treatment and expression of at least the genes GLS2, IER5, TMEM194, PROCR, ITGA2B, FADS3, STMN3, PIB5PA, ROBO3, EDA2R and KIF1A is determined in a biological sample withdrawn from the mammal. The compound is preferably obtained by the screening method of the invention as set forth above. Thus, the prior teaching of the present specification concerning the screening method is valid and applicable without restrictions to method of monitoring if expedient.

The identification of the plurality of genes described above provides a powerful tool for assessing the progression of a state, condition or treatment. Specifically, a plurality of genes can be identified in a patient prior to an event, such as surgery, the onset of a therapeutic regime, or the completion of a therapeutic regime, to provide a base line result. This base-line can then be compared with the result obtained using identical methods either during or after such event. This information can be used for both diagnostic and prognostic purposes.

The inventive method of monitoring can be employed in human and veterinary medicine. The mammal is preferably a laboratory animal and/or a non-human organism. Herein, the compounds can be administered before or following an onset of disease once or several times acting as therapy. The terms "effective amount" or "effective dose" or "dose" are interchangeably used herein and denote an amount of the pharmaceutical compound having a prophylactically or therapeutically relevant effect on a disease or pathological conditions, i.e. which causes in a tissue, system, animal or human a biological or medical response which is sought or desired, for example, by a researcher or physician.

The aforementioned medical products of the inventive use are particularly used for the therapeutic treatment. Monitoring is considered as a kind of treatment, wherein the compounds are preferably administered in distinct intervals, e.g. in order to booster the response and eradicate the pathogens and/or symptoms of the genotoxicity-mediated disease completely. Either the identical compound or different compounds can be applied. The medicament can also be used to reducing the likelihood of developing a disease or even prevent the initiation of those diseases in advance that are associated with proliferation, differentiation and/or damage repair because of a genotoxic impact, or to treat the arising and continuing symptoms. In the meaning of the invention, prophylactic treatment is advisable if the subject possesses any preconditions for the aforementioned physiological or pathological conditions, such as a familial disposition, a genetic defect, or a previously passed disease. The diseases as concerned by the invention are preferably cancer, tumors, metastasis and/or disorders of angiogenesis.

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. The expressions "pharmaceutically acceptable salt" and "physiologically acceptable salt", which are used interchangeable herein, in the present connection are taken to mean an active ingredient which comprises a compound according to the invention in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

Object of the invention is also the use of genes GLS2, IER5, TMEM194, PROCR, ITGA2B, FADS3, STMN3, PIB5PA, ROBO3, EDA2R and KIF1A as marker genes for screening compounds with genotoxic and/or pro-genotoxic activity. The prior teaching of the present specification concerning the screening method is valid and applicable without restrictions to said uses if expedient.

It is still another object of the present invention to use substances specifically interacting with at least one gene product encoded by a gene of Table 1 for detecting genotoxic or pro-genotoxic activity. The term "specific substances" as used herein comprises molecules with high affinity to at least one gene product encoded by the selected genes, in order to ensure a reliable binding. The substances are preferably specific to parts of the gene product. Such parts represent a restriction to those regions which are sufficient for the expression of a specific function, i.e. the provision of a structural determinant for recognition. All truncations are inevitably limited by the requirement of preserving the unique recognition. However, the parts of the gene products can be very small. Preferably, the substances are mono-specific in order to guarantee an exclusive and directed interaction with the chosen single target.

The recognition of the gene product or parts thereof according to the invention can be realized by a specific interaction with substances on the primary, secondary and/or tertiary structure level of a nucleic acid sequence bearing the gene sequence or an amino acid sequence expressed by the gene. The coding function of genetic information favors the primary structure recognition, Contrary to that, the three-dimensional structure is mainly to be considered for protein recognition. In the context of the present invention, the term "recognition" - without being limited thereto - relates to any type of interaction between the specific substances and the target, particularly covalent or non-covalent binding or association, such as a covalent bond, hydrophobic/ hydrophilic interactions, van der Waals forces, ion pairs, hydrogen bonds, ligand-receptor interactions, interactions between epitope and antibody binding site, nucleotide base pairing, and the like. Such association may also encompass the presence of other molecules such as peptides, proteins or other nucleotide sequences.

The specific substances are composed of biological and/or chemical structures capable to interact with the target molecule in such a manner that makes a recognition, binding and interaction possible. In particular, the substances are selected from the group of nucleic acids, peptides, carbohydrates, polymers, small molecules having a molecular weight between 50 and 1.000 Da and proteins, preferably nucleic acids. The specific substances express a sufficient sensitivity and specificity in order to ensure a reliable detection.

The proteins or peptides are preferably selected from the group consisting of antibodies, cytokines, lipocalins, receptors, lectins, avidins, lipoproteins, glycoproteins, oligopeptides, peptide ligands and peptide hormones. More preferably, antibodies are used as specific substance.

The term "nucleic acid" refers to a natural or synthetic polymer of single- or doublestranded DNA or RNA alternatively including synthetic, non-natural or modified nucleotides, which can be incorporated in DNA or RNA polymers. Each nucleotide consists of a sugar moiety, a phosphate moiety, and either a purine or pyrimidine residue. The nucleic acids are preferably single or double stranded DNA or RNA, primers, antisense oligonucleotides, ribozymes, DNA enzymes, aptamers and/or siRNA, or parts thereof. The nucleic acids can be optionally modified as phosphorothioate DNA, locked nucleic acid (LNA), peptide nucleic acid (PNA) or spiegelmer.

It is a preferred object of the present invention to use nucleic acid probes specifically hybridizing under stringent conditions with genes GLS2, IER5, TMEM194, PROCR, ITGA2B, FADS3, STMN3, PIB5PA, ROBO3, EDA2R and KIF1A, or preferably gene products encoded by said genes, or respective parts thereof, for detecting genotoxic and/or pro-genotoxic activity. A "nucleic acid probe" is a nucleic acid capable of binding to a target nucleic acid or complementary sequence through one or more types of chemical bond, usually through complementary base pairing by hydrogen bond formation. As used herein, a probe may include natural (i.e. A, G, C, or T) or modified bases (e.g. 7-deazaguanosine, inosine, etc.). In addition, the bases in a probe may be joined by a linkage other than a phosphodiester bond, so long as it does not interfere with hybridization. It will be understood by one of skill in the art that probes may bind target sequences that lack complete complementarity with the probe sequence depending upon the stringency of the hybridization conditions. The probes are preferably directly labeled with isotopes, e.g. chromophores, luminphores or chromogens, or indirectly labeled with biotin to which a streptavidin complex may later bind. By assaying the presence or absence of the probe, one can detect the presence or absence of a target gene of interest. Particular preferred nucleic acid probes to be used as genotoxicity-specific substances are oligonucleotide probes.

The specific substances can be labeled, in doing so the labeling depends on their inherent features and the detection method to be applied. For the detection of the specific incubation products, the applied methods depend on the specific incubation products to be monitored and are well known to the skilled artisan. Examples of suitable detection methods according to the present invention are fluorescence, luminescence, VIS coloring, radioactive emission, electrochemical processes, magnetism or mass spectrometry.

A labeling method is not particularly limited as long as a label is easily detected. A "labeled nucleic acid or oligonucleotide probe" is one that is bound, either covalently through a linker or a chemical bond, or noncovalently through ionic, van der Waals, electrostatic, hydrophobic interactions or hydrogen bonds, to a label such that the presence of the nucleic acid or probe may be detected by detecting the presence of the label bound to the nucleic acid or probe. In a preferred embodiment of the present invention, the nucleic acids are labeled with digoxigenin, biotin, chemiluminescence substances, fluorescence dyes, magnetic beads, metallic beads, colloidal particles, electron-dense reagents, enzymes; all of them are well-known in the art, or radioactive isotopes. Preferred isotopes for labeling nucleic acids in the scope of the invention are ³H, ¹⁴C, ³²P, ³³P, ³⁵S, or ¹²⁵I, more preferred ³²P, ³³P, or ¹²⁵I.

Yet another object of the invention relates to a gene chip comprising the defined combinations of gene pluralities according to any Table or Figure herein. In particular, the invention may be practiced as an array comprising nucleic acid probes that are capable of specifically hybridizing under stringent conditions with genes GLS2, IER5, TMEM194, PROCR, ITGA2B, FADS3, STMN3, PIB5PA, ROBO3, EDA2R and KIF1A, or preferably gene products encoded by said genes, or respective parts thereof. The invention may also be practiced as an array comprising nucleic acid probes that are capable of specifically hybridizing under stringent conditions with of Figure 1 a+b and/or Figure 2 a+b or gene products encoded by said genes or respective parts thereof. Both arrays are particularly designed to perform the inventive method for screening compounds with genotoxic and/or pro-genotoxic activity. The arrays of the invention may include an article that comprises written instructions or directs the user to written instructions for how to practice the method of the invention. The prior teaching of the present specification concerning the screening method is considered as valid and applicable without restrictions to the kit if expedient.

In the scope of the present invention, a method for screening compounds with genotoxic activity, which applies unique gene expression patterns of at least 11 genes selected from the group comprising the genes of Table 1, is provided for the first time. The present invention teaches characteristic expression fingerprints of a subset of marker genes that are associated with genotoxicity. Statistical data analysis even revealed 91 genes being most representative for the (pro-)genotoxic response. Several processes such as cellular differentiation and the complex interactive regulation of the stress and DNA damage response via the transcriptional modulators STAT1, SP1 and P53 are differentially regulated. The gene set evaluated was advantageously used to predict the genotoxic characteristics of N-nitrosodiethylamine (DEN) after its metabolic activation. DEN could be correctly classified as non-genotoxic without S9 and genotoxic in the presence of the MAS by means of its genomic signature. The data support that mechanistic profiling *in vitro* is a powerful tool compared to single endpoint detections to predict genotoxicity.

The results of the current invention demonstrate that (pro-)mutagenic compounds induce characteristic gene expression patterns in HepG2 cells. Such a genomics-based approach can be applied in the future in addition to the current standard test battery for genotoxicity helping to deal with equivocal results from different *in vitro* tests. Mechanistic profiling is of benefit during interpretation of such data, and mechanistic investigations are a powerful tool facilitating classification of genotoxic compounds. Furthermore, mechanistic data will improve chemical characterization and risk assessment for genotoxic compounds. Applying genomic profiling to early screening during pharmaceutical development helps to rank different molecules and highlight compounds with genotoxic characteristics early in development, saving costs and animals by preventing follow-up testing *in vivo.* The 91 putative marker genes found can be used in the future for the characterization of the genotoxic potential of unknown compounds. The analysis of the differential expressed genes is particularly suitable for higher throughput test systems. Thus, chemicals can be identified with an unknown mode of action and predicting their potential to exert genotoxic effects. The detection method as well as arising monitoring method of the invention can be performed in a simple and fast manner. In addition, the appropriate array is cost-efficiently produced.

When screening compounds *in vitro* and monitoring physiological or pathological conditions, the genes of Table 1, Figure 1 a+b and Figure 2 a+b are qualified as biomarkers for detecting and characterizing genotoxicity. Targeting gene products encoded by said genes is highly specific for the genotoxic activity and driven medical disorders thereof. All substance probes are characterized by a high affinity, specificity and stability as well as low manufacturing costs and convenient handling. These features form the basis for a reproducible action, wherein the lack of cross-reactivity is included, and for a reliable and safe interaction with their matching target structures.

All references cited herein are incorporated by reference in the disclosure of the invention.

It is to be understood that this invention is not limited to the particular methods, specific substances, uses and arrays described herein, as such matter may vary. It is also to be understood that the terminology used herein is for the purpose of describing particular embodiments only and is not intended to limit the scope of the present invention, which is only defined by the appended claims. As used herein, including the appended claims, singular forms of words such as "a," "an," and "the" include their corresponding plural referents unless the context clearly dictates otherwise. Thus, e.g., reference to "a substance" includes a single or several different substances, and reference to "a method" includes reference to equivalent steps and methods known to a person of ordinary skill in the art, and so forth. Unless otherwise defined, all technical and scientific terms used herein have the same meaning as commonly understood by a person of ordinary skill in the art to which this invention belongs.

The techniques that are essential according to the invention are described in detail in the specification. Other techniques which are not described in detail correspond to known standard methods that are well known to a person skilled in the art, or the techniques are described in more detail in cited references, patent applications or standard literature. Although methods and materials similar or equivalent to those described herein can be used in the practice or testing of the present invention, suitable examples are described below. The following examples are provided by way of illustration and not by way of limitation. Within the examples, standard reagents and buffers that are free from contaminating activities (whenever practical) are used. The example are particularly to be construed such that they are not limited to the explicitly demonstrated combinations of features, but the exemplified features may be unrestrictedly combined again if the technical problem of the invention is solved.

The following abbreviations are used herein:
cyclophosphamide (CPA); aflatoxin B₁ (AFB1); 7,12-dimethylbenz[a]anthracene (DMBA); N-nitrosodiethylamine (DEN); actinomycin D (ACT); etoposide (ETO); methyl methanesulfonate (MMS); theophylline (THEO); metformin (MET); metabolic activation system (MAS), cytochrome P450 monooxygenase (CYP); without (w/o)

The aim of this study was to evaluate the suitability of global gene expression profiling for the characterization and identification of mutagens and pro-mutagens *in vitro.* Illumina BeadChip arrays were used to quantify gene expression changes after treatment with three well-known mutagenic, three pro-mutagenic as well as two non-genotoxic reference compounds for a period of 24 or 48 hours. In detail, gene expression profiles of mutagenic (etoposide/ ETO, actinomycin D/ ACT and methyl methanesulfonate/ MMS), pro-mutagenic (cyclophosphamide/ CPA, 7,12-dimethylbenz[a]anthracene/ DMBA, and aflatoxin B₁/AFB1) and non-genotoxic (metformin/MET and theophylline/THEO) test compounds were generated in HepG2 cells after 24 h and 48 h of treatment using Illumina *HumanRef-8 BeadChip* arrays. For pro-mutagen testing, HepG2 cells were treated in combination with β-naphthoflavone/ phenobarbital-induced rat liver S9 homogenate fractions as a metabolic activation system (MAS), supplementing its poor metabolic capability. A comprehensive summary of the cell culture/ MAS system used and the well-known genotoxic characteristics of the compounds studied was previously published (summarized in Boehme et al., 2010, Toxicol. Lett. 198, 272-281). With regards to the test compound selection special attention was paid to choose compounds that have different mechanisms of genotoxicity. Moreover, the pro-mutagens used are metabolically activated via various cytochrome-P450 monooxygenases (CYPs). After the identification of a common gene expression signature for the aforementioned test compounds the signature was then used to predict the genotoxicity of N-nitrosodiethylamine (DEN).

### • Chemicals and cell culture media supplements

Actinomycin D (from Streptomyces sp., purity ≥ 95%), 7,12-dimethylbenz[a]anthracene (purity ≥ 95%), etoposide (purity ≥ 98%), methyl methanesulfonate (liquid, 99%), theophylline (purity ≥ 99%), 1,1-dimethylbiguanide hydrochloride (metformin, purity ≥ 97%), N-nitrosodiethylamine (liquid), β-nicotinamide adenine dinucleotide 2'-phosphate reduced tetrasodium salt hydrate (NADPH) and penicillin/streptomycin solution were purchased from Sigma-Aldrich (Taufkirchen, Germany). Cyclophosphamide (monohydrate, purity ≥ 97%) was from Calbiochem (Darmstadt, Germany) and aflatoxin B₁ from Acros Organics (Geel, Belgium). DMEM/F12, gentamicin, and sodium pyruvate were obtained from Invitrogen Corp. (Karlsruhe, Germany). Foetal bovine serum (FBS) was ordered from Hyclone (Order No. CH30160, Lot No. CRJ0454, Perbio Science, Bonn, Germany). β-naphthoflavone/ phenobarbital-induced rat liver S9 (Order No. R1081.S9, Lot No. 0710507) was purchased from Tebu-bio (Offenbach, Germany). Sodium phosphate monobasic monohydrate, sodium phosphate dibasic heptahydrate, magnesium chloride, and potassium chloride were obtained from Merck KGaA (Darmstadt, Germany).

### • Cell culture

HepG2 cells (Order No. HB-8065, Lot. 3129867, ATCC, Manassas, USA) were routinely maintained in DMEM/F12 with L-Glutamine and 15 mM Hepes supplemented with 10% (v/v) FBS, 1% (v/v) penicillin (10 kU/ml)/ streptomycin (10 mg/ml) solution, 0.1 % (v/v) gentamicin (50 mg/ml), and 1 mM sodium pyruvate at 37°C and 5% CO₂ in culture flasks. Depending on the experiment an appropriate number of cells were seeded onto plates and cells were cultured at 37°C and 5% CO₂ for 24 h prior to treatment. All experiments were performed at least three times with cells of passages 4-20.

### • Cell treatment and dose selection

Cells were seeded onto 6-well-plates (1.5 x 10⁶ cells/well) and left 24 h for adherence and then treated with different direct-acting or pro-genotoxic compounds as well as theophylline and metformin, which are non-genotoxic. DMSO 0.5 % (v/v) served as vehicle control for the experiments with direct-acting genotoxicants whereas only 0.2 % (v/v) DMSO was used for the pro-genotoxicants to avoid interference with the metabolic activation system. The doses of the test compounds were chosen according to previous cytotoxicity and P53 protein activation studies (Boehme et al., 2010, Toxicol. Lett. 198, 272-281). While the daily treatment schedule for direct-acting genotoxicants was continuous, cells were treated for 6 h only when the pro-mutagens plus S9 liver homogenate mixture was used to limit the cytotoxicity of the S9 fraction. The 6 h treatment period was followed by a washing step with culture medium and an 18 h recovery period. After the 18 h recovery period the treatment was repeated according to the description above. The treatment medium for pro-mutagens consisted of 300 µl S9 mixture and 700 µl culture media per well. The S9 mixture contained the following components and concentrations: 8 mM MgCl₂, 32.8 mM KCI, 12 mM NADPH, 124 mM phosphate buffer, and 2500 pmol/ml CYP content in the premixture corresponding to 2.4 mM MgCl₂, 9.8 mM KCI, 3.6 mM NADPH, 37.2 mM phosphate buffer, and 750 pmol/ml CYP as final concentrations in the treatment medium.

### • RNA extraction

After the treatment period, cells were rinsed with PBS (Gibco Invitrogen, Karlsruhe, Germany), harvested and total RNA was extracted using the RNeasy Mini Kit (QIAGEN, Hilden, Germany) as described by the manufacturer, including the QIAshredder spin column procedure and on-column RNase-free DNase digestion. RNA was eluted with 40 µl RNase-free water. RNA quality control and quantification was determined using a NanoDrop® spectrophotometer (Kisker, Steinfurt, Germany) and 2100 Agilent Bio-Analyzer (Agilent Technologies, Waldbronn, Germany). Only RNA with a quality ratio A₂₆₀/A₂₈₀ between 1.9 and 2.1 and no evidence of peak degradation (18s/28s) was used.

### • cRNA synthesis and Illumina whole genome chip hybridization

Gene expression analysis was executed using Illumina Sentrix® HumanRef-8 V2 BeadChip arrays (Illumina Inc., San Diego, CA, USA) allowing the analysis of ∼23,000 transcripts. Synthesis of biotin-labeled cRNA was performed in an automated procedure using a Theonyx Liquid Performer (Aviso GmbH, Greiz, Germany) and MessageAmp™ II aRNA amplification Kit (Ambion, Darmstadt, Germany) with several modifications requested by Illumina to optimize the process (Zidek et al., 2007, Toxicol Sci 99, 289-302). Instead of column cleanup, the bead-based Agencourt® RNAclean™ system (Beckman Coulter, Krefeld, Germany) was applied to purify cDNA and cRNA. cRNA quantity was measured spectrophotometrically (NanoDrop®) and the 2100 Agilent Bio-Analyzer was used for quality assessment.

Seven hundred and fifty nanograms of amplified biotinylated cRNA were hybridized onto the Illumina Sentrix® BeadChip in a Hybridization Cartridge under humidified conditions for 20 h at 58°C (Hybridization oven, Illumina Inc., San Diego, CA, USA). The chips were then washed, stained for 10 minutes with 1 µg/ml streptavidin-conjugated Cy3 (Amersham Biosciences, Buckinghamshire, UK), and finally dried by centrifugation according to the protocol provided. Fluorescence detection was carried out by confocal laser scanning with the Illumina® BeadArray Reader (Illumina, Inc., San Diego, CA, USA) at 532 nm and 0.8 µm resolution.

### • Statistical data analysis

Illumina® BeadStudio Software was used for condensing raw data and further to ensure array quality based on different control bead parameters as described for a previous study (Boehme et al., 2009, Toxicol. Appl. Pharmacol. 236, 85-96). Thereafter, data were uploaded into Genedata's Expressionist® Analyst software (Genedata AG, Basel, Switzerland) for data normalization and statistical analysis. Data were normalized using Lowess (Locally Weighted Linear Regression) for mutagen experiments and on a median signal intensity of 100 for pro-mutagen studies to offset non-biological differences (systematic variation) between the samples and arrays. After normalization fold-regulations were calculated for each individual compound treatment against the corresponding vehicle control samples with and without S9, respectively. Transformation to relative values was imperative to achieve comparability of the data from different studies.

For identification of characteristic genes, representing the common mutagenic mode of action of the compounds tested, a training data set was used which constituted the (pro-)genotoxic group and theophylline and metformin as non-genotoxic compounds. DEN was used as an "unknown" test compound and therefore, excluded from the training set. The most predictive genes were identified by gene ranking with an ANOVA for group separation followed by support vector machine (SVM) algorithm for classifier calculation. The predictivity was evaluated by *k*-fold cross validation (*k*=10, repetition 50 times) and the classifier built from the training set was then used to predict the toxicity of DEN.

For investigation of compound-specific differentially expressed genes (S9 and DEN effects), gene expression profiles were individually compared against the vehicle control (for evaluation of S9 effects) and reference compounds (for evaluation of DEN effects) by Student's T-Test or analysis of variance (ANOVA), respectively. BH-q-values (Benjamini and Hochberg false discovery rate) were used as a significance measure in addition to the p-value (Benjamini and Hochberg, 1995, J.R. Statist. Soc. 57, 289-300).

Following statistical analysis, data were interpreted in a biological/ functional context using databases and pathway analysis tools from GeneGo (Metacore™), Ingenuity (IPA^{®}), and Cambridge Cell Networks (ToxWiz). All data were recorded in compliance with MIAME (Minimum Information About a Microarray Experiment) recommendations including detailed experimental/data analysis descriptions and are available as supplementary information.

Table 1 lists the deregulation values and ranking statistics of the 91 putative marker genes identified for the genotoxic and pro-genotoxic test compounds (classification model). HepG2 cells were treated with 7,12-dimethylbenz[a]anthracene (DMBA), diethylnitrosamine (DEN), cyclophosphamide (CPA) and aflatoxin B1 (AFB1) as well as theophylline (THEO) and metformin (MET) as controls daily for 6 h followed by 18 h recovery over a total period of 48 h in the presence and absence of a metabolic activation system (ß-naphthoflavone/ phenobarbital-induced S9). In contrast to the pro-genotoxic treatments cells were exposed continuously with direct-acting genotoxicants over a period of 24 h and 48 h. Cell culture media containing the test compound was aspirated and replaced every day by fresh treatment media. The 91 top-scored genes were identified by gene ranking with an ANOVA for group separation followed by support vector machine (SVM) algorithm for classifier calculation. Data listed in the current table represent mean values of the gene regulations relative to the corresponding vehicle/S9 controls from the three different experiments with cell passages ranging from 3 to 25. ¹Experimental part pro-genotoxic compounds, ²Experimental part genotoxic compounds, ³Rank number of the ANOVA ranking statistics, ⁴General regulation tendency in (pro-)genotoxic samples.

Table 2 lists the deregulation values of selected genes, which displayed consistent regulations in response to the treatment with (pro-)genotoxic model compounds. The table contains the deregulation values of selected genes from the classification model based on Illumina microarray data. All eight genes were significantly induced (FC ≥ 1.5-fold (shown in orange) in at least 80 % of responsive samples group positive genotoxic samples) in HepG2 cells after 48 h treatment with (pro-)genotoxic model compounds. Data listed in the table represent mean values of the gene regulations relative to the corresponding vehicle/S9 controls from the three different experiments with cell passages ranging from 3 to 25. Legend: actinomycin D (ACT), methyl methanesulfonate (MMS), etoposide (ETO), aflatoxin B1 (AFB1), 7,12-dimethylbenz[a]anthracene (DMBA), cyclophosphamide (CPA), diethylnitrosamine (DEN). *Significance determined by Student's t-test, p-value < 0.05.

Table 3 lists categorization of the test samples for the evaluation of the common genotoxic response. Class allocation was based on compound class as well as P53 and gene expression analysis of single compounds. ^{#} Samples were categorized as genotoxic due to significant P53 activation and marked gene expression changes compared to the vehicle controls. The positive response without a MAS is caused by the metabolic competency of HepG2 cells as shown previously (Boehme et al., 2010, Toxicol. Lett. 198, 272-281).

Figure 1 a+b shows the gene-function-heatmap of putative marker genes in HepG2 cells treated with genotoxic compounds. Gene regulations and functions of the genes which were significantly deregulated (ANOVA p-/ BH-q-value < 0.01) by more than 1.5-fold up (red) or down (green) in response to 48 h treatment with actinomycin D (ACT), methyl methanesulfonate (MMS) and etoposide (ETO). Treatment concentrations were 500 nM for ETO, 500 µM (nd = low dose) and 2 mM (hd = high dose) for MMS as well as 250 nM for ACT. Theophylline (THEO) at a dose of 100 µM served as negative control. Cells were treated daily for a period of 6 h, 24 h and 48 h following gene expression analysis using Illumina microarrays [figure has been modified according to Genedata's Expressionist® Analyst, Basel/ Switzerland].

Figure 2 a+b shows the characteristic gene regulations of pro-genotoxic compounds and functional categorization of these genes. HepG2 cells were treated with 7,12-dimethylbenz[a]anthracene (DMBA), diethylnitrosamine (DEN), cyclophosphamide (CPA) and aflatoxin B₁ (AFB1) as well as theophylline (THEO) and metformin (MET) as controls daily over a total period of 48 h in the presence and absence of a metabolic activation system (ß-naphthoflavone/ phenobarbital-induced S9). Experiments were repeated three times with different cell passages followed by gene expression quantification using Illumina Human Ref-8 BeadChip arrays. (A) HeatMap displaying relative expression values of the 88 genes referred to the vehicle controls. Genes were identified by means of an ANOVA (p-value < 0.01/ BH-q-value < 0.3) in combination with a filtering approach applying a deregulation cutoff of 1.5-fold in at least 40% of the samples, which showed a positive genotoxic response. Up-regulated genes (≥ 1.5-fold) are shown in red and down-regulated genes in green (≤ -1.5-fold). The regulation bar is scaled logarithmically. (B) Functional categorization of the genes using MetaCore™ (GeneGo, St. Joseph/ USA) and ToxWiz (Cambridge Cell Networks, Cambridge/ UK) databases. (C) Deregulated genes directly associated with P53 are displayed as network objects. The table below summarizes genes involved in the whole P53 signaling cascade. Regulation bars indicate gene expression values from the different treatments ordered analogously to the header of the HeatMap (A) [figure has been modified according to Genedata's Expressionist® Analyst (A) and ToxWiz (C)].

Figure 3 shows the gene ranking with the training data set to evaluate a convenient classification algorithm and identify the most appropriate genes for compound classification. For building the model a training data set was built out of the (pro-)genotoxic group: Actinomycin D, methyl methanesulfonate and etoposide treatments at 24 h and 48 h were chosen as representatives for the direct-acting genotoxicants. In contrast to that 48 h treatments of the pro-genotoxins 7,12-dimethylbenz[a]anthracene (DMBA), diethylnitrosamine (DEN), cyclophosphamide (CPA) and aflatoxin B₁ (AFB1) were used exclusively. However, CPA -S9 as well as AFB1 0.5 µM -S9 and DMBA 10 µM -S9 were categorized as non-genotoxic due to the lacking or extremely weak gene expression response. Theophylline (THEO), metformin (MET) and the S9 controls were also grouped together with the non-genotoxins. An ANOVA was applied as gene ranking method in combination with three different classification algorithms: Support Vector Machine (SVM) is shown in blue, K Nearest Neighbors (KNN) is presented by the green graph and Sparse Linear Discriminant Analysis by the red line. A linear Kernel and a Penalty factor of 10 were used as parameters for the SVM method. KNN was run with positive correlation as distance measure and k was set to 4. The predictivity was evaluated by k-fold cross validation (k=10, 50 repetitions). SVM revealed the lowest misclassification rates in dependence of the number of genes used for classifier calculation. A minimal misclassification rate of 4.7 % was achieved with the 91 top-scored genes. Gene ranking was carried out using the software package Expressionist® Analyst from Genedata (Basel/ Switzerland).

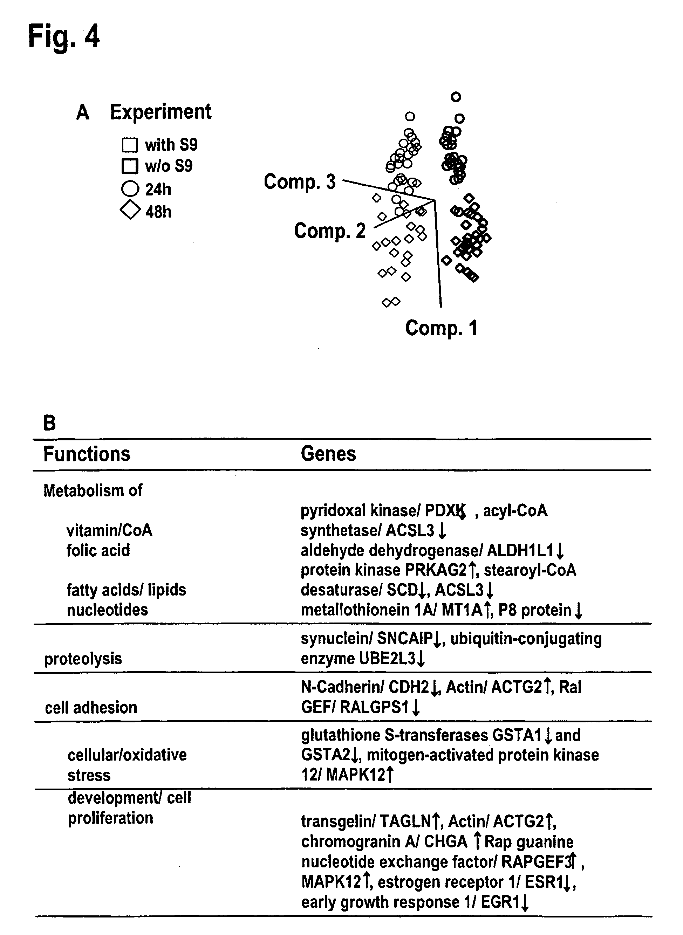

Figure 4 (A) shows principal component analysis (PCA) of the whole genome expression data from the pro-genotoxic and non-genotoxic test compounds. Illumina expression data were normalized on median signal intensity before subjecting the data to a PCA. The most important effectors on cluster separation were treatment time (circles: 24 h, rhombuses: 48 h) and the metabolic activation system (MAS/ rat liver S9: black symbols; without MAS: white symbols) used. Figure (B) shows examples of differentially regulated genes in HepG2 cells in response to S9 exposure (ANOVA p-/BH-q-value < 0.05 and fold-change ≥ 1.5/ ≤ - 1.5 after 24 and/or 48h treatments) and corresponding gene functions.

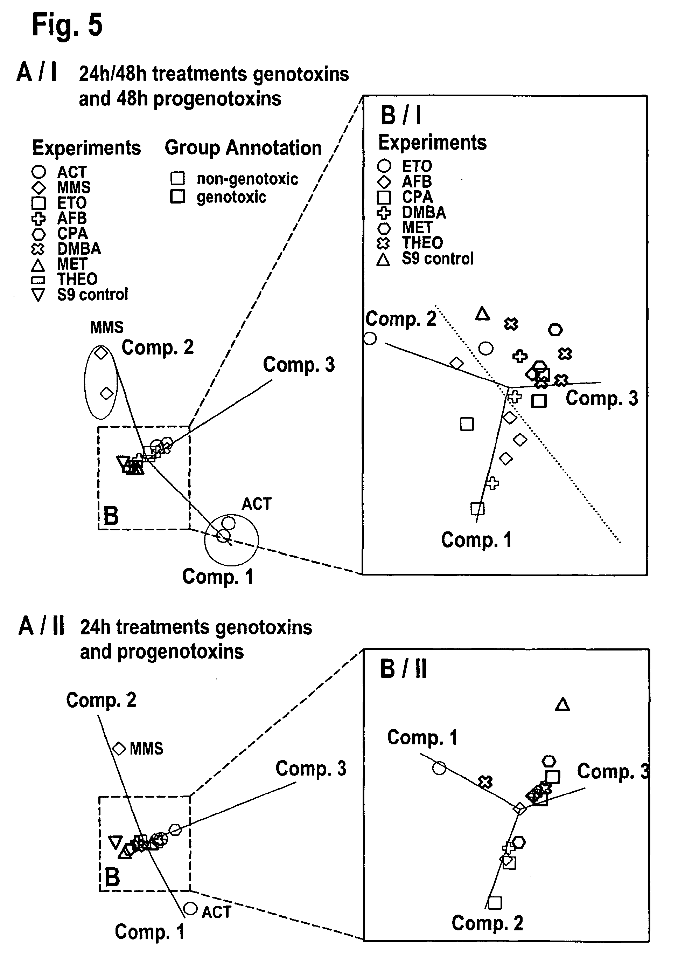

Figure 5 (A/I and B/I) shows PCAs of the whole genome expression data from the (pro-)genotoxic (black symbols) and non-genotoxic (white symbols) training samples (detailed information on group allocation is provided by Table 3). The data shown are relative values calculated against the appropriate vehicle/ vehicle-S9 controls. While PCA A/I contains all genotoxic and pro-genotoxic test samples, B/I represents a zoom into the cluster near the coordinate origin of the three separating components. The dotted line indicates an imaginary separator between the (pro-)genotoxic and non-genotoxic samples. Compared to the analyses A/I and B/I, which comprise 24 h and 48 h data of the genotoxic but 48 h data of the pro-genotoxic test compounds only, A/II and B/II show the corresponding data after 24 h treatment with (pro-)genotoxicants and non-genotoxicants. Random assembly of the samples within PCA B/II illustrates the overlapping, still largely similar profiles between controls/ non-genotoxicants at this early time point.

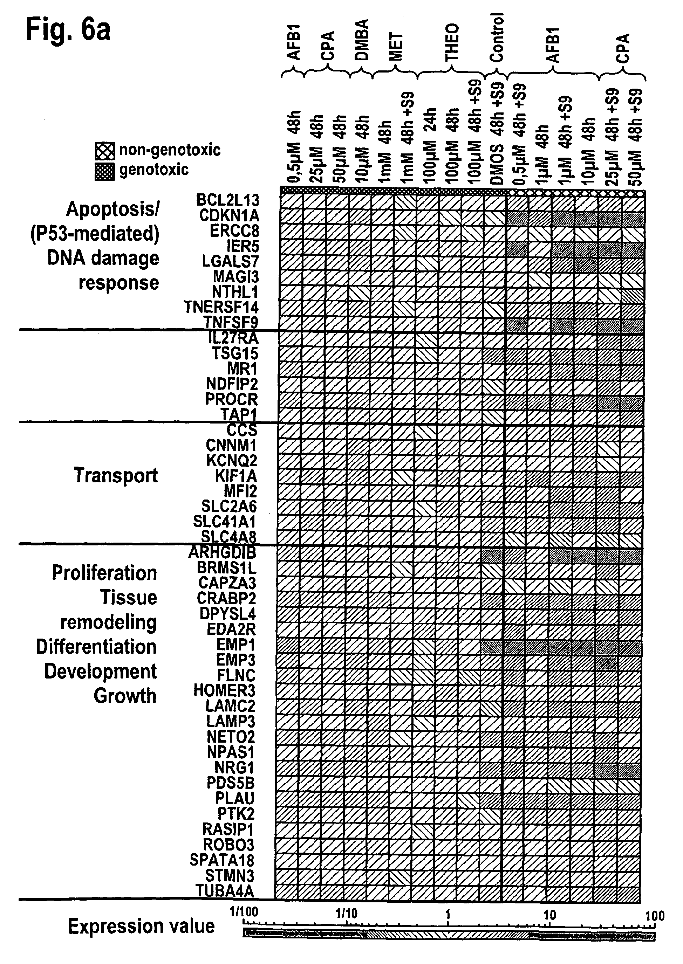

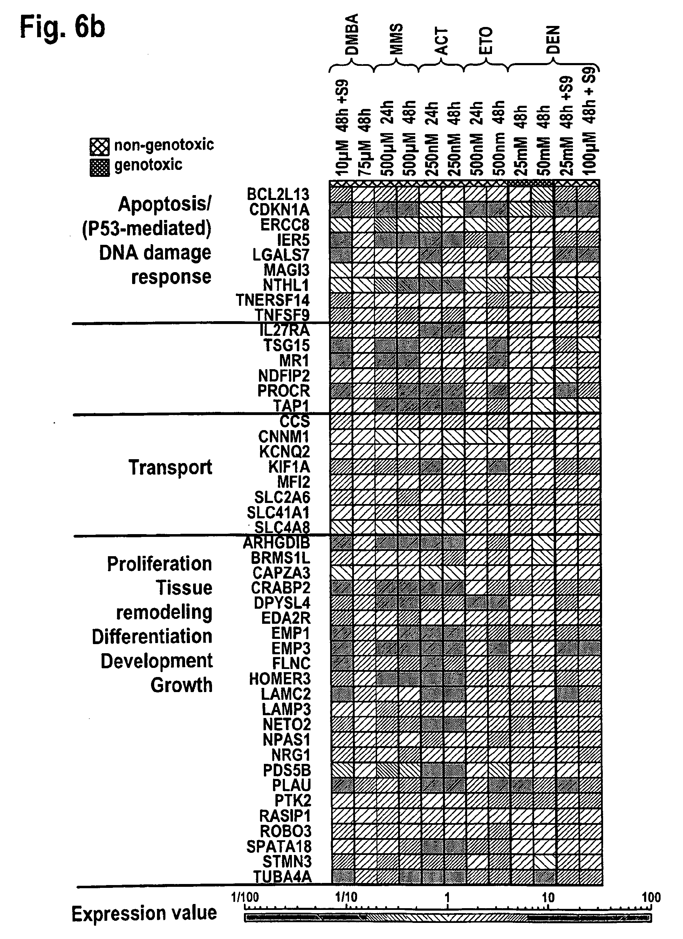

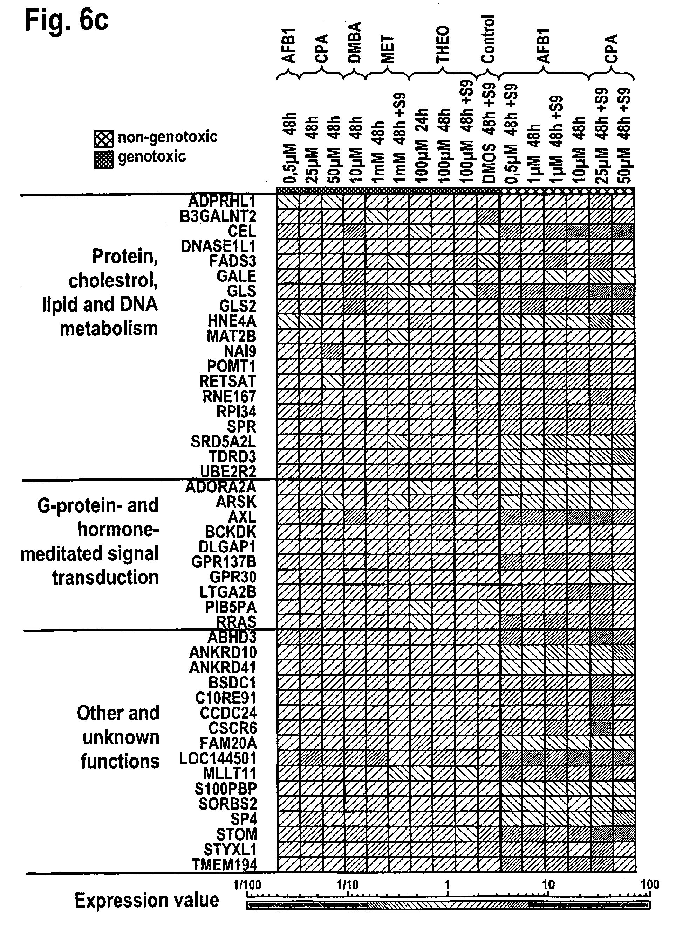

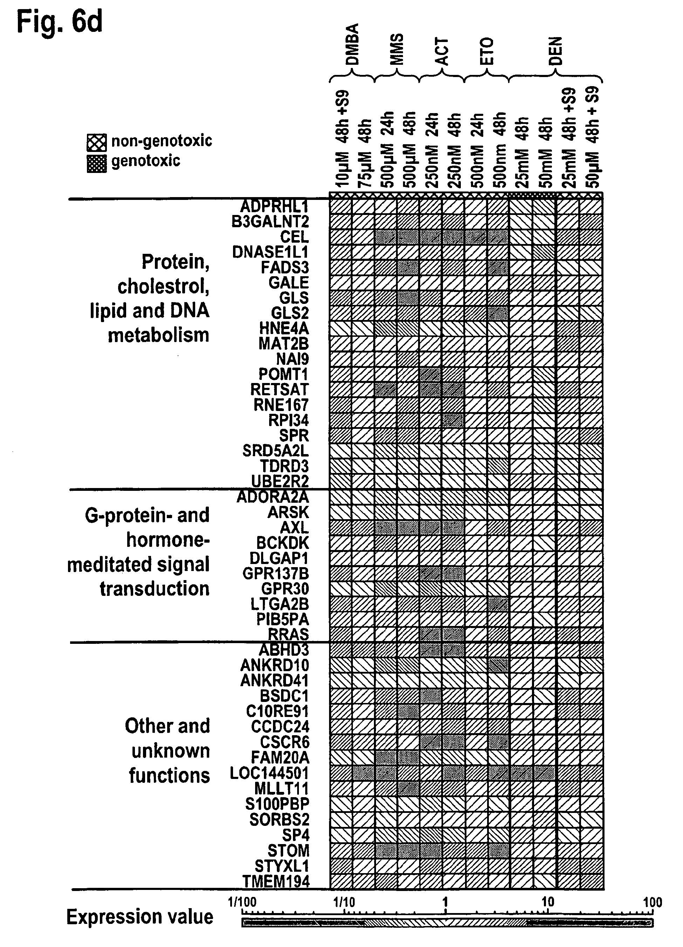

Figure 6 a-d shows gene function profile display of differentially regulated genes in HepG2 cells after treatment with various (pro-)genotoxic (yellow bar) and non-genotoxic (blue bar) compounds for 24 and/ or 48 h. Responses to the test compounds were analyzed using Illumina arrays. The 91 genes, which are shown in the heatmap, were identified to be most predictive by gene ranking with an ANOVA for group separation followed by support vector machine algorithm for classifier calculation. The misclassification rate, which was evaluated by *k*-fold cross validation (*k*=10, repetition 50 times), only accounts for 0.25% using this gene set. Gene expression regulations of the 91 genes are also given for DEN, which was not part of the training set. The color scale corresponds to fold-change in gene expression: up-regulated genes are shown in red, down-regulated genes in green, and genes not regulated in black.

Figure 7 shows putative regulation and interaction of the genes within DNA damage response. Arrows indicate the general regulation tendency, up- (↑) and down (↓)-regulation, respectively. While genes displayed without parentheses can be found under the 91 characteristic genes for the (pro-)genotoxic test compounds, genes surrounded by parentheses were regulated more than 1.5-fold by some test compounds only. Underlined genes are known to be controlled by the key transcriptional mediators STAT1, P53, or SP1, which were identified by means of target gene regulations in the Illumina gene expression profiling experiments.

Figure 8 shows potential mechanisms of P53 induction and signaling by genotoxic test compounds. Accumulation of P53 following methyl methanesulfonate (MMS)-induced DNA damage is suggested to be mediated by the components of the base excision repair (BER) and inhibition of the P53 inhibitor APAK via ATM and checkpoint kinases activated after the DNA damage. The latter mechanism is postulated to be responsible for etoposide (ETO)-induced P53 induction in addition to the topoisomerase II (TOPO2) inhibitive characteristics. In contrast, inhibition of RNA polymerase II seemed to be mainly responsible for P53 accumulation by actinomycin D (ACT). Moreover, inhibition of the TOPO2, as well as the expression of a non-functional mdm2 variant (Mdm2*), may also play a role in P53 activation by ACT. Arrows indicate the regulation tendency of the genes in Illumina microarrays (↑/ ↓= up-/down-regulation by a fold-induction ≥ 1.5-fold).

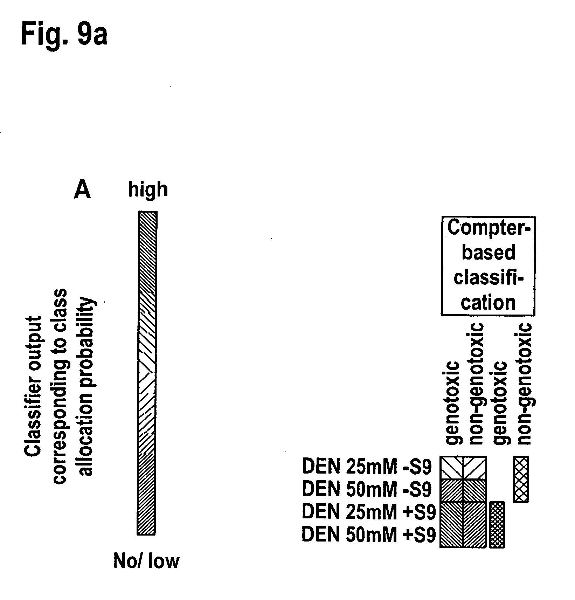

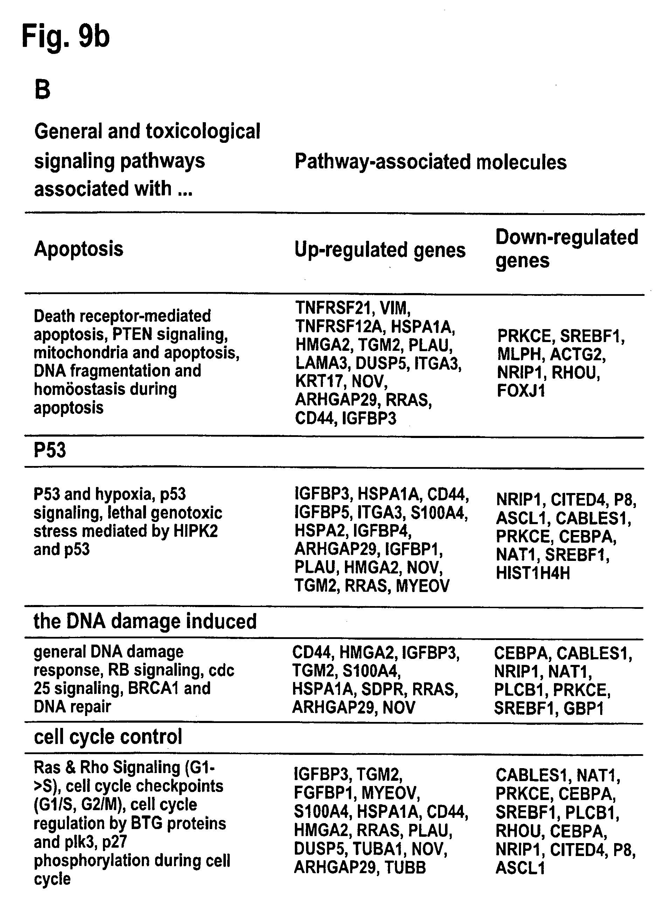

Figure 9 a+b shows gene expression pattern of N-nitrosodiethylamine (DEN). (A) Classification of DEN as genotoxic with S9 and non-genotoxic without an external metabolic activation system using the 91 gene-classifier calculated from training set by gene ranking combined with a SVM algorithm. (B) Significantly deregulated genes (ANOVA p-value/BH-q-value < 0.01 and fold-change more than 1.5-fold in at least 40% of the 48 h DEN samples) functionally involved in the response to the DEN-induced DNA toxicity.

### EXAMPLE 1: Parameters influencing global gene expression

In the current invention, gene expression patterns of well-known genotoxic, pro-genotoxic and non-genotoxic compounds were investigated. Initially, parameters influencing gene expression were identified by means of principal component analysis (PCA). PCA revealed an effect of time and a marked effect of the metabolic activation system on the gene expression of the test compounds used (Figure 4A), while different biological replicates (cell passages) had no separating influence on global data distribution. The analysis of the S9-induced gene expression revealed 164 significantly genes deregulated by more than 1.5-fold after 24 h and/or 48 h treatment (ANOVA of both time points against the DMSO controls, p-/ BH-q-value < 0.05). One hundred and fifty of these genes were found to be down-regulated and 14 up-regulated. Seventy seven percent of the down-regulated genes showed similar regulations after 24 h and 48 h. Genes found to be differentially regulated after S9 exposure were involved in processes such as vitamin/CoA, folic acid, fatty acid/lipid, and nucleotide metabolism as well as proteolysis, cell adhesion, the response to cellular/oxidative stress, and genes regulating development/ cell proliferation (Figure 4B).

### EXAMPLE 2: Gene expression patterns of (pro-)genotoxic and non-genotoxic compounds

The substantial influence of the MAS on the gene expression signatures was a challenge because any specific substance effects were masked. Therefore, data were converted to relative values by reference to their vehicle/ vehicle-S9 control in the same cell passage to make them independent from the experiment and adjust the substance-mediated effect by the S9-mediated one. Figure 5 shows that this type of correction puts forth the substance effect and genotoxic and non-genotoxic compounds are now clustered separately. Due to marked gene expression changes after treatment with ACT and MMS cluster separation was difficult to fully resolve (Figure 5A/I). However, if ACT and MMS were excluded from the PCA (zoom in) the clear separation of genotoxic from non-genotoxic compound classes became visible (Figure 5B/I). In contrast, no clear separation was visible for the pro-genotoxic compounds after 24 h treatment (Figures 5A/II and 5B/II). Statistical analysis of single compounds (paired two-tailed T-Tests against the appropriate vehicle controls) confirmed this negative response after 24 h. Moreover, CPA w/o S9, AFB1 0.5 µM w/o S9 and DMBA 10 µM w/o S9 all caused only extremely weak or no statistically significant gene expression changes, respectively. Therefore, these samples were categorized as non-genotoxic together with MET and THEO. In general, the classification of the compounds for further analysis was based on a combination of the compound class, P53 (Boehme et al., 2010, Toxicol. Lett. 198, 272-281) and gene expression assessments from single compound analysis as listed in Table 3.

In Figure 1 a+b and Figure 2 a+b, data analysis has been separately performed for the corresponding class of compounds, i.e. Figure 1 a+b relates to genotoxic compounds having a direct mode of action, whereas Figure 2 a+b relates to pro-genotoxic compounds that are effective after metabolic activation. In Figure 6a-d and Table 1, a classification model has been established with both data sets of Figure 1 a+b and Figure 2 a+b. Since both classes (whether acting directly or indirectly) are genotoxic, they were assigned to a single group and compared with control compounds. DEN was negative with regards to P53 activation and was therefore excluded from the data set and hence, the training data set without DEN was used to build a molecular classifier to identify the most predictive genes for (pro-)genotoxic compounds. An ANOVA-based gene ranking following a SVM algorithm-based classifier determination method, revealed the best predictivity using the 91 top-scored genes. Regulations of these genes and functional categorization are shown in Figure 6a-d in a function-heat-map view. The results corroborate the group allocation of genotoxic and non-genotoxic samples from the previous experiments. CPA w/o a MAS did not differentially regulate the 91 genes, while with S9 a marked up- and down-regulation could be observed. Moreover, while the lowest dose of AFB1 (0.5 µM) w/o S9 did not change the gene expression of the 91 genes compared to the control, higher doses or the addition of S9 at lower doses lead to an altered response within the expression of the selected genes. A similar gene regulation profile was observed for DMBA, whereby gene expression changes at 75 µM w/o S9 were relatively weak compared to the genotoxic response of the other test compounds. Moreover, ETO's response was also weak after 24 h, but stronger after 48 h treatment. DMBA (75µM) w/o S9 and ETO (after 24 h) were thus the restricting compounds for prediction and were only predicted correctly when using the 91 top-scored genes, but not if using all genes, because the background of non-differentially regulated genes was extremely high compared to the deregulated genes. The results were verified by determining the regulation of 11 out of the 91 genes and 32 out of the 91 genes (Figure 3).

### EXAMPLE 3: Genomics-based classification of N-Nitrosodiethylamine

With the aid of the 91 genes found to be most predictive for the (pro-)genotoxic test compounds the gene expression response of DEN was evaluated. In contrast to previous P53 activation experiments, where no reproducible positive response could be established, DEN was correctly predicted to be genotoxic in the presence of S9 by means of gene expression analysis (Figure 9A). This prompted us to further analyze DEN-specific gene expression. Statistical analysis (ANOVA against the non-genotoxic compounds MET and THEO, p-value/ BH-q-value < 0.01) revealed 88 up- and 57 down-regulated genes, which were above the deregulation cut-off of at least 1.5-fold in more than 40% of the 48 h DEN samples. The cut-off criteria were oriented to the 48 h samples because of the lack of or weak response at 24 h treatments as mentioned for the other test compounds. The general toxicological pathways identified, clearly reflected the cellular stress situation and the genotoxic response after exposure of the cells to DEN (Figure 9B). Especially for the latter, many pathways mediating - among others - apoptosis, cell cycle checkpoints/ arrest, and DNA repair as well as genes involved in P53 signaling directly, were identified to be similar to present other test compounds. Moreover, if the regulation pattern of the DEN-induced genes, comprising the 145 genes from single analysis as well as the 91 putative marker genes, is compared, similar regulation could be detected without S9 addition although expression values were often higher in the presence of S9. Thus, the data suggest an insufficient metabolic activation or detoxification, leading to an increased genotoxic effect, already at lower doses of DEN with the MAS. Metabolic activation by the HepG2 cells themselves is rather unlikely because it has been demonstrated previously that HepG2 cells do not sufficiently express CYP2A6 and CYP2E1 (Boehme et al., 2010, Toxicol. Lett. 198, 272-281) representing the main enzymes that convert DEN to the ultimate genotoxic metabolite. Apparently, the addition of an external MAS activates DEN only partially, however, while weak changes were recorded by means of gene expression analysis, activation is presumably not efficient enough or detoxification to pronounced to evoke a stable, detectable P53 response.

In fact, the difficulty of screening nitrosamines has been described several times in the literature. Although DNA reactivity and carcinogenicity *in vivo* is generally accepted for this compound class (most nitrosamines are IARC group 2A and 2B classified as potential human carcinogens), different *in vitro* assays often only reveal weak or even negative results. While screening difficulties with nitrosamines in cell lines were often explained by a lack of CYP2E1 activity, the study do not favor restricted phase I metabolism as the major reason. A positive response for DEN was demonstrated in alkaline elution/ UDS assay using CYP competent primary hepatocytes at very high concentrations only (10-32 mM). Moreover, β-naphthoflavone/ phenobarbital-induced S9 was tested, but also isoniazide (CYP2E1)-induced microsomes. Cytotoxicity and P53 activation studies revealed no difference between both MASs suggesting another cause of the missing response. HepG2 cells do indeed have phase II metabolic capability. Phase II biotransformation of nitrosamines takes place by conjugation with glutathione, amino acids, sulphuric acid or glucuronides. A typical conjugation reaction of nitroamines to glucuronides is catalyzed by UDP glucuronosyltransferases (UGTs). Although basal expression was weak in HepG2 cells, selected UGT1A enzymes could be induced by rifampicin or 3-methylcholanthren. Moreover, the ATP transporters MRP1 and MRP2 have been shown to be partially induced and are probably involved in the excretion of nitrosamines. Beside phase II metabolism, the volatile character of the diazonium ion may also be responsible for the controversial results *in vitro.*

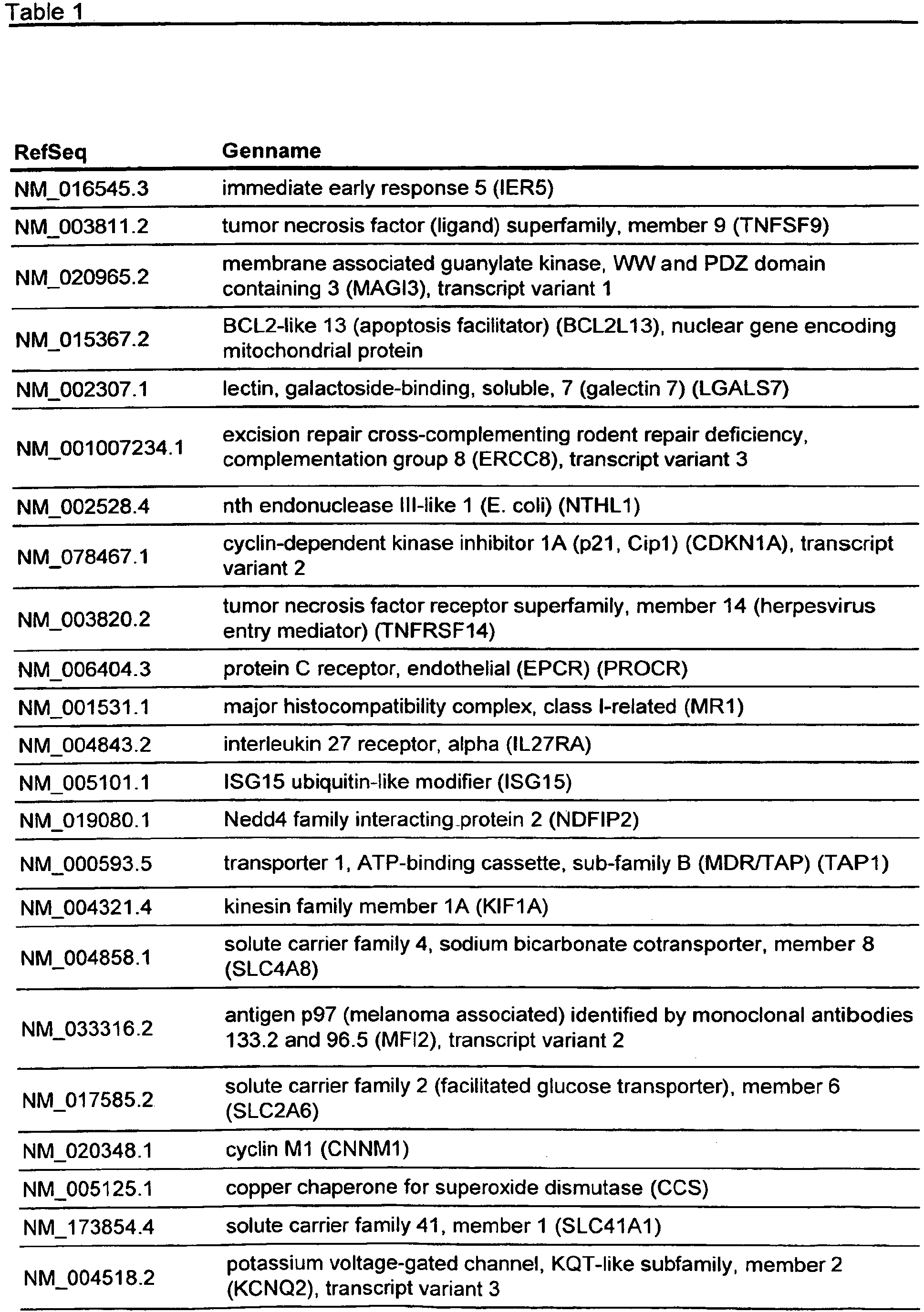

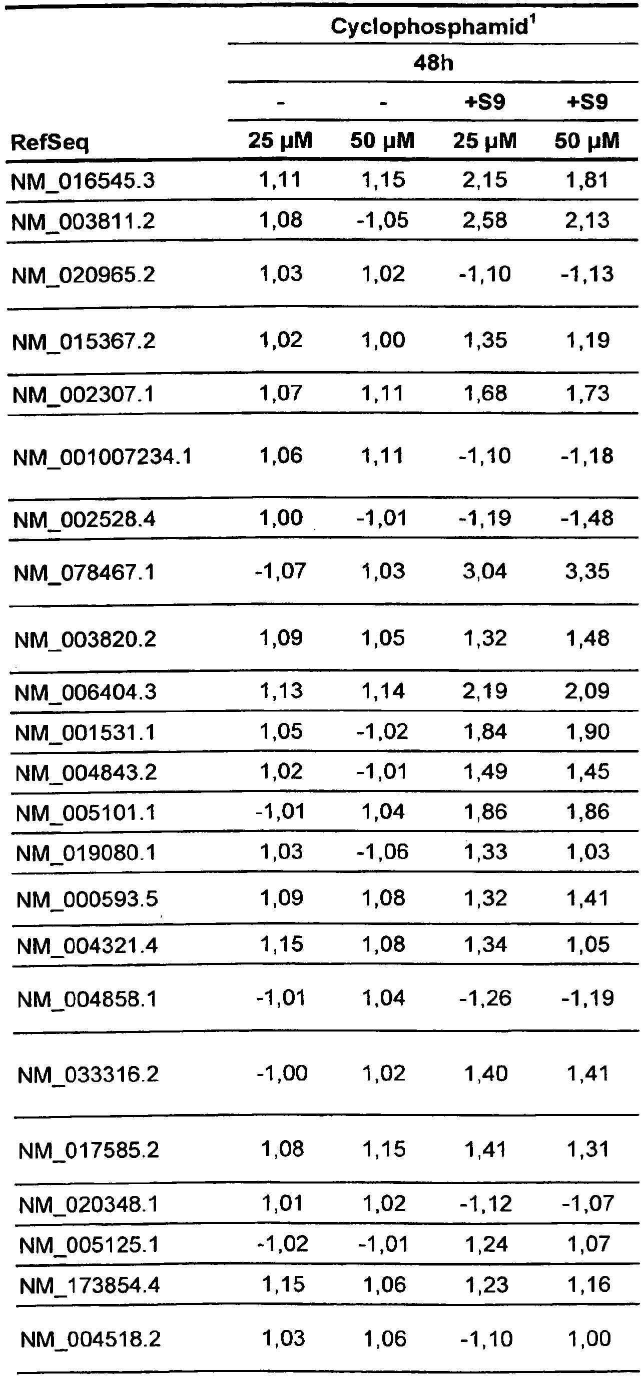

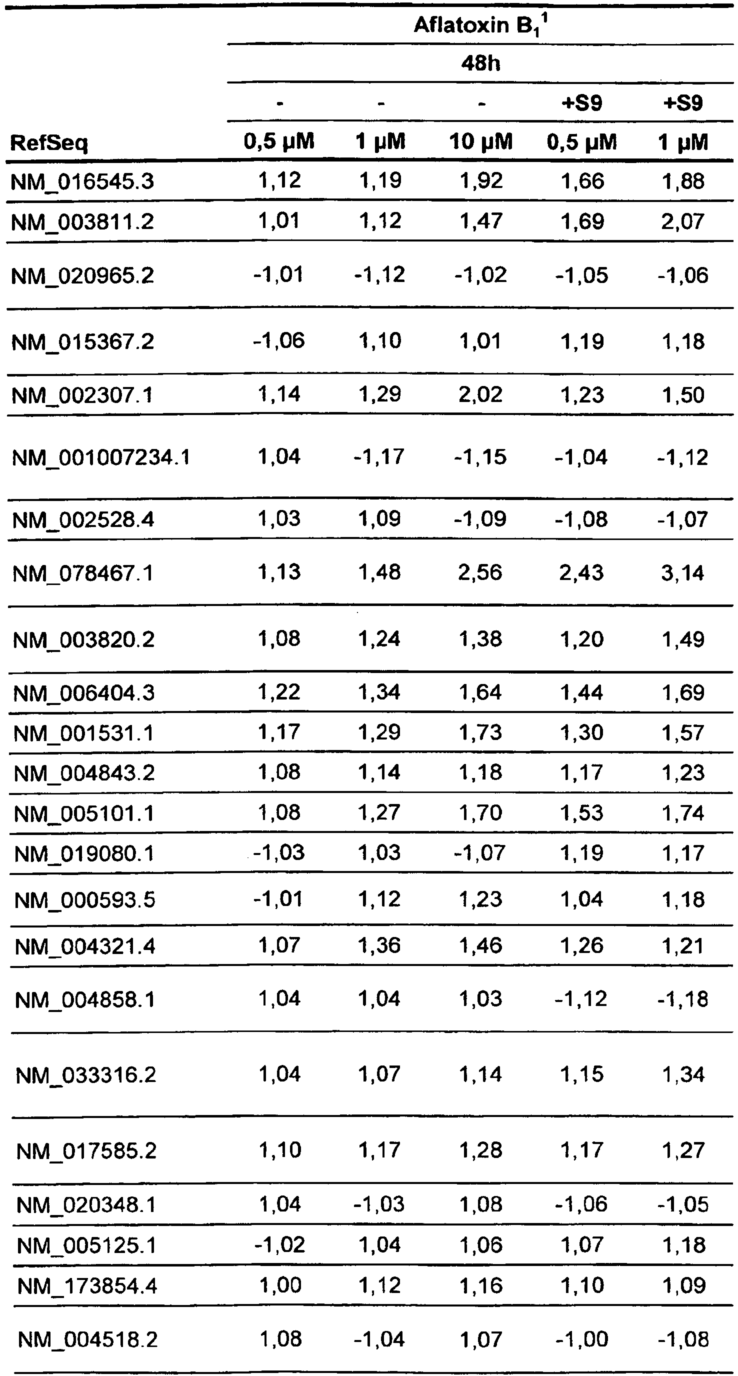

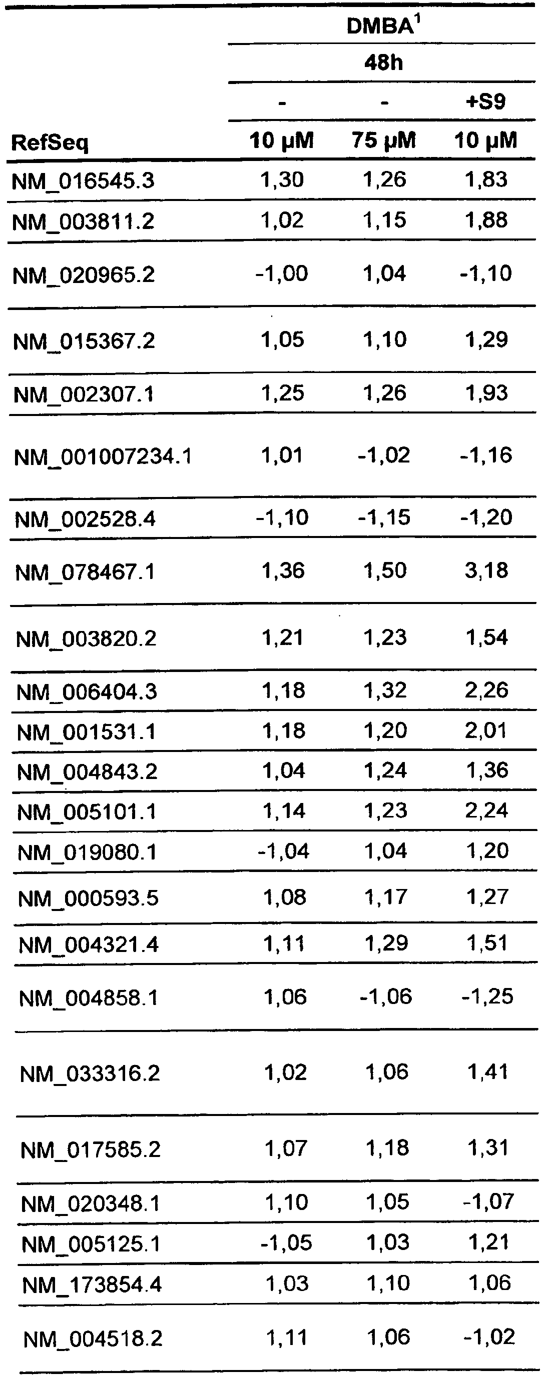

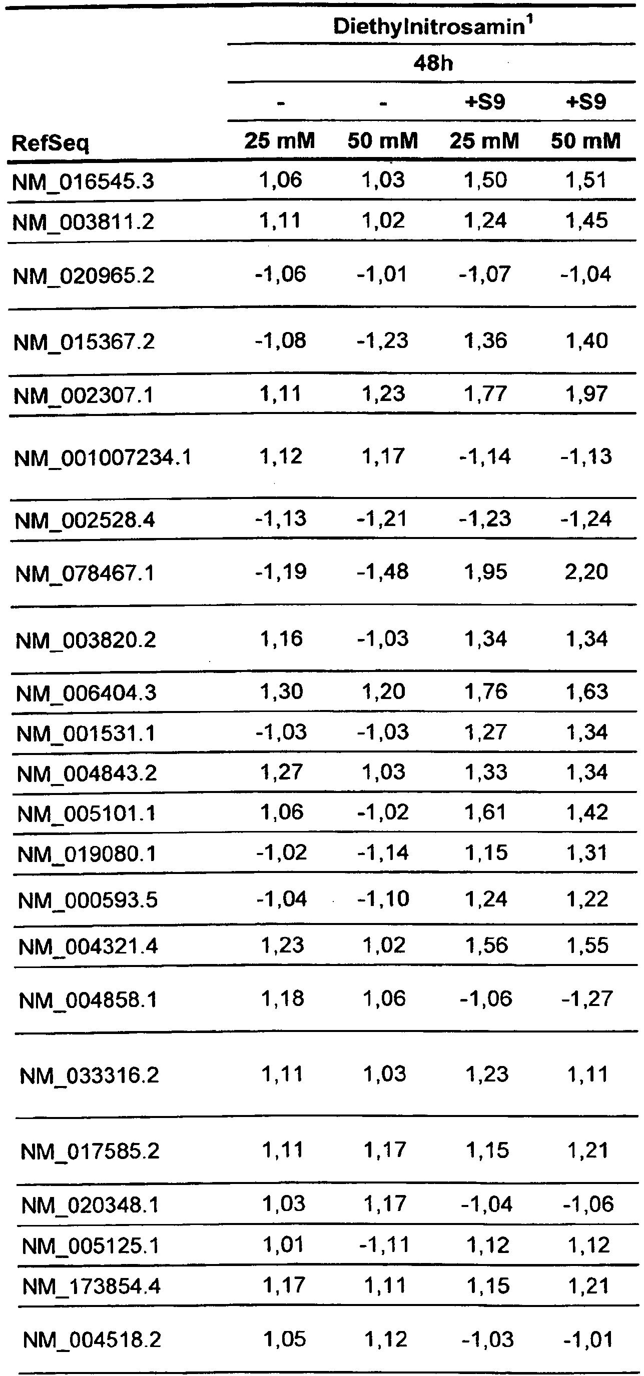

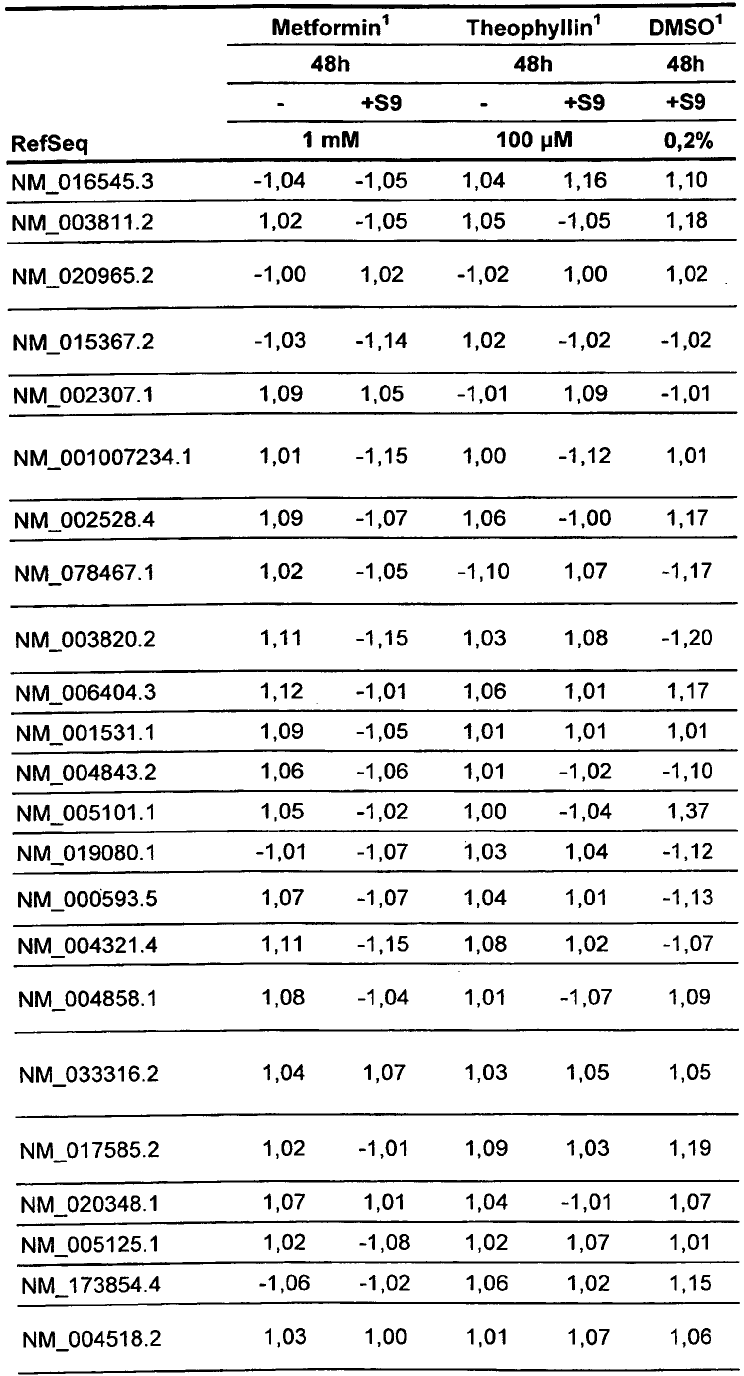

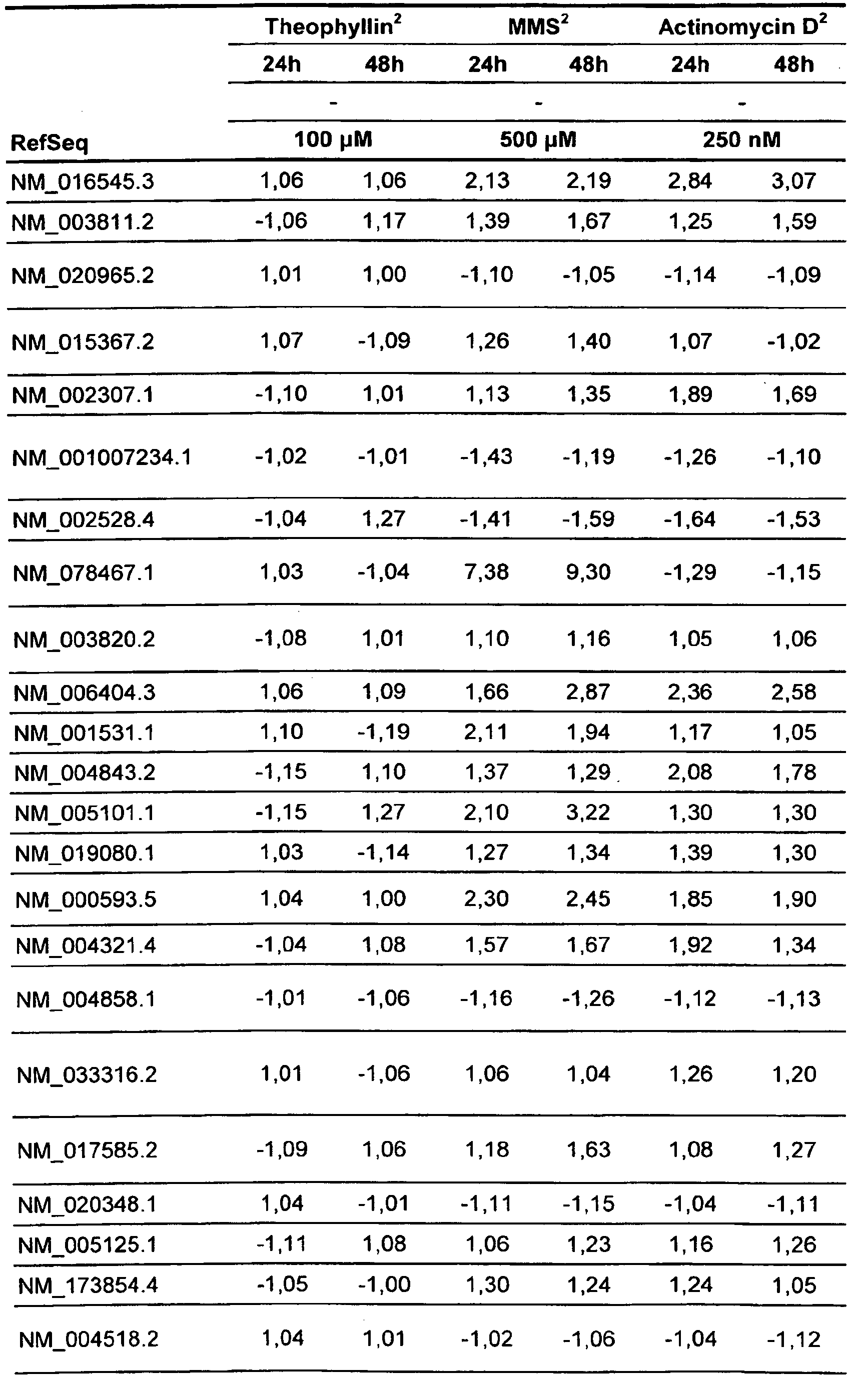

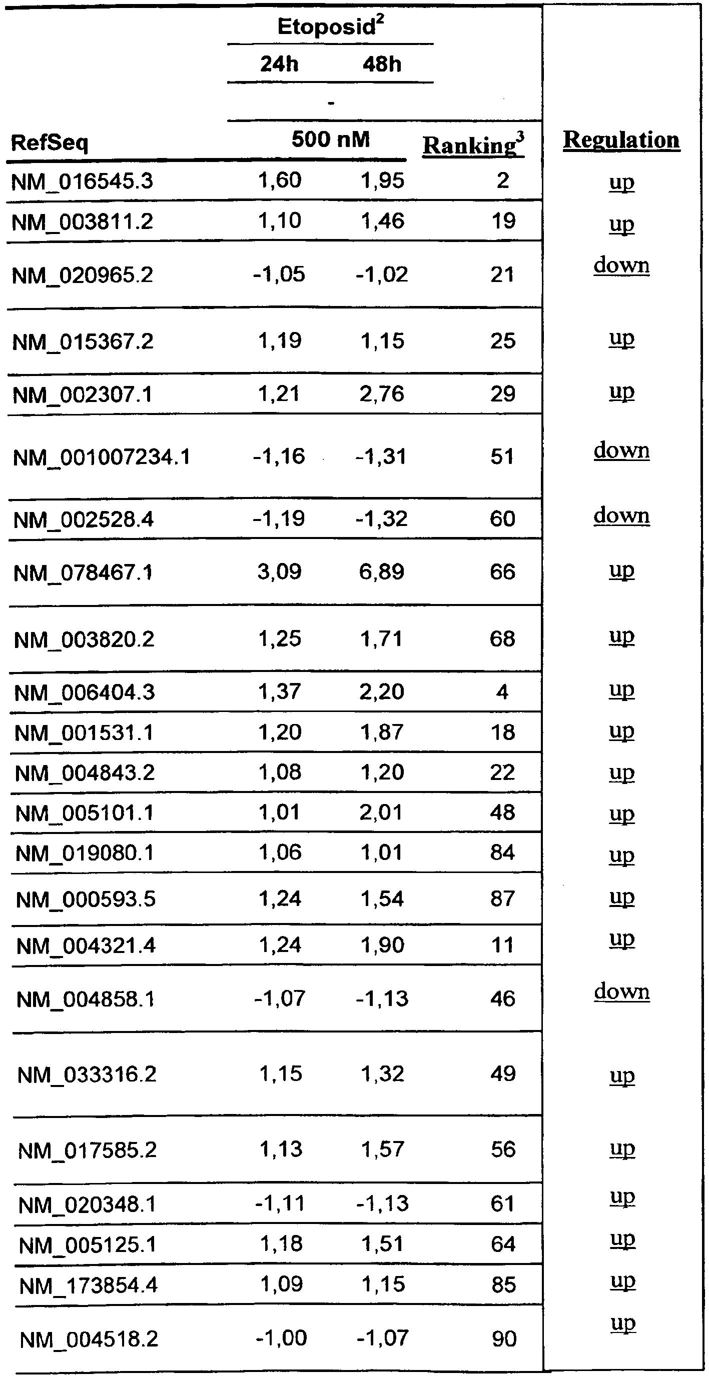

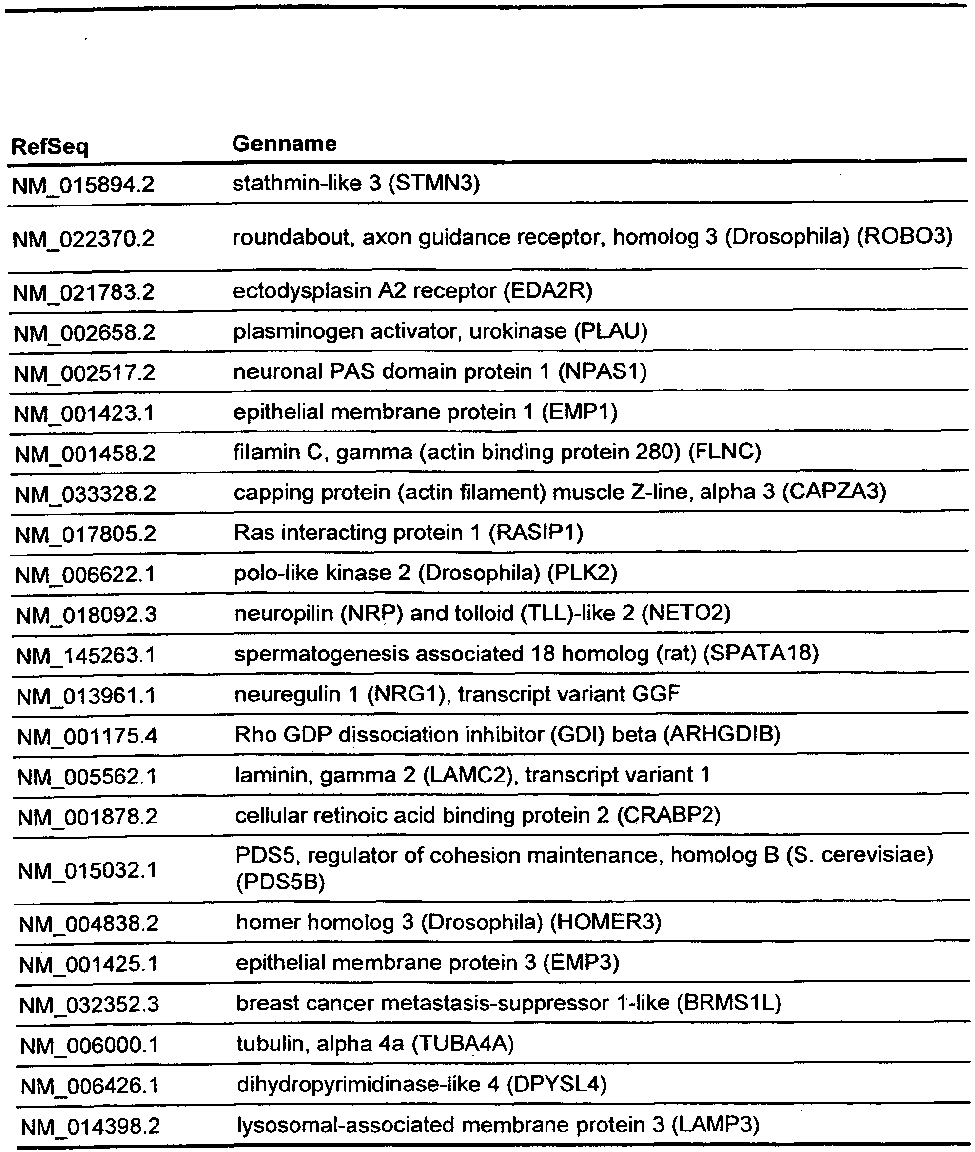

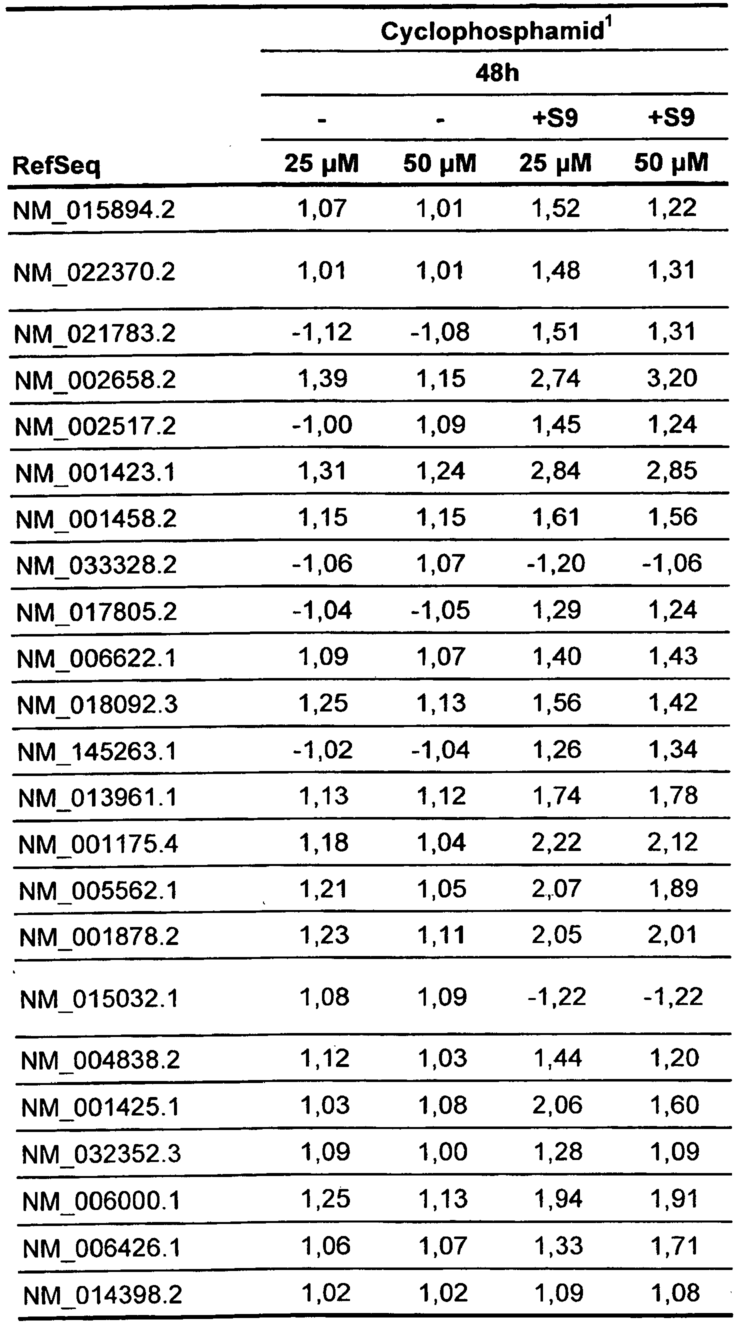

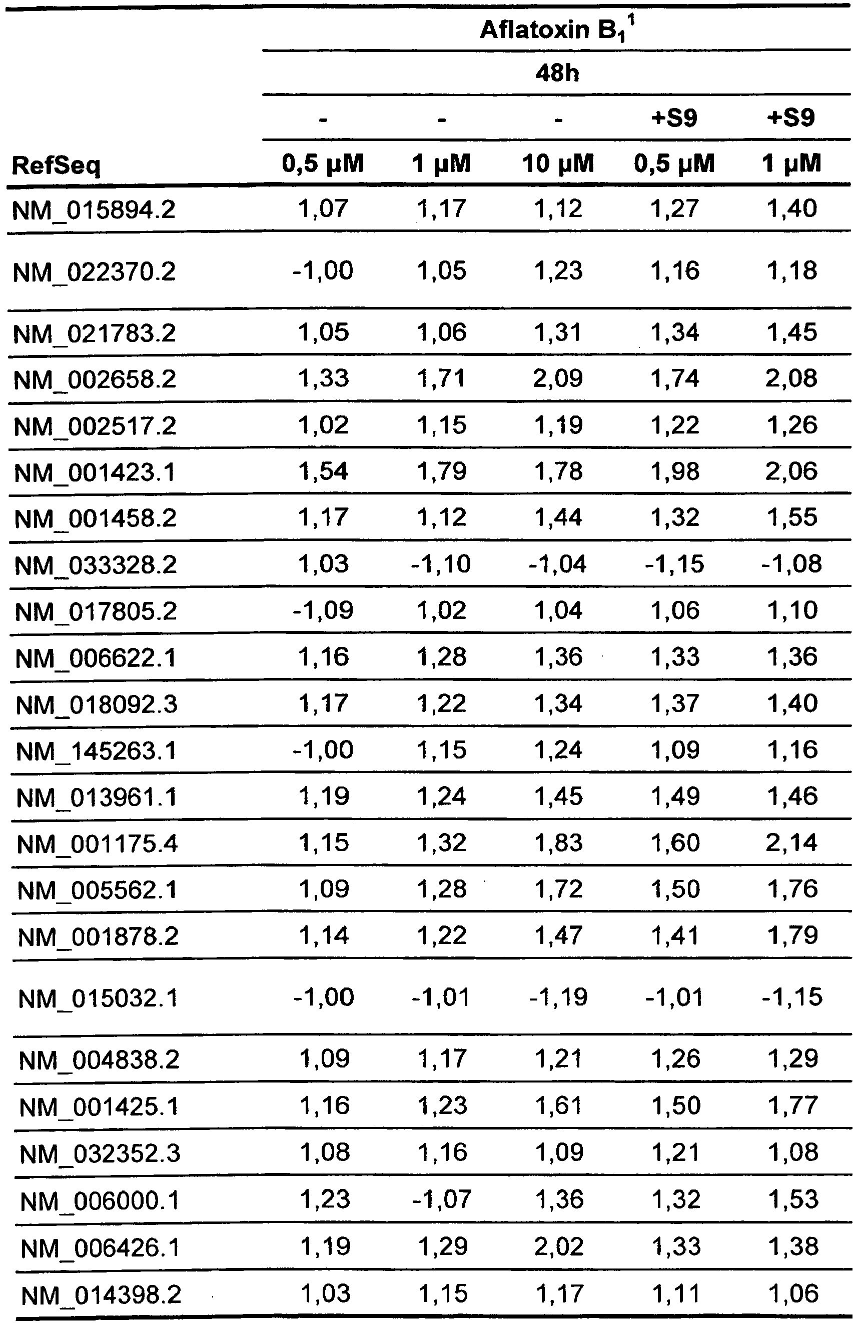

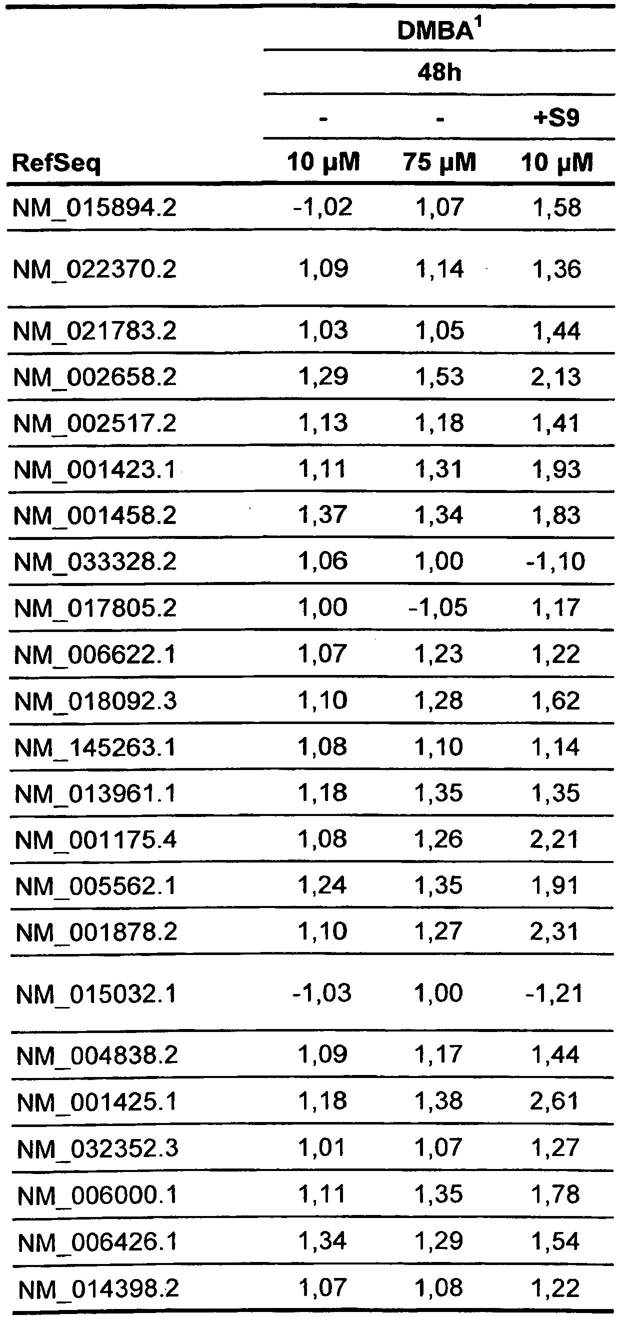

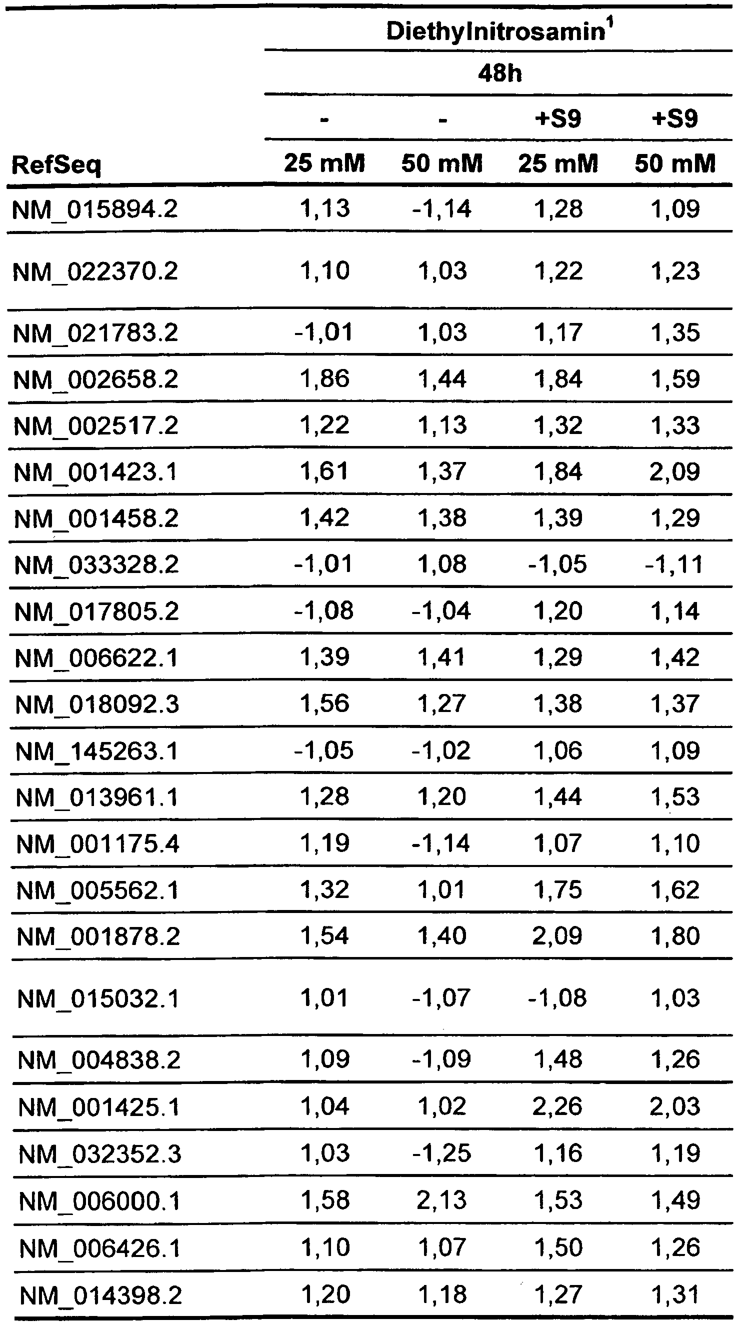

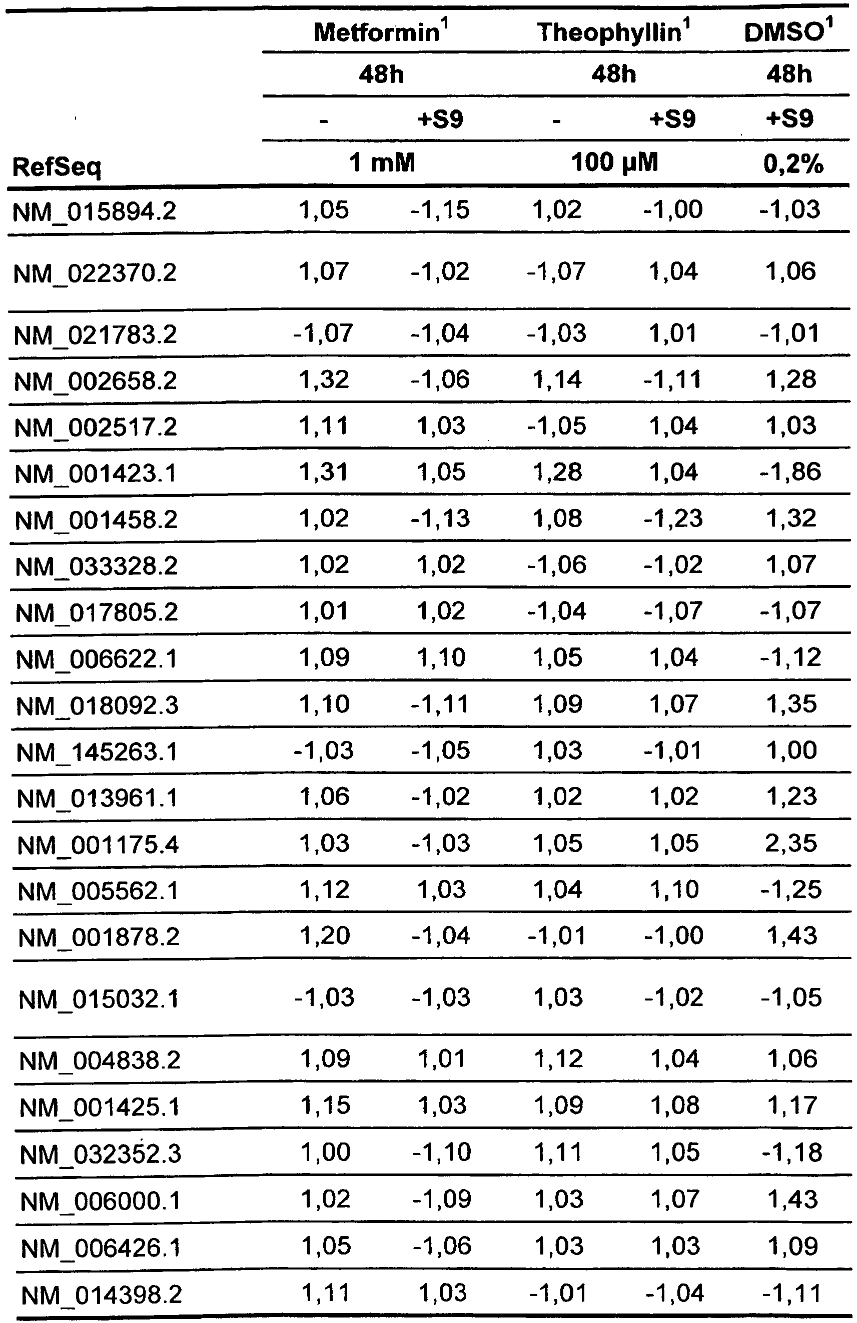

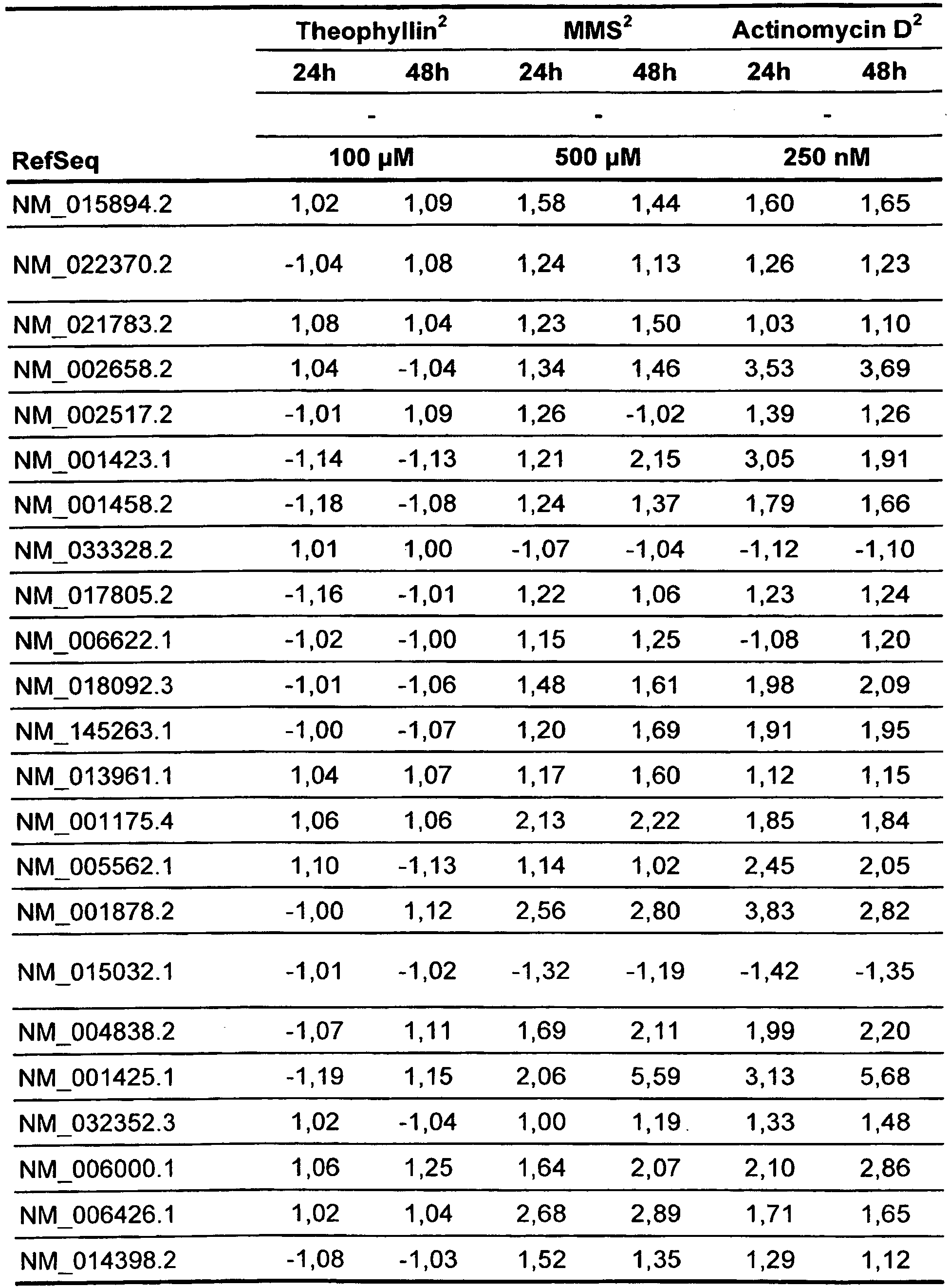

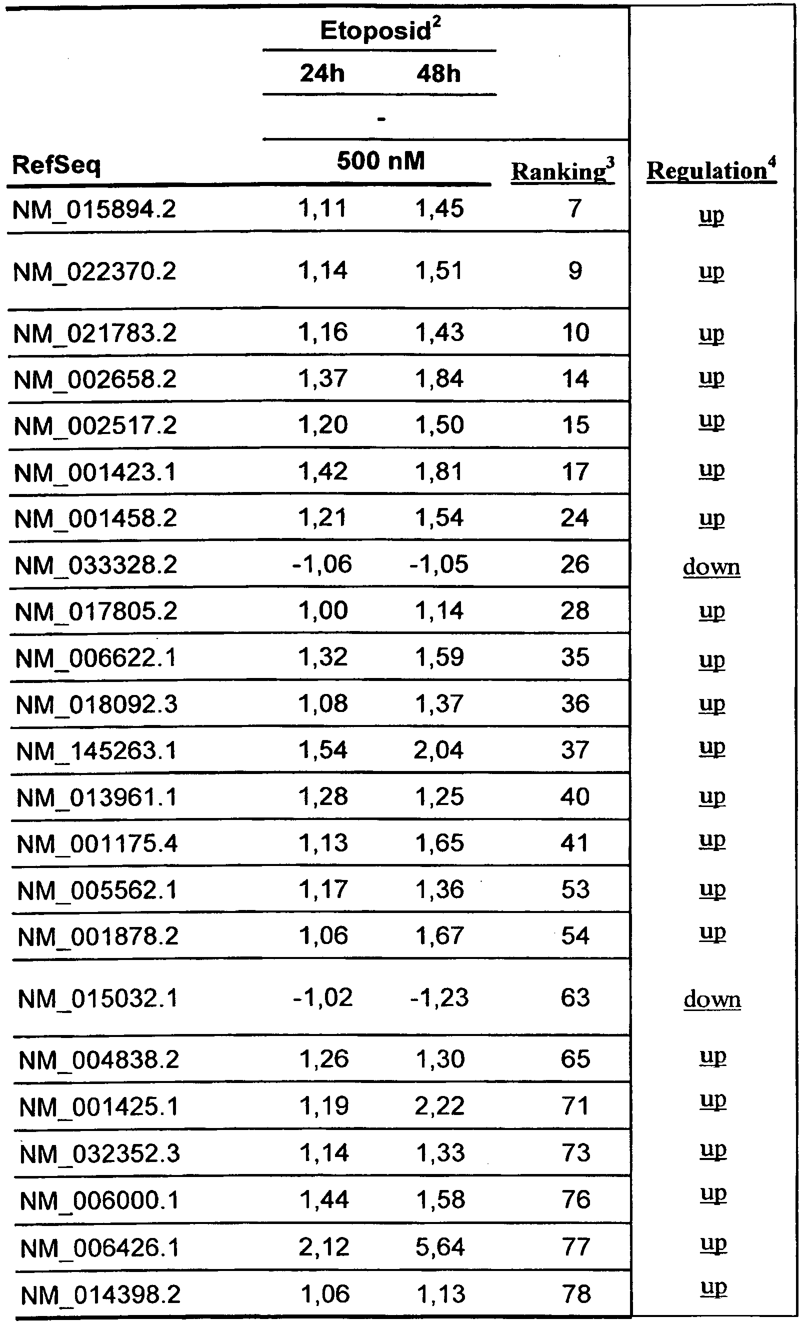

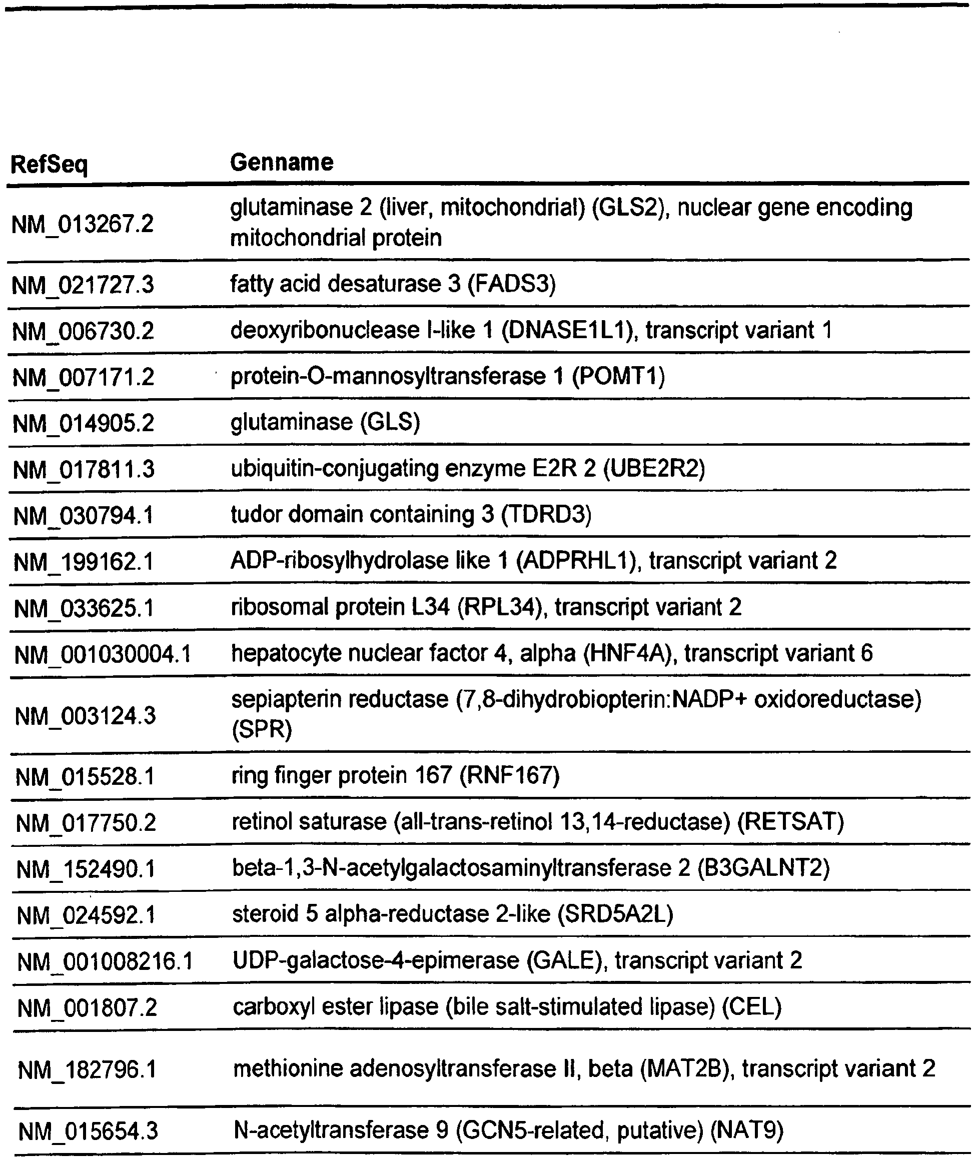

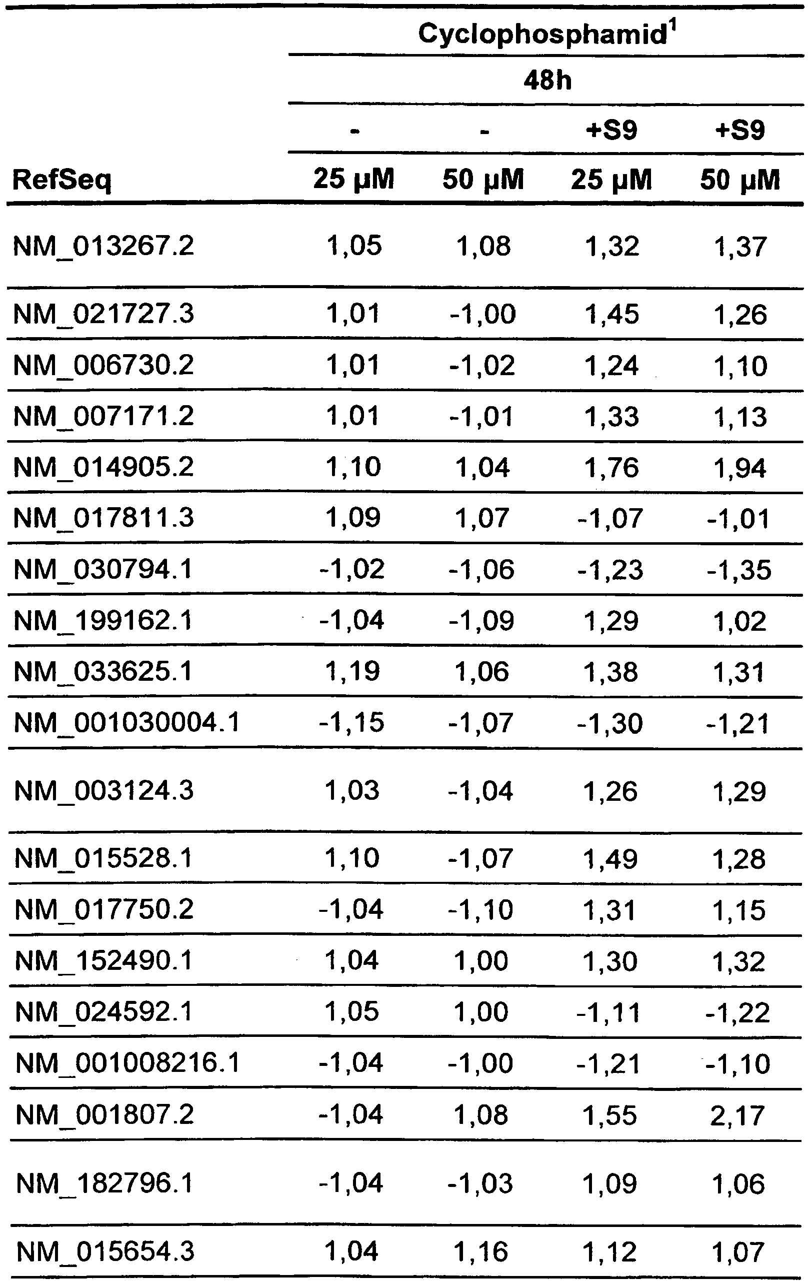

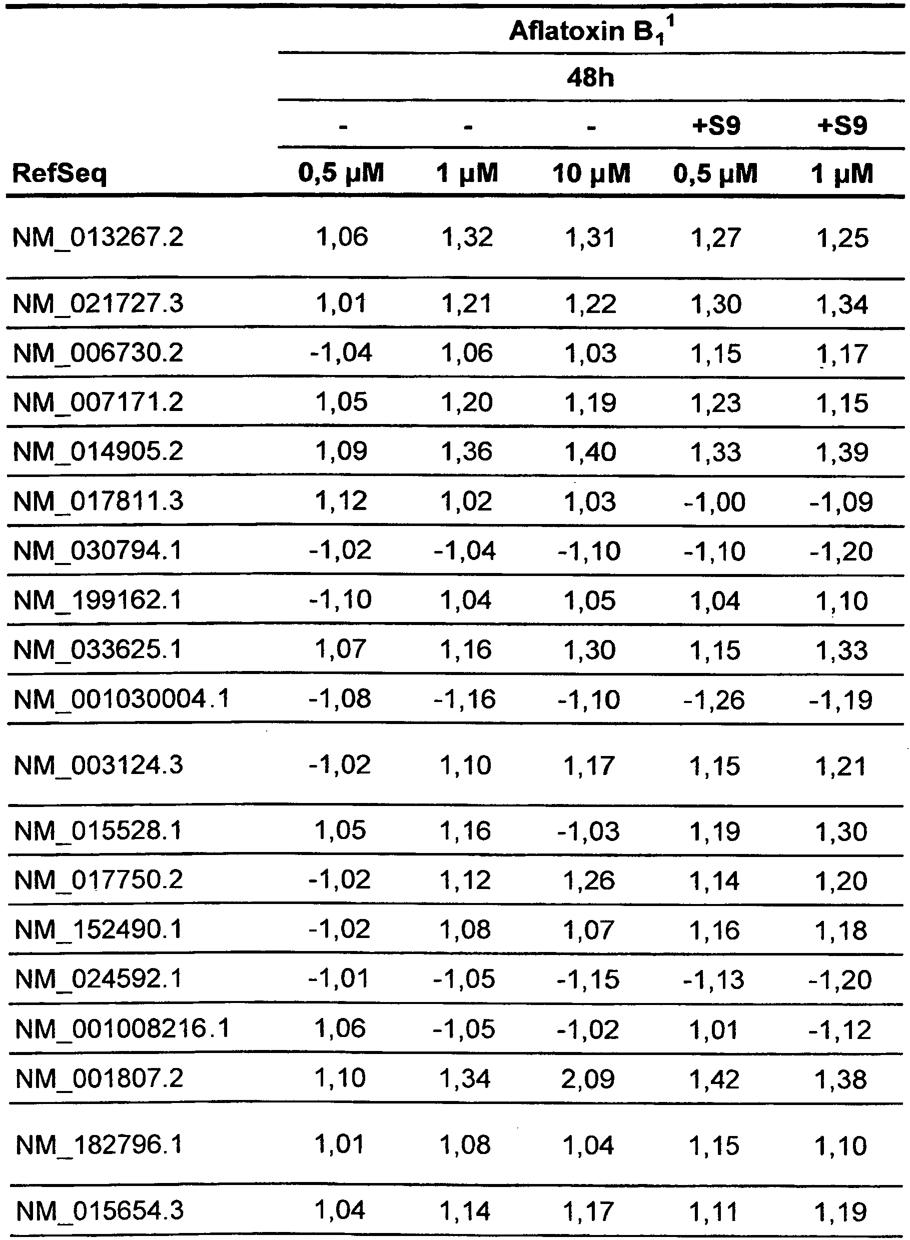

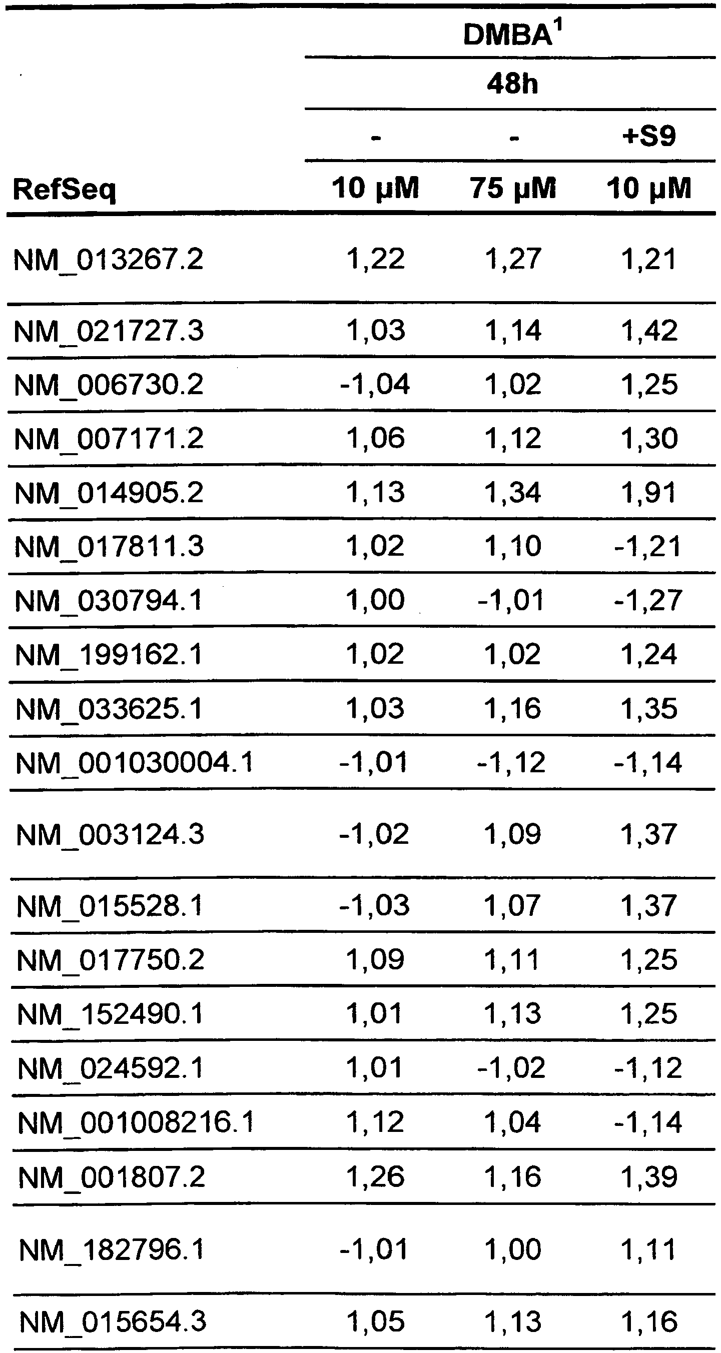

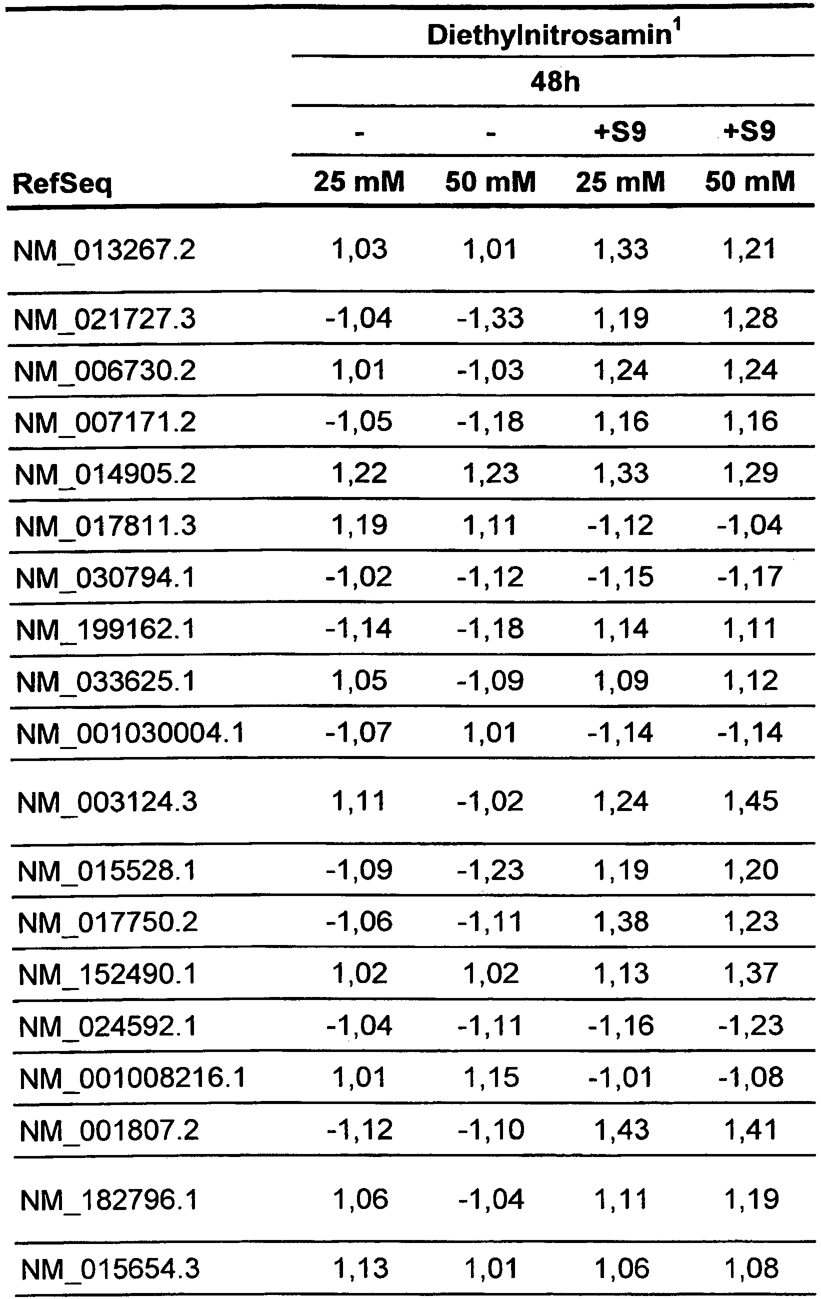

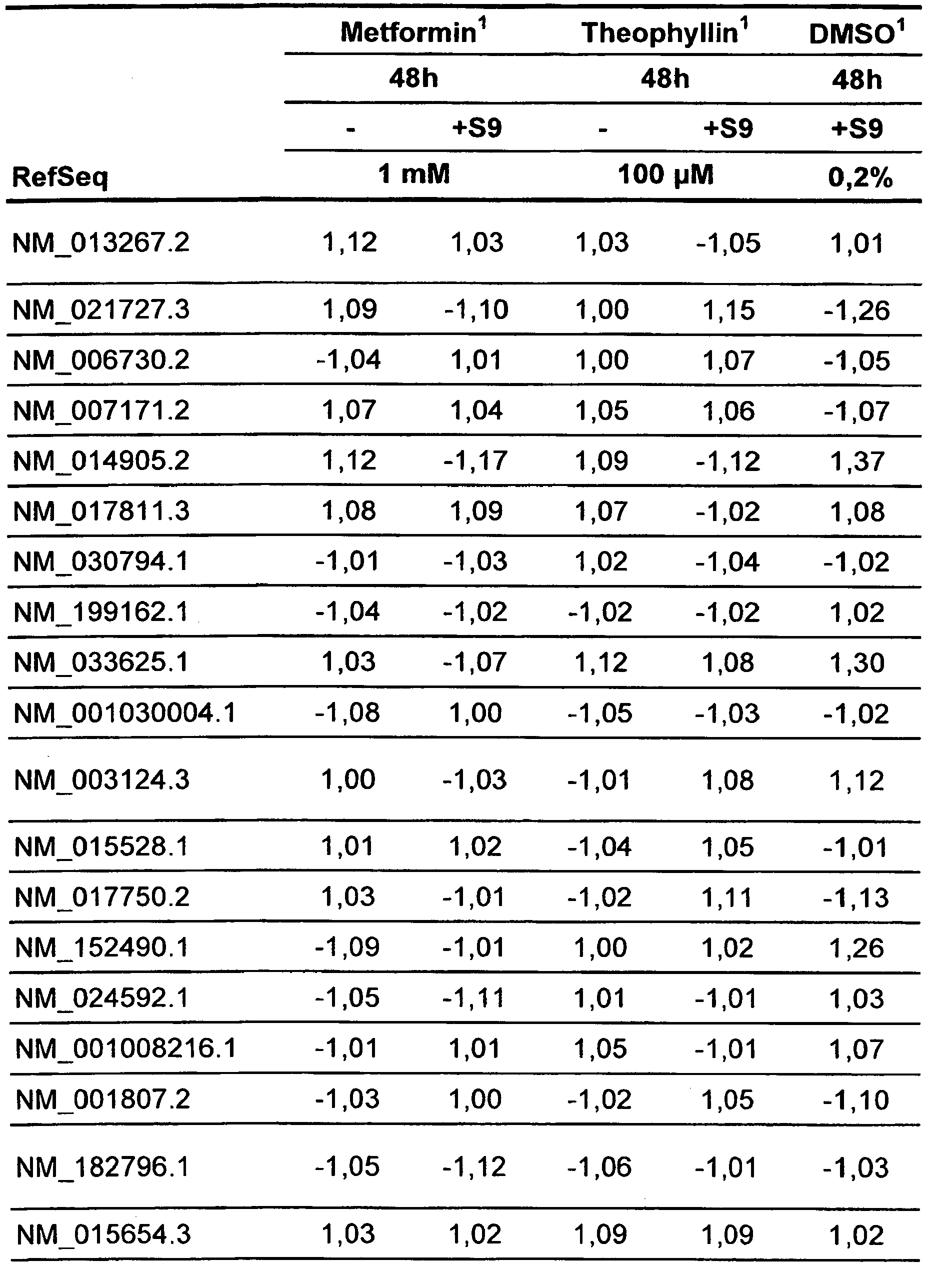

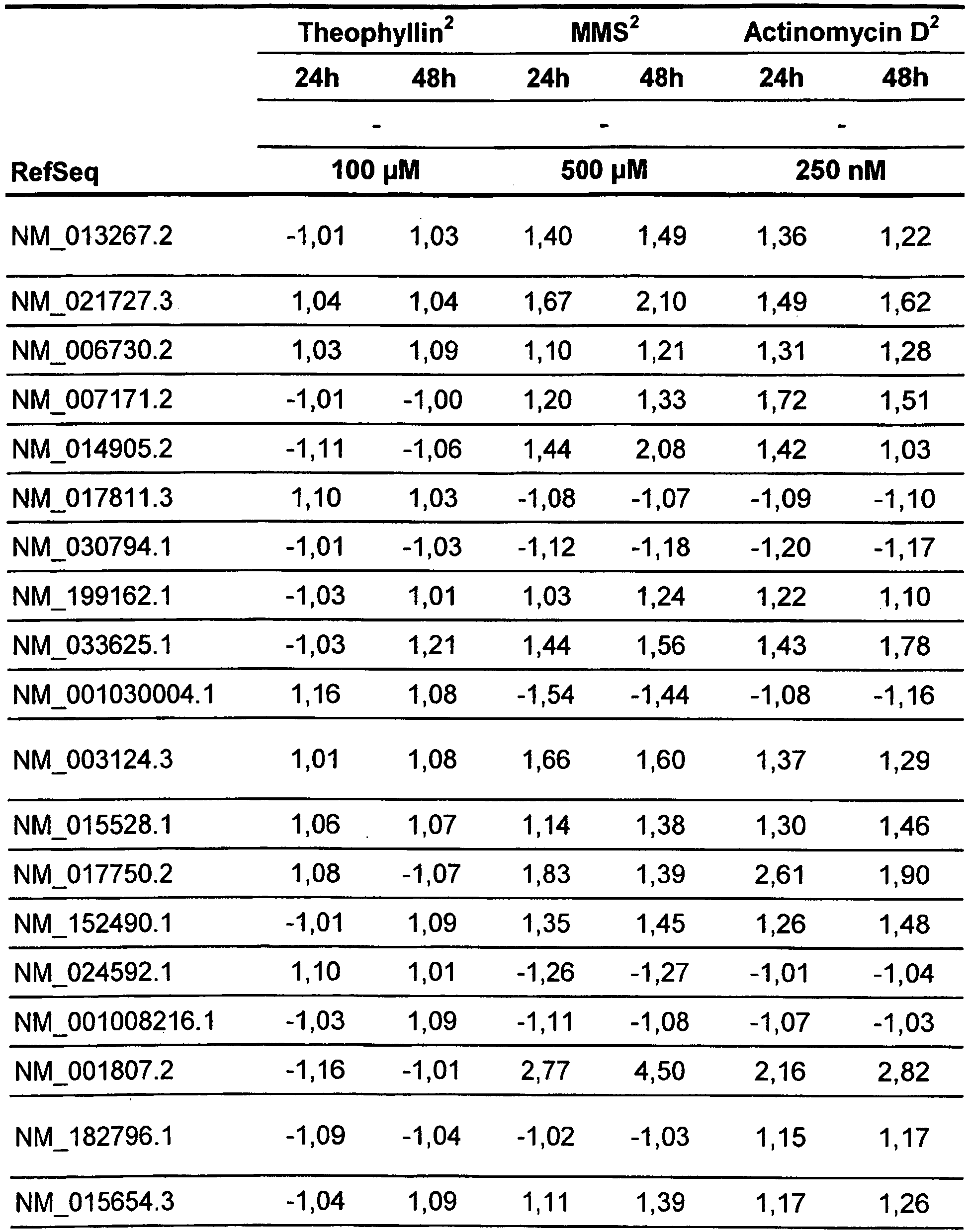

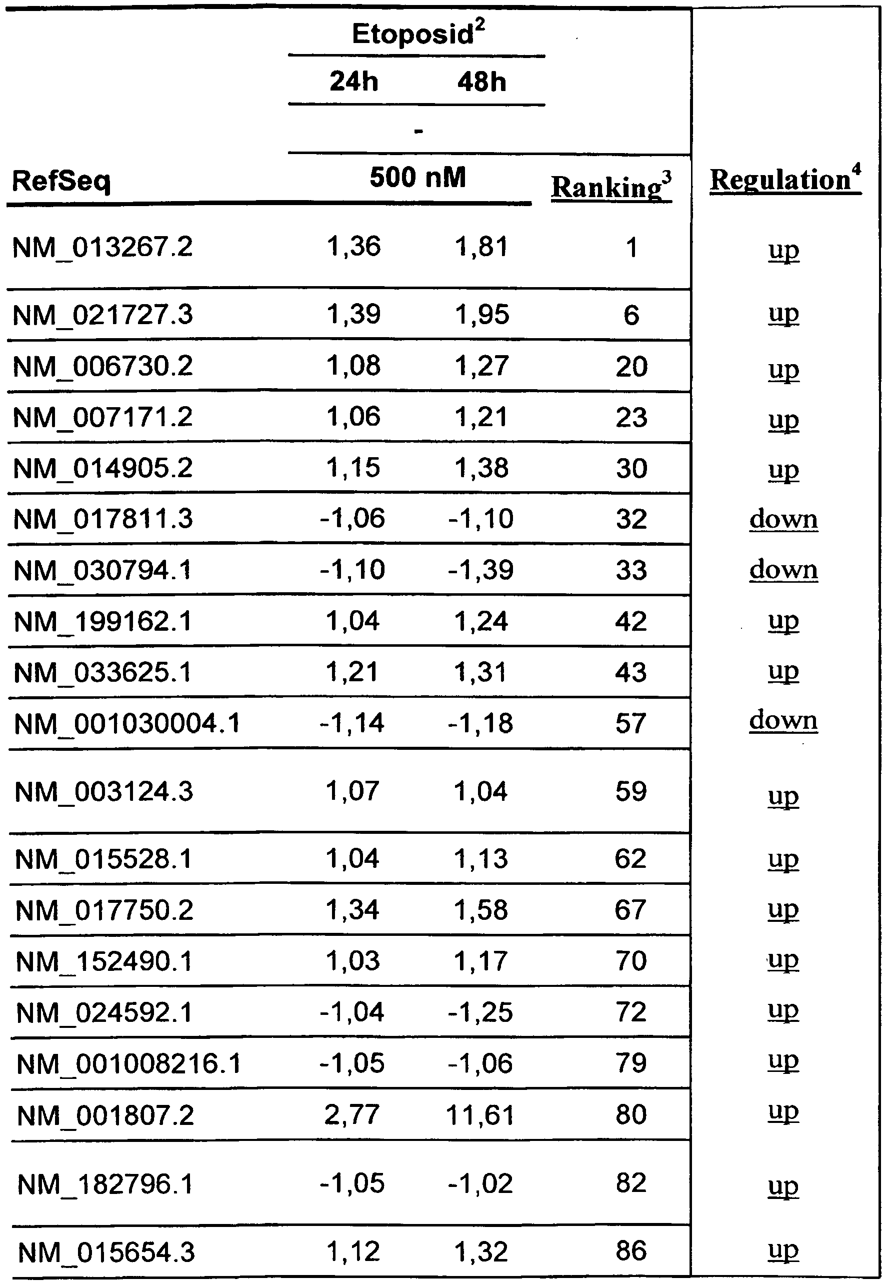

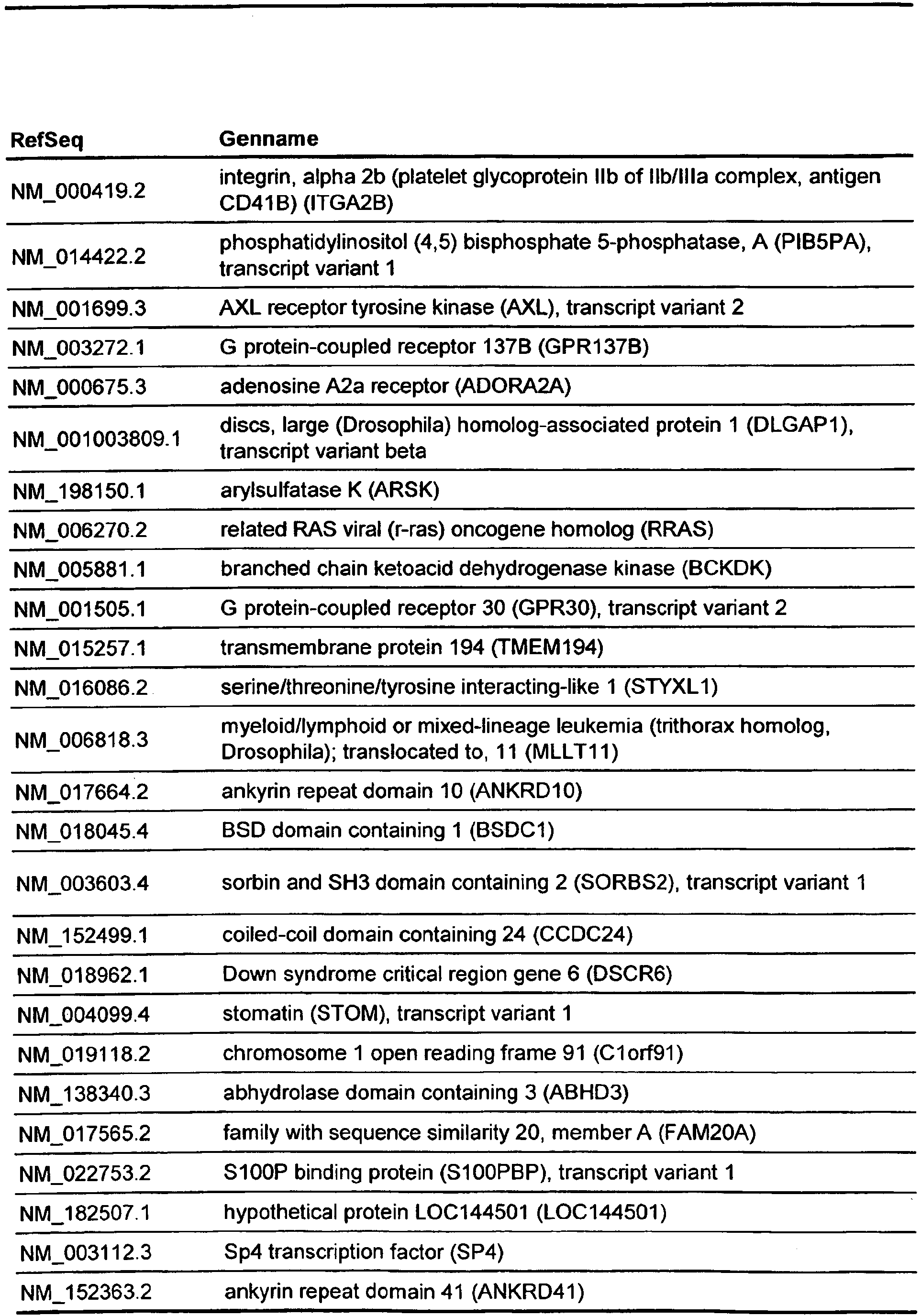

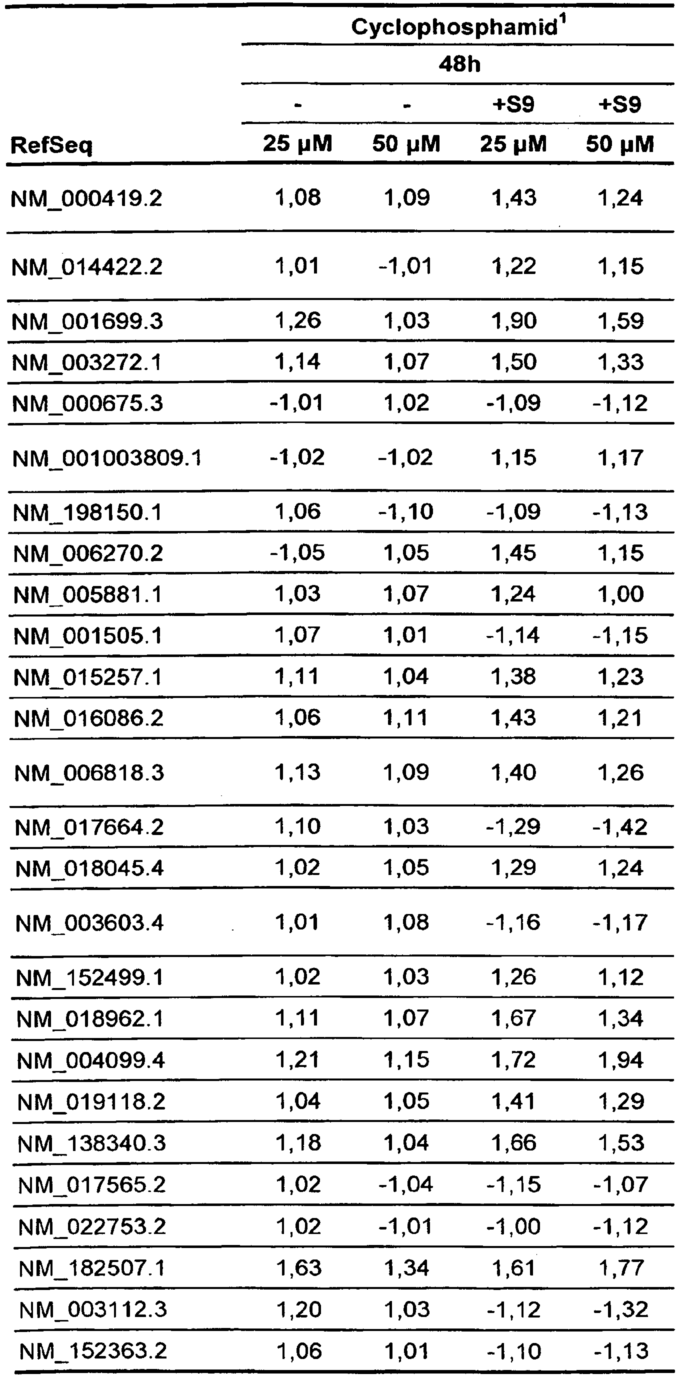

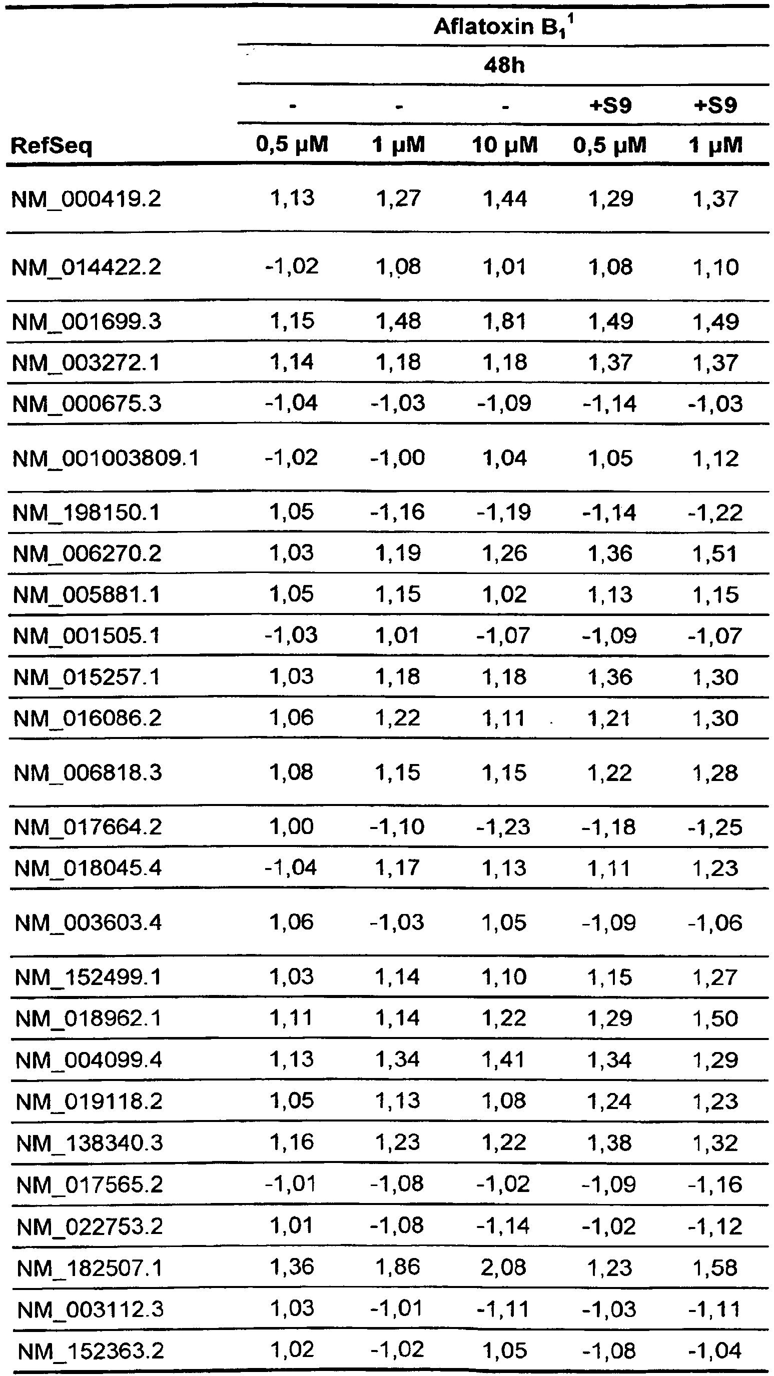

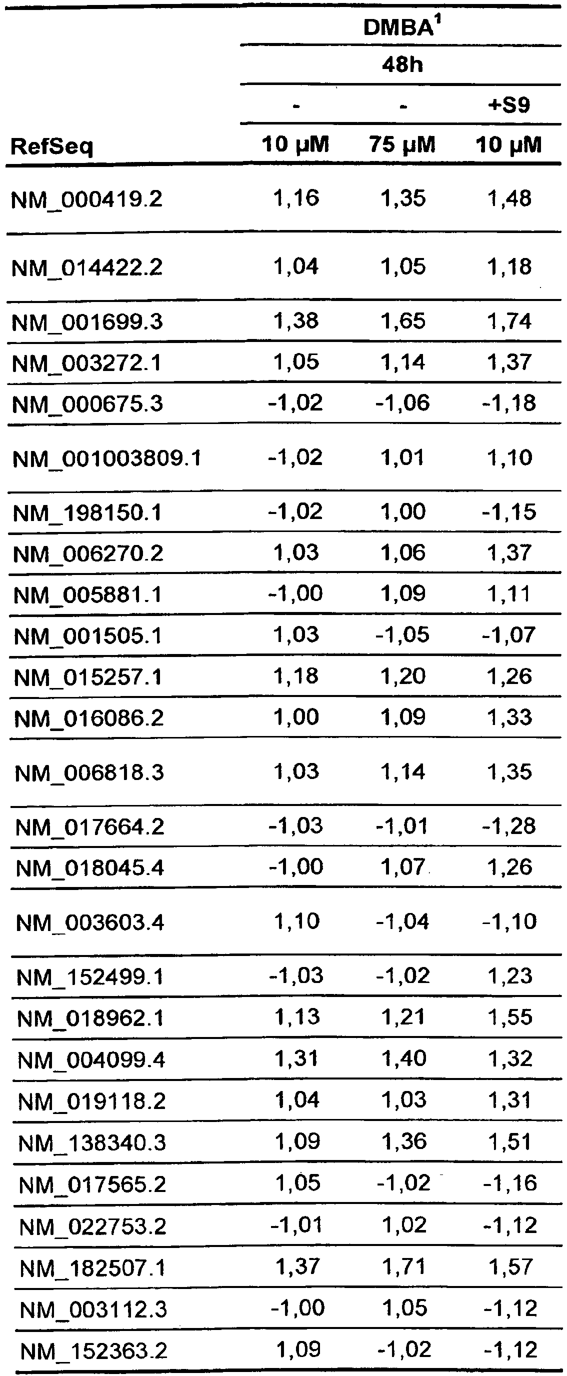

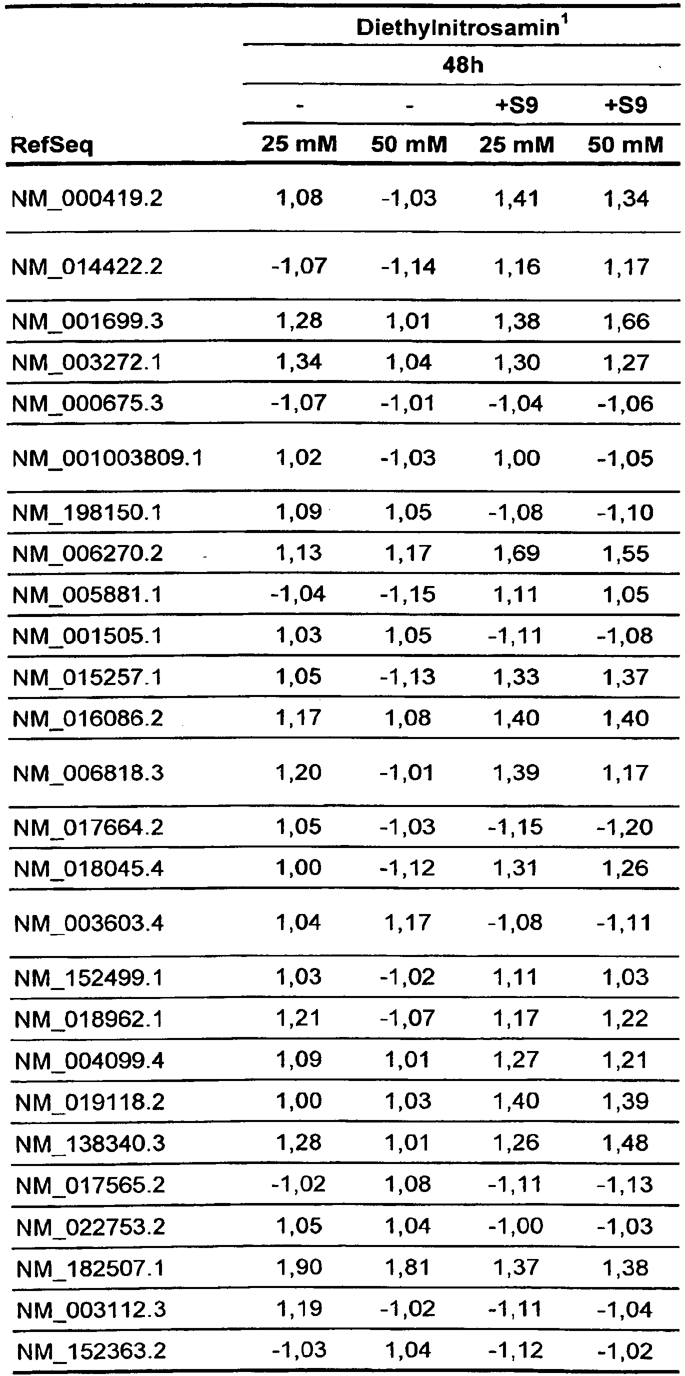

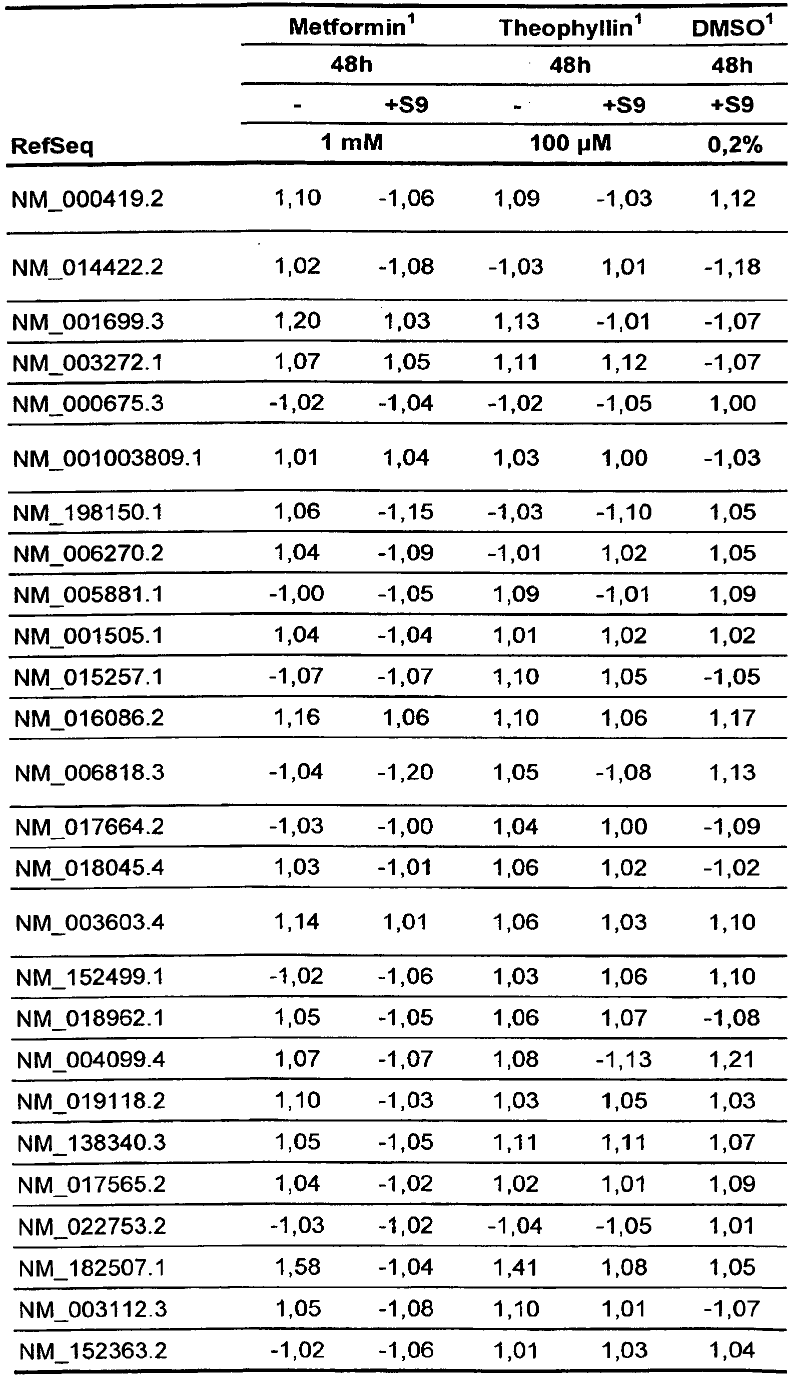

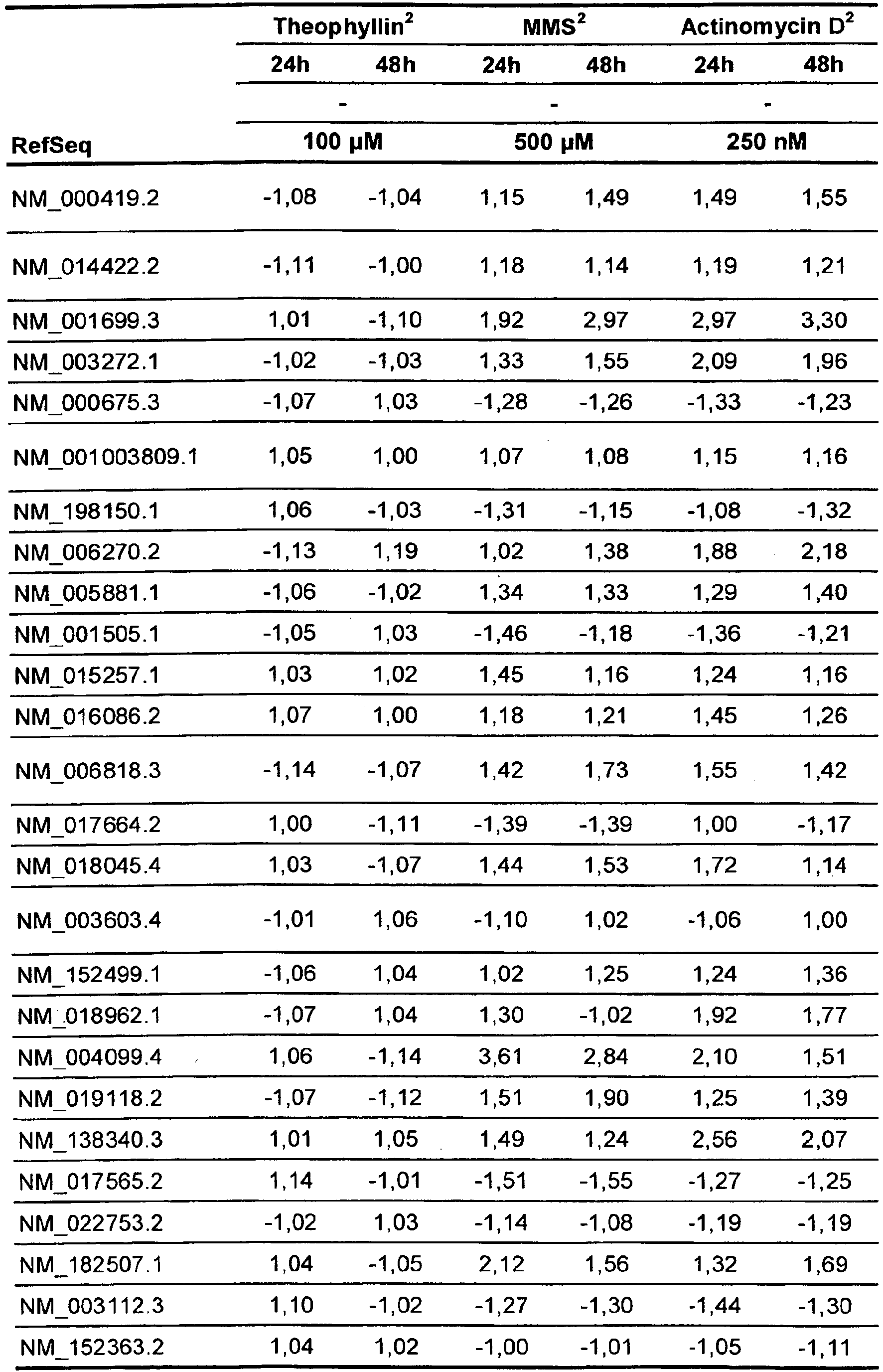

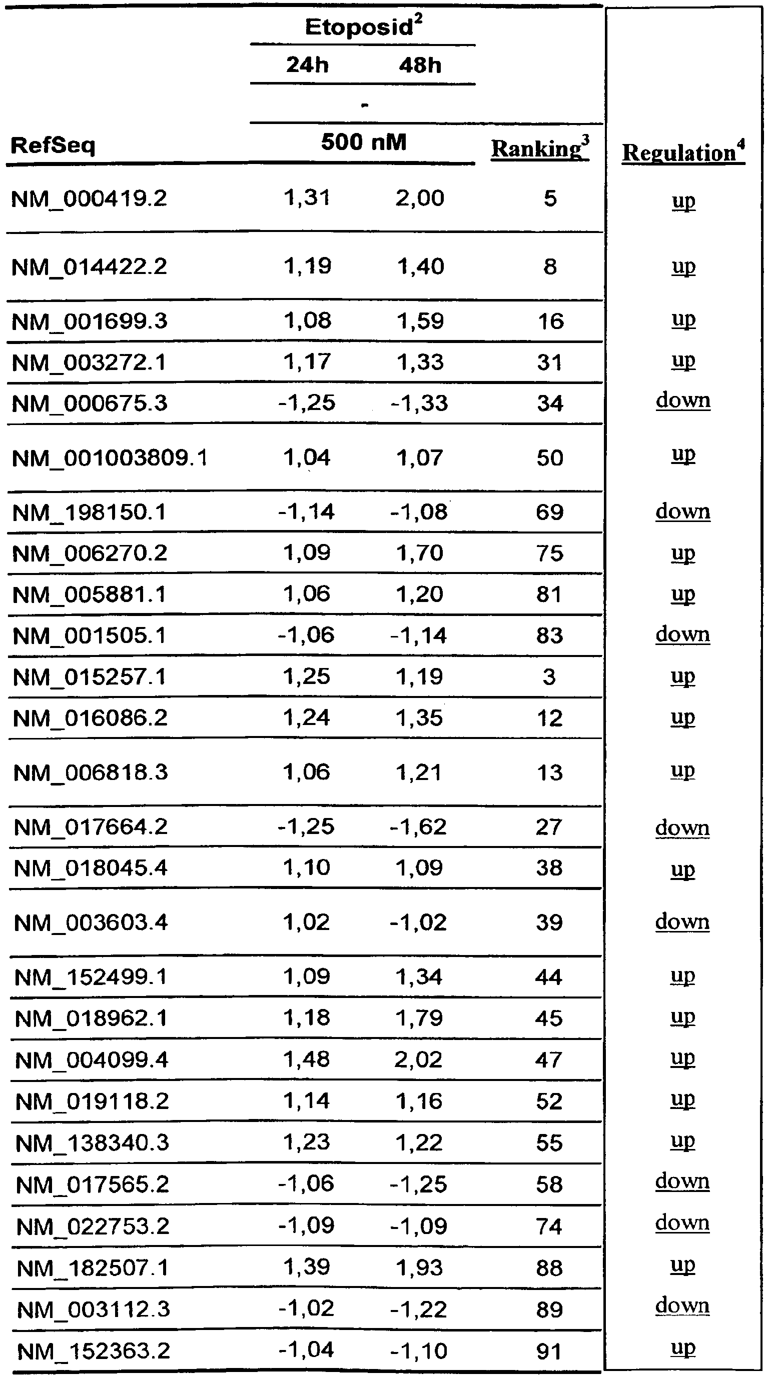

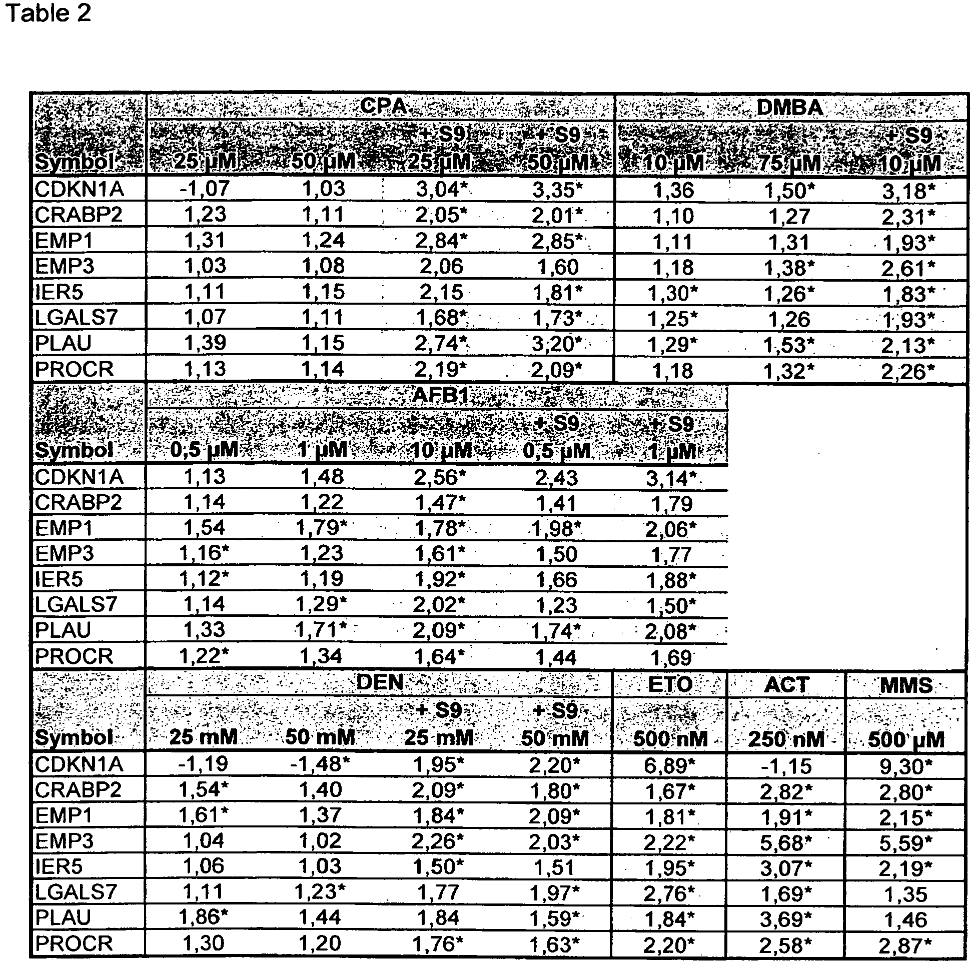

**Table 3**

| **Test sample** | **Dose** | **Time** | **MAS** | **Compound class** | **Categorization** | **P53** |
|---|---|---|---|---|---|---|
| | | | **(+) with S9** | | | **(+) FC≥1.5/FC≥1.5 and** |
| | | | **(-) without S9** | | | **p-value≤0.05** |
| methyl methane-sulfonate | 500 µM | **24h** | - | Genotoxic | genotoxic | +/+ |
| | 500 µM | **48h** | - | Genotoxic | genotoxic | +/+ |
| actinomycin D | 250 nM | **24h** | - | Genotoxic | genotoxic | +/+ |
| | 250 nM | **48h** | - | Genotoxic | genotoxic | +/+ |
| etoposide | 500 nM | **24h** | - | Genotoxic | genotoxic | +/+ |
| | 500 nM | **48h** | - | Genotoxic | genotoxic | +/+ |
| cyclophosphamide | 25 µM | **48h** | - | pro-genotoxic | non-genotoxic | -/- |
| | 25 µM | **48h** | + | pro-genotoxic | genotoxic | +/+ |
| | 50 µM | **48h** | - | pro-genotoxic | non-genotoxic | -/- |
| | 50 µM | **48h** | + | pro-genotoxic | genotoxic | +/+ |
| aflatoxin B₁ | 0.5 µM | **48h** | - | pro-genotoxic | non-genotoxic | -/- |
| | 0.5 µM | **48h** | + | pro-genotoxic | genotoxic | +/- |
| | 1 µM | **48h** | - | pro-genotoxic | genotoxic^{#} | +/+ |
| | 1 µM | **48h** | + | pro-genotoxic | genotoxic | +/+ |
| | 10 µM | **48h** | - | pro-genotoxic | genotoxic^{#} | +/+ |
| 7,12-dimethyl-benz[a]-anthracene | 10 µM | **48h** | - | pro-genotoxic | non-genotoxic | +/+ |
| | 10 µM | **48h** | + | pro-genotoxic | genotoxic | +/- |
| | 75 µM | **48h** | - | pro-genotoxic | genotoxic^{#} | +/+ |
| theophylline | 100 µM | **24h** | - | non-genotoxic | non-genotoxic | -/- |
| | 100 µM | **48h** | - | non-genotoxic | non-genotoxic | -/- |
| | 100 µM | **48h** | + | non-genotoxic | non-genotoxic | -/- |
| metformin | 1 mM | **48h** | + | non-genotoxic | non-genotoxic | -/- |
| | 1 mM | **48h** | - | non-genotoxic | non-genotoxic | -/- |
| S9 control | 0.2% DMSO | **48h** | + | non-genotoxic | non-genotoxic | -/- |

## Claims

1. A method for testing a compound for genotoxic and/or pro-genotoxic activity, comprising the steps of:
(a) contacting said compound to be tested with a test cellular system that expresses at least a panel of genes, which are GLS2, IER5, TMEM194, PROCR, ITGA2B, FADS3, STMN3, PIB5PA, ROBO3, EDA2R and KIF1A, wherein the system is selected from the group of single cells, cell cultures, tissues, organs and non-human mammals; and
(b) detecting and quantitating the expression of said panel of genes in said test system, wherein the gene expression is correlated with an amount of signal or change in signal, and comparing the expression of said panel of genes in said test system with the expression of said panel of genes in a negative control cellular system, wherein an up-regulation of expression of said panel of genes in said test system, as compared to the expression of said panel of genes in said control system, indicates that said compound has genotoxic and/or pro-genotoxic activity.

2. The method according to claim 1, wherein in step (a) said compound to be tested is administered to a non-human mammal, and in step (b) the expression of said panel of genes in a biological sample withdrawn from the non-human mammal is compared to the expression of said panel of genes in a biological sample withdrawn from a non-human mammal showing non-genotoxic effects, wherein said up-regulation indicates an increased likelihood of said compound to have a therapeutic effect for a genotoxicity-mediated pathological condition.

3. A method for monitoring the likelihood of response to a treatment of cancer, tumors, metastasis or disorders of angiogenesis, which are caused, mediated and/or propagated by deregulation of proliferation, differentiation and/or damage repair, wherein expression levels of at least a panel of genes, which are GLS2, IER5, TMEM194, PROCR, ITGA2B, FADS3, STMN3, PIB5PA, ROBO3, EDA2R and KIF1A, are determined in a biological sample withdrawn from a mammal in need of such treatment with at least one genotoxic or pro-genotoxic compound, or a physiologically acceptable salt thereof, which was administered to said mammal, wherein an up-regulated level indicates an increased likelihood that said mammal responds to the treatment with said compound.

4. Use of a panel of genes, which are GLS2, IER5, TMEM194, PROCR, ITGA2B, FADS3, STMN3, PIB5PA, ROBO3, EDA2R and KIF1A, as marker genes for testing a compound for genotoxic and/or pro-genotoxic activity.

5. Use of nucleic acid probes specifically hybridizing under stringent conditions with a panel of genes, which are GLS2, IER5, TMEM194, PROCR, ITGA2B, FADS3, STMN3, PIB5PA, ROBO3, EDA2R and KIF1A, or gene products encoded by said panel of genes, or respective parts thereof, for detecting and quantitating the expression of said panel of genes, which is representative for a genotoxic and/or pro-genotoxic response in a cellular system.

6. Array for testing a compound for genotoxic and/or pro-genotoxic activity, comprising nucleic acid probes that specifically hybridize under stringent conditions with a panel of genes, which are GLS2, IER5, TMEM194, PROCR, ITGA2B, FADS3, STMN3, PIB5PA, ROBO3, EDA2R and KIF1A, or gene products encoded by said panel of genes, or respective parts thereof.

## Patentansprüche

1. Verfahren zum Testen einer Verbindung auf genotoxische und/oder progenotoxische Aktivität mit den folgenden Schritten:
(a) Zusammenbringen der Verbindung, die getestet werden soll, mit einem zellulären Testsystem, das zumindest ein Panel von Genen exprimiert, bei denen es sich um GLS2, IER5, TMEM194, PROCR, ITGA2B, FADS3, STMN3, PIB5PA, ROBO3, EDA2R und KIF1A handelt, wobei das System aus der Gruppe mit Einzelzellen, Zellkulturen, Geweben, Organen und nicht-menschlichen Säugetieren ausgewählt ist, und
(b) Nachweisen und Quantifizieren der Expression des Panels von Genen in dem Testsystem, wobei die Genexpression mit einer Höhe eines Signals oder einer Veränderung in einem Signal korreliert, und Vergleichen der Expression des Panels von Genen in dem Testsystem mit der Expression des Panels von Genen in einem zellulären Negativkontrollsystem, wobei eine Aufregulation der Expression des Panels von Genen in dem Testsystem im Vergleich zu der Expression des Panels von Genen in dem Kontrollsystem darauf hindeutet, dass die Verbindung genotoxische und/oder progenotoxische Aktivität besitzt.

2. Verfahren nach Anspruch 1, wobei in Schritt (a) die Verbindung, die getestet werden soll, einem nicht-menschlichen Säugetier verabreicht wird und in Schritt (b) die Expression des Panels von Genen in einer von dem nicht-menschlichen Säugetier entnommenen Probe mit der Expression des Panels von Genen in einer Probe verglichen wird, die von einem nicht-menschlichen Säugetier entnommen wurde, das nicht-genotoxische Wirkungen zeigt, wobei die Aufregulation auf eine erhöhte Wahrscheinlichkeit hindeutet, dass die Verbindung eine therapeutische Wirkung bei einem genotoxizitätsvermittelten pathologischen Zustand besitzt.

3. Verfahren zur Überwachung der Wahrscheinlichkeit eines Ansprechens auf eine Behandlung von Krebs, Tumoren, Metastasierung oder Störungen der Angiogenese, die durch eine Deregulation der Proliferation, Differenzierung und/oder Reparatur von Schäden verursacht, vermittelt und/oder verbreitet werden, wobei die Expressionsspiegel von mindestens einem Panel von Genen, bei denen es sich um GLS2, IER5, TMEM194, PROCR, ITGA2B, FADS3, STMN3, PIB5PA, ROBO3, EDA2R und KIF1A handelt, in einer biologischen Probe bestimmt werden, die von einem Säugetier entnommen wurde, das einer solchen Behandlung bedarf, mit mindestens einer genotoxischen oder progenotoxischen Verbindung oder einem physiologischen annehmbaren Salz davon, die/das dem Säugetier verabreicht wurde, wobei ein nach oben regulierter Spiegel auf eine erhöhte Wahrscheinlichkeit hindeutet, dass das Säugetier auf die Behandlung mit der Verbindung anspricht.

4. Verwendung eines Panels von Genen, bei denen es sich um GLS2, IER5, TMEM194, PROCR, ITGA2B, FADS3, STMN3, PIB5PA, ROBO3, EDA2R und KIF1A handelt, als Markergene zum Testen einer Verbindung auf genotoxische und/oder progenotoxische Aktivität.

5. Verwendung von Nukleinsäuresonden, die unter stringenten Bedingungen spezifisch an ein Panel von Genen, bei denen es sich um GLS2, IER5, TMEM194, PROCR, ITGA2B, FADS3, STMN3, PIB5PA, ROBO3, EDA2R und KIF1A handelt, oder von dem Panel von Genen codierte Genprodukte oder jeweilige Teile davon hybridisieren, zum Nachweisen und Quantifizieren der Expression des Panels von Genen, das repräsentativ für eine genotoxische und/oder progenotoxische Reaktion in einem zellulären System ist.

6. Array zum Testen einer Verbindung auf genotoxische und/oder progenotoxische Aktivität, umfassend Nukleinsäuresonden, die unter stringenten Bedingungen spezifisch an ein Panel von Genen, bei denen es sich um GLS2, IER5, TMEM194, PROCR, ITGA2B, FADS3, STMN3, PIB5PA, ROBO3, EDA2R und KIF1A handelt, oder von dem Panel von Genen codierte Genprodukte oder jeweilige Teile davon hybridisieren.

## Revendications

1. Procédé pour tester un composé quant à une activité génotoxique et/ou pro-génotoxique, comprenant les étapes de :
(a) mise en contact dudit composé destiné à être testé avec un système cellulaire de test qui exprime au moins un ensemble de gènes, lesquels sont GLS2, IER5, TMEM194, PROCR, ITGA2B, FADS3, STMN3, PIB5PA, ROBO3, EDA2R et KIF1A, dans lequel le système est choisi parmi le groupe des cellules individuelles, des cultures de cellules, des tissus, des organes et des mammifères non humains ; et
(b) détection et quantification de l'expression dudit ensemble de gènes dans ledit système de test, dans lequel l'expression des gènes est corrélée avec une valeur de signal ou une variation de signal, et comparaison de l'expression dudit ensemble de gènes dans ledit système de test avec l'expression dudit ensemble de gènes dans un système cellulaire de contrôle négatif, dans lequel une régulation à la hausse de l'expression dudit ensemble de gènes dans ledit système de test, par comparaison avec l'expression dudit ensemble de gènes dans ledit système de contrôle, indique que ledit composé présente une activité génotoxique et/ou pro-génotoxique.

2. Procédé selon la revendication 1, dans lequel, au niveau de l'étape (a), ledit composé destiné à être testé est administré à un mammifère non humain, et au niveau de l'étape (b), l'expression dudit ensemble de gènes dans un échantillon biologique extrait à partir du mammifère non humain est comparée à l'expression dudit ensemble de gènes dans un échantillon biologique extrait à partir d'un mammifère non humain présentant des effets non génotoxiques, dans lequel ladite régulation à la hausse indique une probabilité augmentée que ledit composé présent un effet thérapeutique pour une condition pathologique favorisée par la génotoxicité.

3. Procédé pour surveiller la probabilité de réponse à un traitement du cancer, de tumeurs, de métastases ou de troubles de l'angiogénèse, lesquels sont générés, favorisés et/ou propagés par une dérégulation de la prolifération, de la différentiation et/ou de la réparation d'un dommage, dans lequel des niveaux d'expression d'au moins un ensemble de gènes, lesquels sont GLS2, IER5, TMEM194, PROCR, ITGA2B, FADS3, STMN3, PIB5PA, ROBO3, EDA2R et KIF1A, sont déterminés dans un échantillon biologique extrait à partir d'un mammifère qui a besoin d'un traitement avec au moins un composé génotoxique ou pro-génotoxique, ou un sel physiologiquement acceptable afférent, lequel a été administré audit mammifère, dans lequel un niveau de régulation à la hausse indique une probabilité augmentée que ledit mammifère réponde au traitement avec ledit composé.

4. Utilisation d'un ensemble de gènes, lesquels sont GLS2, IER5, TMEM194, PROCR, ITGA2B, FADS3, STMN3, PIB5PA, ROBO3, EDA2R et KIF1A, en tant que gènes marqueurs pour tester un composé quant à une activité génotoxique et/ou pro-génotoxique.

5. Utilisation de sondes d'acides nucléiques s'hybridisant de façon spécifique sous des conditions stringentes avec un ensemble de gènes, lesquels sont GLS2, IER5, TMEM194, PROCR, ITGA2B, FADS3, STMN3, PIB5PA, ROBO3, EDA2R et KIF1A, ou des produits de gènes codés par ledit ensemble de gènes, ou des parties respectives afférentes, pour détecter et quantifier l'expression dudit ensemble de gènes, laquelle est représentative d'une réponse génotoxique et/ou pro-génotoxique dans un système cellulaire.

6. Réseau pour tester un composé quant à une activité génotoxique et/ou pro-génotoxique, comprenant des sondes d'acides nucléiques qui s'hybridisent de façon spécifique sous des conditions stringentes avec un ensemble de gènes, lesquels sont GLS2, IER5, TMEM194, PROCR, ITGA2B, FADS3, STMN3, PIB5PA, ROBO3, EDA2R et KIF1A, ou des produits de gènes codés par ledit ensemble de gènes, ou des parties respectives afférentes.
